(19) Europäisches Patentamt
European Patent Office
Office européen des brevets

(11) EP 4 289 952 A1

(12) # EUROPEAN PATENT APPLICATION
published in accordance with Art. 153(4) EPC

(43) Date of publication:
**13.12.2023 Bulletin 2023/50**

(21) Application number: **22749093.5**

(22) Date of filing: **28.01.2022**

(51) International Patent Classification (IPC):
***C12N 15/113*** *(2010.01)* ***C12N 15/85*** *(2006.01)*
***A61P 35/00*** *(2006.01)* ***A61K 31/713*** *(2006.01)*

(52) Cooperative Patent Classification (CPC):
**A61K 31/713; A61P 35/00; C12N 15/113; C12N 15/85**

(86) International application number:
**PCT/CN2022/074635**

(87) International publication number:
**WO 2022/166814 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.02.2021 CN 202110175652**
**08.02.2021 PCT/CN2021/075958**

(71) Applicant: **Ractigen Therapeutics**
**Nantong, Jiangsu 226400 (CN)**

(72) Inventors:
- **LI, Longcheng**
**Nantong, Jiangsu 226400 (CN)**
- **KANG, Moorim**
**Nantong, Jiangsu 226400 (CN)**

(74) Representative: **Goddar, Heinz J.**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

(54) **CHEMICALLY MODIFIED SMALL ACTIVATING RNA**

(57) Provided is a small activating RNA used for promoting an expression level of a target gene. The small activating RNA is composed of a sense oligonucleotide chain and an antisense oligonucleotide chain. A nucleotide of the sense or antisense oligonucleotide chain has 2'-fluoro, 2'-methoxy, and a phosphorothioate modification to the nucleotide backbone.

EP 4 289 952 A1

## Description

FIELD OF THE INVENTION

The present disclosure relates to the field of biomedicine, in particular to a chemically modified small activating RNA, which is a double-stranded small RNA that up-regulates the expression of a gene by targeting the promoter sequence of the gene.

BACKGROUND OF THE INVENTION

Oligonucleotides can regulate target gene expression through various molecular mechanisms such as RNAi, RNase H activity, miRNA inhibition, and RNA activation (RNAa), which are referred to as siRNA, antisense oligonucleotide (ASO), miRNA mimics or inhibitors, and small activating RNA (saRNA), respectfully. Different from siRNA and ASO, saRNA is a double-stranded RNA oligonucleotide targeting the non-coding sequence in the nucleus to stimulate the expression of target genes at the transcriptional and epigenetic levels (Li, Adv Exp Med Biol. 2007, 983:1-20).

It has been well recognized that chemical modification of oligonucleotides has played a key role in the development of oligonucleotides as therapeutic agents (Manoharan, Curr Opin Chem Biol. 2004, 8(6): 570-579; Khvorova and Watts. Nat Biotechnol. 2017, 35(3): 238-248). Chemical modification of oligonucleotide sequences can improve their *in vivo* activity in various ways, such as increasing resistance to nucleases, improving cellular uptake, reducing immune stimulation and off-target effects, but at the same time, it may also affect the activity of oligonucleotides by reducing a mechanism of itself such as gene knockdowning (similar to the situation in RNAi) (Khvorova and Watts. Nat Biotechnol, 2017, 35(3):238-248). Many different chemical modifications have been used in the field of siRNA and antisense oligonucleotide (ASO) drug development. The most commonly used modifications are 2'-O-methyl and 2'-fluoro (2'F) modifications to the ribose sugar group and modification by replacing phosphodiester bonds with phosphorothioate (PS) bonds (Watts, Deleavey et al. Drug Discov Today, 2008, 13(19-20):842-855). These modifications can be used alone or in combination to produce chimeric oligonucleotides. However, how to improve the pharmaceutical properties of saRNA oligonucleotides, including increasing their stability, suppressing immunostimulatory activity and mitigating off-target effects, remains to be problematic.

SUMMARY OF THE INVENTION

One object of the present disclosure is to improve the pharmaceutical properties of saRNA oligonucleotides and provide chemical modifications to nucleotides in saRNA. The inventors have surprisingly found that optimized chemical modifications can stabilize saRNA duplexes, suppress their immunostimulatory activity, and/or mitigate off-target effects. Most importantly, these modifications can also increase the potency of saRNA-induced RNA activation.

The application provides a chemically modified small activating RNA that can be used to activate target genes. The small activating RNA is composed of a sense oligonucleotide strand and an antisense oligonucleotide strand, the sense oligonucleotide strand or the antisense oligonucleotide strand being at least 75%, at least 80%, at least 85%, at least 90%, at least 95% or 100% homologous or complementary to the target gene promoter; the two nucleotides at the 5' end and at the 3' end of the sense oligonucleotide strand are all chemically modified nucleotides, and the antisense oligonucleotide strand comprises at least one chemically modified nucleotide. In a specific embodiment, the chemical modification is selected from one or more of the following modifications: (1) chemical modification of nucleotide ribose(s); (2) chemical modification to inter-nucleotide phosphodiester bond(s); (3) chemical modification of nucleotide base(s); (4) inclusion of locked nucleic acid(s); and (5) modification with vinylphosphonate at 5'-end nucleotide(s).

Another object of the present disclosure is to provide a small activating RNA for human $p21^{WAF1/CIP1}$ gene (P21 gene). The p21 gene is a member of the Clp family, which is a cyclindependent kinase inhibitor located downstream of the p53 gene. p21 and p53 can jointly constitute the G1 checkpoint of the cell cycle. As a cell with unrepaired DNA damage cannot pass the checkpoint, the replication and accumulation of damaged DNA are reduced, and thus cancer is suppressed. p21 is not only closely related to tumor suppression, but also can coordinate the relationship between cell cycle, DNA replication and repair by inhibiting the activity of cyclin -dependent kinase (CDK) complex, so as to link between tumor suppression and cell cycle control. p21 is associated with tumor differentiation, depth of infiltration, proliferation and metastasis.

The present disclosure provides a nucleic acid molecule comprising a segment encoding the small activating RNA. The present disclosure also provides a cell comprising the small activating RNA or the nucleic acid molecule as described.

The present disclosure provides a pharmaceutical composition comprising the small activating RNA, or the nucleic acid molecule, and optionally, a pharmaceutically acceptable carrier. The present disclosure also provides a preparation comprising the small activating RNA, or the nucleic acid molecule, or the cell, or the pharmaceutical composition as described. And, also provided is a kit comprising the small activating RNA, or the nucleic acid molecule, or the cell, or the pharmaceutical composition, or the preparation as described.

The present disclosure further provides a combined pharmaceutical composition, comprising, the small activating RNA, and a small molecule drug. Optionally, the small molecule drug is selected from Mitomycin C or Valrubicin.

The present disclosure further provides use of the small activating RNA, or the nucleic acid molecule, or the cell, or the pharmaceutical composition, or the preparation as described in the manufacture of a medicine or formulation for activating or up-regulating the expression of p21 gene in cells. In some embodiments, the use can be for the preparation of medicines or formulations for preventing, treating or alleviating malignant tumors or benign proliferative lesions. In some embodiments, the malignant tumor can be bladder cancer, prostate cancer, liver cancer, or colorectal cancer. In some embodiments, the bladder cancer can be non-muscle-invasive bladder cancer (NMIBC).

The present disclosure also provides a method for preventing, treating or alleviating malignant tumors or benign proliferative lesions, comprising, administering the small activating RNA, or the nucleic acid molecule, or the cell, or the pharmaceutical composition, or the preparation to an individual in need thereof. In some embodiments, the malignant tumor is bladder cancer, prostate cancer, liver cancer, or colorectal cancer. In some embodiments, the bladder cancer is non-muscle invasive bladder cancer (NMIBC).

Those skilled in the art can easily perceive other aspects and advantages of the present disclosure from the following detailed description. In the following detailed description, exemplary and illustrative embodiments of the present disclosure are shown and described. As those skilled in the art will understand, the content of the present disclosure enables those skilled in the art to make changes to the specific embodiments which are disclosed without departing from the spirit and scope of the invention. Correspondingly, the drawings and descriptions in the specification are only exemplary and illustrative rather than restrictive.

DESCRIPTION OF THE DRAWINGS

The particular features of the invention are set forth in the appended claims. The features and advantages of the invention can be better understood with reference to the exemplary embodiments described in detail hereinafter and the accompanying drawings. A brief description of the accompanying drawings is as follows.

Figure 1 shows specific patterns of chemically modified sense and antisense strands. Figure 1-A shows an unmodified sense oligonucleotide strand and its four modified derived sequences, each derived sequence has a different modification pattern. Figure 1-B shows an unmodified antisense oligonucleotide strand (AS) and 10 modified derived sequences thereof, each with a different modification pattern.

Figure 2 shows specific patterns of chemically modified dsRNA duplexes. The sense oligonucleotide strands in Figure 1-A and the antisense oligonucleotide strands in Figure 1-B are arranged in a pairwise manner, resulting in 32 different saRNA duplexes.

Figure 3 shows the effect of chemical modification on the stability of dsRNA duplexes in the presence of RNase A. dsRNA (1.5 μL, 10 μM) diluted in 10 μL PBS was incubated with RNase A (final concentration: 0.01 μg/μL) at 37 °C for different durations (lane 1: 0 min; lane 2: 5 min; lane 3: 10 min; lane 4: 15 min; lane 5: 30 min; lane 6: 1 hr). The duplexes obtained at each time point above were each added to 1 μL of 10× loading buffer, and then frozen in liquid nitrogen. After incubation, all samples were detected for the double-stranded RNA by 4% agarose gel electrophoresis, and the content of each duplex was analyzed by Image J software. M represents a 20 bp DNA marker.

Figure 4 shows the effect of chemical modification on the stability of individual RNA duplexes in the presence of RNase A. Rnase A (final concentration: 0.01 μg/μL) was incubated with double-stranded RNA (1.5 μL, 10 μM) diluted in 10 μL PBS at 37 °C for 0, 24, 48 and 96 hours, respectively. The duplexes obtained at each time point indicated above were each added to 1 μL of 10× loading buffer, and then frozen in liquid nitrogen. After incubation, all samples were detected for the double-stranded RNA by 4% agarose gel electrophoresis, and the content of each duplex was analyzed by Image J software. RAG1-40-XX at the top of the figure is the name of the each sample with XX replaced by the number above the sample. M represents a 20 bp DNA marker.

Figure 5 shows the effect of chemical modification on the stability of RNA duplex in human urine. Double-stranded RNA (7.5 μL, 10 μM) diluted in 17.5 μL PBS was incubated with 25 μL human fresh urine at 37 °C for 0, 1, 24 and 48 hours, respectively. The duplexes obtained at each time point indicated above were each added to 1 μL of 10× loading buffer, and then frozen in liquid nitrogen. After incubation, all samples were detected for the double-stranded RNA by 4% agarose gel electrophoresis, and the content of each duplex was analyzed by Image J software. M represents a 20bp DNA marker.

Figure 6 shows the inhibited proliferation of Ku-7-LG cells by structurally optimized saRNAs. Ku-7-LG cells were transfected with the indicated saRNA at 10 nM for 3 days. Figure 6A shows the expression of p21 mRNA in Ku-7-LG cells with a single saRNA; Figure 6B shows the level of inhibition of the proliferation of Ku-7-LG cells with a single saRNA; and Figure 6C shows the luciferase activity in Ku-7-LG cells with a single saRNA. The closer the luciferase value is to the control group, the lower the probability of off-target effects of saRNA is. Control and dsCon2 were blank transfection and double-stranded scramble RNA transfection, respectively. The value of the ordinate represents the mean ± SD of two repeated treatments.

Figure 7 shows that chemically modified saRNA promotes p21 mRNA expression in human bladder cancer cell lines. Ku-7-LG and T24 cells were transfected with the indicated saRNA at a final concentration of 10 nM for 3 days. After transfection, the RNA was extracted with the Qiagen RNeasy kit. After reverse transcription, the ABI 7500 fast real-time PCR system was used for qPCR amplification. The GAPDH gene was amplified as an internal reference. Figure 7A shows that chemically modified saRNA promotes the expression of p21mRNA in Ku-7-LG cells, and Figure 7B shows that chemically modified saRNA promotes the expression of p21mRNA in T24 cells. Dashed line indicates duplex Rag1-40 with no chemical modification in comparison, and the values above the line indicate stronger activity. The control was blank transfection without oligonucleotide. dsCon2 is transfected with a double-stranded scramble RNA. The value of the ordinate represents the mean ± SD of 2 repeated treatments.

Figure 8 shows the inhibitory effect of Rag1-derived sequences on the proliferation of human cancer cell lines. Cells were transfected with the indicated duplexes at a final concentration of 10 nM for 72 hours. After the transfection, the number of viable cells was detected with a CCK8 kit, and plotted as the percentage of the blank control treatment group. Figure 8A shows the inhibitory effect of Rag 1-derived sequences on Ku-7-LG cells, and Figure 8B shows the inhibitory effect of Rag 1-derived sequences on T24 cells. A transfection sample without oligonucleotide served as a blank control. Non-specific duplex (dsCon) transfected sample served as a negative control. Data are presented as mean ± SD of at least two independent experiments. The values below the dashed line indicate stronger growth inhibitory activity compared to duplex Rag1-40 without chemical modification.

Figure 9 shows that chemical modifications can suppress the immunostimulatory activity of RNA duplexes. Cells were treated with the indicated duplexes at a final concentration of 10 nM or lipopolysaccharide (LPS) as a positive control for 24 hours, respectively. The levels of INF-α and TNF-α in the cell supernatant were detected by ELISA kit. Figure 9A shows the content of INF-α in PBMC cells, and Figure 9B shows the content of TNF-α in PBMC cells. Data represent mean ± SD of two independent experiments. The dashed line indicates the comparison to duplex Rag1-40 without chemical modification. The values below the line indicate suppressed activities. The RNA duplex RAG1-IS-1 known to have strong immunostimulatory activity was used as a positive control (the sequence of which is shown in Table 3).

Figure 10 shows the effect of chemical modification of saRNA on off-target effects. A 26 bp fragment comprising the target site of Rag1-40 was cloned in the antisense orientation into the 3' UTR region of the firefly luciferase gene in a pmirGLO dual luciferase vector. Vector plasmids were co-transfected with the indicated saRNAs into COS-1 cells. Cells were lysed 48 hours after transfection, and the resulting lysates were assessed for luciferase activity. Data represent mean ± SD of at least two independent experiments. Dashed line represents comparisons to blank control (top dashed line) and Rag1-40 (bottom dashed line). Values close to the top dashed line indicate no off-target effect. Values above and below the bottom dashed line represent reduced and increased off-target effects compared to Rag1-40 without chemical modification.

Figure 11 shows that chemically modified saRNA duplexes retain their activation activity in bladder cancer cells treated for different durations. Figure 10A shows the expression level of p21 mRNA in Ku-7-LG cells after being treated with RAG1-40-31 and RAG1-40-53 at 10 nM in for 1, 2, 3, 4, 7 and 9 days, respectively. Figure 10B shows the expression level of p21 mRNA in T24 cells after being treated with RAG1-40-31 and RAG1-40-53 at 10 nM for 1, 2, 3, 4, 7 and 9 days, respectively. The control and dsCon2 were blank transfection and scramble dsRNA transfection, respectively. The values in the ordinate represent the mean ± SD of 2 repeated treatments.

Figure 12 shows that chemically modified saRNA duplexes inhibit cellular proliferation in Ku-7-LG cells by arresting cell cycle progression. RAG1-40-31 and RAG1-40-53 were transfected into Ku-7-LG cells at the final concentrations of 0.1, 1, 10 and 50 nM, and the transfection duration was 48 hours. Two replicate wells were used for each treatment. After transfection, the cell cycle was analyzed by flow cytometry, and the collected data were analyzed by MotFit software.

Figure 13 shows that chemically modified saRNA duplexes promote cell apoptosis in co-culture of J82 cells and PBMC cells. J82 cells were transfected with RAG1-40-31 and RAG1-40-53 at final concentrations of 0.1, 1, 10 and 25 nM, and 24 hours after transfection, fresh medium was replaced and a certain ratio of PBMC cells was added (J82 cells : PBMC cells = 1:4). The cells were co-cultured for 48 hours. After co-culture, cells were collected and cell apoptosis was detected by flow cytometry. Figures 13A-13B show the relative proliferation proportion of J82 cells after the combined treatment with chemically modified saRNA RAG1-40-31 and Mitomycin C. Figure 13C shows the combination index (CI) of the chemically modified RAG1-40-31 in combination with Mitomycin C. Control and DMSO are the blank transfection control and the solvent for dilution control, respectively.

Figure 14 shows that chemically modified saRNA duplex combined with mitomycin C can suppress the proliferation of J82 cells. J82 cells were transfected with RAG1-40-31 at concentrations of 0.1, 0.5, 1, 5, 10, 25 and 50 nM for 24 hours, and then Mitomycin C at concentrations of 1, 10, 100, 1000 and 10000 nM was added. The combined treatment was carried out with concentrations in pairs. After treatment, the proliferation of cells was detected using CCK8 kit. Figures 14A-14B show the relative proliferation proportion of J82 cells after the combined treatment with chemically modified saRNA RAG1-40-31 and valrubicin. Figure 14C shows the combination index (CI) of the chemically modified RAG1-40-31 in combination with valrubicin. Control and DMSO are the blank transfection control and the solvent for dilution control, respectively.

Figure 15 shows that chemically modified saRNA duplex combined with valrubicin can suppress the proliferation of J82 cells. J82 cells were transfected with RAG1-40-31 at concentrations of 0.1, 0.5, 1, 5, 10, 25 and 50 nM for 24 hours, and then valrubicin at concentrations of 1, 10, 100, 1000 and 10000 nM was added. The combined treatment was carried out with concentrations in pairs. After treatment, the cellular proliferation was detected by CCK8 kit. Figures 15A-15B show the relative proliferation proportion of T24 cells after the combined treatment with chemically modified saRNA RAG1-40-31 and Mitomycin C. Figure 15C shows the combination index (CI) of the chemically modified RAG1-40-31 in combination with Mitomycin C. Control and DMSO are the blank transfection control and the solvent for dilution control, respectively.

Figure 16 shows that chemically modified saRNA duplex combined with mitomycin C can suppress the proliferation of T24 cells. T24 cells were transfected with RAG1-40-31 at concentrations of 0.1, 0.5, 1, 5, 10, 25 and 50 nM for 24 hours, and then Mitomycin C was added at concentrations of 1, 10, 100, 1000 and 10000 nM. The combined treatment was carried out with concentrations in pairs. After treatment, the cellular proliferation was detected by CCK8 kit. Figures 16A-16B show the relative proliferation proportion of T24 cells after the combined treatment with chemically modified saRNA RAG1-40-31 and valrubicin. Figure 16C shows the combination index (CI) of the chemically modified RAG1-40-31 in combination with valrubicin. Control and DMSO are the blank transfection control and the solvent for dilution control, respectively.

Figure 17 shows that chemically modified saRNA duplex combined with valrubicin can suppress the proliferation of T24 cells. T24 cells were transfected with RAG1-40-31 at concentrations of 0.1, 0.5, 1, 5, 10, 25 and 50 nM for 24 hours, and then valrubicin at concentrations of 1, 10, 100, 1000 and 10000 nM was added. The combined treatment was carried out with concentrations in pairs. After treatment, the cellular proliferation was detected by CCK8 kit.

DETAILED DESCRIPTION OF THE INVENTION

The embodiments of the invention will be described in the following specific examples, and those skilled in the art can easily understand other advantages and effects of the invention from the content disclosed in this specification.

The application discloses a small activating RNA for activating a target gene, wherein the small activating RNA is composed of a sense oligonucleotide strand and an antisense oligonucleotide strand, and the sense oligonucleotide strand or the antisense oligonucleotide strand is at least 75%, at least 80%, at least 85%, at least 90%, at least 95% or 100% homologous or complementary to the target gene promoter; the two nucleotides at the 5' end and at the 3' end of the sense oligonucleotide strand are all chemically modified nucleotides, and the antisense oligonucleotide strand comprises at least one chemically modified nucleotide.

The chemical modification described herein can be the alteration, addition, reduction or replacement of atoms/chemical groups at different positions in an oligonucleotide strand, between single nucleotides in the oligonucleotide strand, or single nucleotide residues, or the alteration, addition, reduction or replacement of chemical bonds between atoms, or covalent attachment of groups, ligands, or conjugates to nucleotides at any position in the oligonucleotide strand. In some embodiments, the chemical modification can be selected from one or more of the following modifications:

(1) chemical modification of nucleotide ribose(s);

(2) chemical modification of inter-nucleotide phosphodiester bond(s);
(3) chemical modification of nucleotide base(s);
(4) inclusion of locked nucleic acid(s); and
(5) modification with vinylphosphonate at 5'-end nucleotide(s).

The chemical modification of ribose described herein can be those commonly used in the art, including, for example, the modification of the hydroxyl group (2'-OH) in the pentose of a nucleotide. In some embodiments, the modification of the hydroxyl group in the pentose of a nucleotide can include 2'-fluro (2'F), 2'-O-methyl (2'-OMe), 2'-methoxyethyl (2'-MOE), 2,4'-dinitrophenol modification (2'-DNP), locked nucleic acid (LNA), 2'-amino and the like. Alternatively, in some embodiments, the chemical modification can be substitution of the ribose 2'-OH in a nucleotide with one or more of 2'-fluoro, 2'-O-methyl, 2'-methoxyethyl, 2,4'-dinitrophenol, LNA, 2'-amino, 2'-H. Among them, the substitution of the ribose 2'-OH with 2'-H can change the chemically modified nucleotide from a ribonucleic acid (RNA) to a deoxyribonucleic acid (DNA). Some typical chemical modifications of nucleotides can include, for example, the nucleotides shown below:

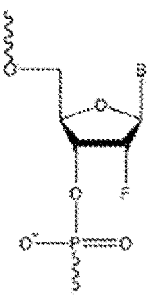
2'-F

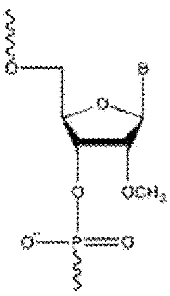
2'-OMe

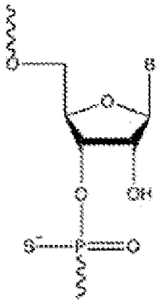
PS

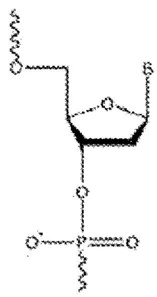
DNA

In some embodiments, the modification of bases may refer to a chemical modification of nucleotide base(s), for example, may include 5'-bromouracil modification, 5'-iodouracil modification, N-methyl uracil modification and/or 2,6-diaminopurine modification. In some embodiments, the modification of phosphodiester bond can be one commonly used in the art, including but not limited to the modification of a non-bridging oxygen atom in the phosphodiester bond. For example, it can include phosphorothioate modification (PS) and/or boranophosphate modification.

In some embodiments, the small activating RNA of the present disclosure can comprise a sense oligonucleotide strand, and the two nucleotides at the 5' end and at the 3' end of the sense oligonucleotide strand can all be chemically modified nucleotides. In some embodiments, the segment between the two nucleotides at the 5' end and the two nucleotides at the 3' end of the sense oligonucleotide strand may comprise no more than 40%, no more than 30%, no more than 20%, no more than 10%, no more than 5% of chemically modified nucleotides, or is completely free of chemically modified nucleotides. In some embodiments, the two nucleotides at the 5' end and/or 3' end of the sense oligonucleotide strand can all be chemically modified nucleotides. In certain cases, the other nucleotides of the sense oligonucleotide strand can all be nucleotides without chemical modification.

In some embodiments, one of the last two phosphodiester bonds at the 3' end of the sense oligonucleotide strand of the small activating RNA of the present disclosure can be chemically modified, or the last two phosphodiester bonds at the 3' end can both be chemically modified. In some embodiments, the last two phosphodiester bonds at the 3' end can be modified independently with sulfur and borane, respectively, to replace the non-bridging oxygen atoms in the phosphodiester bonds. In some embodiments, the last two phosphodiester bonds at the 3' end of the sense oligonucleotide strand can both be modified with sulfur to replace the non-bridging oxygen atom in the phosphodiester bonds, that is, phosphorothioate (PS) modification.

In some embodiments, the sense oligonucleotide strand of the small activating RNA of the present disclosure comprises at least one chemically modified nucleotide. The chemical modification may be a chemical modification of the ribose 2'-OH in nucleotide(s), a chemical modification of inter-nucleotide phosphodiester bond(s), a chemical modification of nucleotide base(s), the replacement of any nucleotide with a LNA, or modification with vinylphosphonate at 5'-end nucleotide(s) of the oligonucleotide. In some embodiments, the sense oligonucleotide strand can comprise at least 2, at least 3, at least 4, at least 5, at least 6, at least 7, at least 8, at least 9, at least 10, at least 11, at least 12, at least 13, at least 14, at least 15, at least 16, at least17, at least 18, at least 19, at least 20, at least 21, at least 22, at least 23, or more chemically modified nucleotides. In some embodiments, the sense oligonucleotide strand can comprise at least 25%, at least 30%, at least 35%, at least 40%, at least 45%, at least 50%, at least 55%, at least 60%, at least 65%%, at least 70%, at least 75%, at least 80%, at least 85%, at least 90%, at least 95%, at least 97%, or 100% of

chemically modified nucleotides. In some embodiments, all nucleotides comprised in the sense oligonucleotide strand can be chemically modified nucleotides.

In some embodiments, one of the last two phosphodiester bonds at the 3' end or 5' end of the antisense oligonucleotide strand of the small activating RNA of the present disclosure can be chemically modified, or both of the last two phosphodiester bonds at 3' end or 5' end can be chemically modified. In some embodiments, the last two phosphodiester bonds at the 3' end and/or 5' end of the antisense oligonucleotide strand can comprise phosphorothioate modifications or boranophosphate modifications or a combination thereof. In some embodiments, both of the last two phosphodiester bonds at the 3' end of the antisense oligonucleotide strand can be phosphorothioate modified. In some embodiments, both of the last two phosphodiester bonds at the 5' end of the antisense oligonucleotide strand can be phosphorothioate-modified. In some embodiments, all of the last two phosphodiester bonds at the 5' end and the 3' end of the antisense oligonucleotide strand can be phosphorothioate modified.

In some embodiments, the antisense oligonucleotide strand of the small activating RNA of the present disclosure can comprise at least one (for example, at least 2, at least 3, at least 4, at least 5, at least 6, at least 7 at least 8, at least 9, at least 10, at least 11, at least 12, at least 13, at least 14, at least 15, at least 16, at least 17, at least 18, at least 19, at least 20, at least 21, at least 22, at least 23, or more) chemically modified nucleotides. The chemical modification can be a chemical modification of the ribose 2'-OH in nucleotide(s), including, for example, one or more of 2'-fluoro, 2'-O-methyl, 2'-methoxyethyl, 2,4'-dinitrophenol, LNA, 2'-amino; a chemical modification of inter-nucleotide phosphodiester bond(s), including, for example, phosphorothioate modification or boranophosphate modification; a chemical modification of nucleotide base(s); replacement of any nucleotide with a locked nucleic acid (LNA); or modification with vinylphosphonate at 5'-end nucleotide(s) of the oligonucleotide. In some embodiments, the antisense oligonucleotide strand can comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, or more chemically modified nucleotides. In some embodiments, the antisense oligonucleotide strand can comprise at least 25%, at least 30%, at least 35%, at least 40%, at least 45%, at least 50%, at least 55%, at least 60%, at least 65%, at least 70%, at least 75%, at least 80%, at least85%, at least 90%, at least 95%, at least 97%, or 100% of chemically modified nucleotides. In some embodiments, all nucleotides comprised in the antisense oligonucleotide strand can be chemically modified nucleotides.

In some embodiments, 1-56 nucleotides at the 5'-end of the antisense oligonucleotide strand can have a certain proportion of the ribose 2'-OH modification, for example, at least 2, at least 3, at least 4, at least 5 or more 2'-fluoro modifications. In some embodiments, 1-6 nucleotides at the 3'-end of the antisense oligonucleotide strand can have a higher proportion of chemical modification of the ribose 2'-OH, for example, at least 3, at least 4, at least 5, at least 6 or more 2'-fluoro modifications. The inventors found that the 2'-fluoro modification at these positions may be critical for stability.

In some embodiments, the most central 1-5 nucleotides of the antisense oligonucleotide strand can have a higher proportion of modification of the ribose 2'-OH, for example, at least 1, at least 2, at least 3, at least 4, at least 5 or more nucleotides of the most central nucleotides can have a chemical modification of the ribose 2'-OH.

In some embodiments, the antisense oligonucleotide strand can comprise a combination of three modifications: 2'-fluoro, 2'-O-methyl and phosphorothioate (PS) modifications. In some embodiments, the sense oligonucleotide strand can comprise a combination of three modifications: 2'-fluoro, 2'-O-methyl and phosphorothioate (PS) modifications. In some embodiments, the small activating RNA duplex can comprise a combination of three modifications: 2'-fluoro, 2'-O-methyl and phosphorothioate (PS) modification.

The inventors have found that the combination of the three modifications composed of 2'-fluoro, 2'-O-methyl and phosphorothioate (PS) modifications (the combination of the three) can increase the ability of small activating RNA duplexes to induce gene expression; in some embodiments, the combination of the three can increase the ability of the small activating RNA to induce p21 gene expression; in some embodiments, the combination of the three can increase the effect of the small activating RNA of suppressing the proliferation of cancer cells; in some embodiments, the cancer cells can include bladder cancer cells.

In some embodiments, the antisense oligonucleotide strand does not comprise modifications in which ribonucleotides can be replaced with deoxyribonucleotides (DNA). The inventors have found that RNA-to-DNA modification in the oligonucleotide strand can attenuate the cellular proliferation-suppressing activity of small activating RNA duplexes. In certain cases, modifications that convert RNA to DNA in the oligonucleotide strand should be avoided.

In some embodiments, when a majority (e.g., at least 50%, at least 60%, at least 70%, at least 80%, or at least 90%) or all of the nucleotides of the antisense oligonucleotide strand are chemically modified (for example, with 2'-fluoro and/or 2'-O-methyl), the sense oligonucleotide strand can comprise at least 1, at least 2, at least 3, at least 5 or more of 2'-O-methyl modifications. The inventors have found that chemically modifying most or all of the nucleotides in the antisense oligonucleotide strand can have a negative impact on the activity of the small activating RNA (such as p21-targeting saRNAs) to inhibit cellular proliferation. The inventors have also found that the negative impact on the suppression of cellular proliferation activity brought about by modifying most or all of the nucleotides of the antisense

oligonucleotide strand can be offset or corrected by 2'OMe modifications of the sense oligonucleotide strand.

Through exploration of innumerable permutations and combinations of different types of chemical modifications at each nucleotide position of the sense and antisense oligonucleotide strands of the small activating RNA, the inventors have found, surprisingly, that the chemical modifications to the sense oligonucleotide strand and the antisense oligonucleotide strand of the small activating RNA disclosed herein can make the small activating RNA of the present disclosure exhibit better pharmaceutical properties, including, for example, stability of saRNA duplex, lower immune stimulation and less off-target effects; more importantly, these modifications can also improve the efficacy of saRNA-induced RNA activation. In some embodiments, the efficacy of saRNA-induced RNA activation of a target gene can be at least 1. 1-fold, at least 1.2-fold, at least 1.3-fold, at least 1.5-fold, at least 1.6-fold, at least 1.8-fold, At least 2-fold, at least 2.5-fold, at least 3-fold, at least 4-fold, at least 5-fold, at least 6-fold, at least 8-fold, at least 10-fold, at least 12-fold, at least 15-fold, or higher, compared with a baseline level of expression of the gene in a control group or cell. In some embodiments, the target gene described in the present disclosure may be human or mouse $p21^{WAF1/CIP1}$ gene (P21 gene). In some embodiments, the target gene described in the present disclosure can be human $p21^{WAF1/CIP1}$ gene, and by using the small activating RNA the sense oligonucleotide strand and the antisense oligonucleotide strand of which are chemically modified as described herein, the efficacy of saRNA-induced RNA activation of the p21 gene can be at least 1.1-fold, at least 1.5-fold, at least 2-fold, at least 5-fold, at least 10-fold, or higher compared with the baseline level of expression of the gene in a control group or cell.

Through exploration of innumerable permutations and combinations of different types of chemical modifications at each nucleotide position of the sense and antisense oligonucleotide strands of the small activating RNA, the inventors have found, more surprisingly, that in some embodiments, the small activating RNAs described herein can stimulate enhanced immune activity.

In some embodiments, the chemical modification of the sense oligonucleotide strand can be selected from the group consisting of four patterns: RAG1-SS-S6A, RAG1-SS-S6B, RAG1-SS-S6C, and RAG1-SS-S6D.

In some embodiments, the chemical modification of the antisense oligonucleotide strand can be selected from the group consisting often patterns: RAG1-SS-AS2A, RAG1-SS-AS2B, RAG1-SS-AS2C, RAG1-SS-AS2D, RAG1-SS-AS2E, RAG1-SS-AS2F, RAG1-SS- AS2G, RAG1-SS-AS2H, RAG1-SS-AS2I, and RAG1-SS-AS2J.

In some embodiments, the chemical modification of the sense oligonucleotide strand can be selected from the group consisting of four patterns: RAG1-SS-S6A, RAG1-SS-S6B, RAG1-SS-S6C, and RAG1-SS-S6D, and the chemical modification of the antisense oligonucleotide strand can be selected from the group consisting often patterns: RAG1-SS-AS2A, RAG1-SS-AS2B, RAG1-SS-AS2C, RAG1-SS-AS2D, RAG1-SS-AS2E, RAG1-SS-AS2F, RAG1-SS-AS2G, RAG1-SS-AS2H, RAG1-SS-AS2I, and RAG1-SS-AS2J.

In some embodiments, the chemical modification of the sense oligonucleotide strand can be the pattern set forth in RAG1-SS-S6D, and the chemical modification of the antisense oligonucleotide strand can be the pattern set forth in RAG1-SS-AS2G.

In some embodiments, either or both of the sense oligonucleotide strand or the antisense oligonucleotide strand of the small activating RNA of the present disclosure can have an overhang of 6, 5, 4, 3, 2, 1 or 0 nucleotides, which can be located at the respective 5' end and/or 3' end of the sense oligonucleotide strand and/or the antisense oligonucleotide strand.

In some embodiments, the 3' end of the antisense oligonucleotide strand can have an overhang of 0-6 nucleotides. In some embodiments, the 3' end of the antisense oligonucleotide strand can have an overhang of 2 or 3 nucleotides. In some embodiments, the 3' end of the sense oligonucleotide strand and the 5' end of the antisense oligonucleotide strand do not have an overhang, or are of blunt ends. In some embodiments, the overhang can be dTdT or dTdTdT, or of nucleotide(s) identical or complementary to the nucleotide(s) at the corresponding position(s) of the target gene, or neither identical nor complementary to the nucleotide(s) at the corresponding position(s) of the target gene. In some embodiments, the overhang can be nucleotide(s) that is/are complementary to nucleotide(s) at a corresponding position(s) on the target gene.

The length of the sense oligonucleotide strand and the antisense oligonucleotide strand of the small activating RNA of the present disclosure can vary depending on different target genes, different positions of action of target genes, or different activation effects brought about by different lengths. In some embodiments, the sense oligonucleotide strand and the antisense oligonucleotide strand can each have 17-30 nucleotides in length, for example, 17, 18, 19, 20, 21, 22, 23, 24, 25, 26, 27, 28, 29, or 30 nucleotides in length. In some embodiments, the sense oligonucleotide strand and the antisense oligonucleotide strand form a duplex region of base complementarity comprising at least 10, at least 11, at least 12, at least 13, at least 14, or at least 15 nucleotides in length, for example, 10, 11, 12, 13, 14, 15, 16, 17, 18, 19, 20, 21, 22, 23, 24, 25, 26, 27, 28, 29, or 30 base-paired nucleotide pairs.

In some embodiments, there can be 1-3 nucleotide mismatches in the sense oligonucleotide strand and the antisense oligonucleotide strand. In some embodiments, there can be 1-3 nucleoside mismatches in the 3' or 5' region of the sense oligonucleotide strand and the corresponding 5' or 3' region of the antisense oligonucleotide strand, for example, there can be 1, 2, or 3 nucleotide mismatches in the nucleotides in the 3' region of the sense oligonucleotide

strand and the nucleotides in the corresponding 5' region of the antisense oligonucleotide strand. In some embodiments, there can be 1-3 nucleoside mismatches in the nucleotides in the corresponding middle region of the sense oligonucleotide strand and the antisense oligonucleotide strand other than the 5' or 3' end nucleotides.

In some embodiments, there can be 1-3 nucleotide mismatches between the 3' or 5' end of the sense oligonucleotide strand and the corresponding nucleotides on the target gene. In some embodiments, there can be 1-3 nucleotide mismatches between the 3' or 5' end of the antisense oligonucleotide strand and the corresponding nucleotides on the target gene. In some embodiments, there can be 1, 2, or 3 nucleotide mismatches between the 3' end of the sense oligonucleotide strand and the corresponding nucleotides on the target gene.

In some embodiments, the sense oligonucleotide strand or the antisense oligonucleotide strand of the small activating RNA of the present disclosure can be conjugated with one or more groups selected from the group consisting of: lipid, fatty acids, fluorescent groups, ligands, sugars, polymers, polypeptides, antibodies, and cholesterol. The conjugation can be at the 3' end or the 5' end of the sense oligonucleotide strand or the antisense oligonucleotide strand, or between the 3' end and the 5' end. In some embodiments, the sense oligonucleotide strand or the antisense oligonucleotide strand of the small activating RNA of the present disclosure can be conjugated with at least one lipid group, at the 3' end or 5' end of the oligonucleotide strand. In some embodiments, the lipid group can be one or more selected from fatty acid (fatty acyl), cationic lipid, anionic lipid, ionizable lipid, saccharolipid, glycerolipid, glycerophospholipid, sterol lipid, sphingolipid, prenol lipid, and polyketide. In some embodiments, the sense oligonucleotide strand or the antisense oligonucleotide strand of the small activating RNA of the present disclosure can be conjugated with at least one cholesterol group, which, for example, can be at the 3' end or 5' end of the oligonucleotide strand. In some embodiments, the sense oligonucleotide strand or the antisense oligonucleotide strand of the small activating RNA of the present disclosure can be conjugated with at least one sugar group, which, for example, can be at the 3' end or 5' end of the oligonucleotide strand. The sugar group includes, for example, N-acetyl galactosamine (GalNAc), glucose, mannose and other suitable sugar groups. In some embodiments, the conjugation of one or more groups can make the small activating RNA of the present disclosure exhibit better ability to enter into specific organs, tissues or cells, and/or enable the small activating RNA of the present disclosure to possess desired pharmaceutical properties, such as pharmacokinetics (pK), pharmacodynamics (pD), toxicity, and the properties in terms of the body's absorption, distribution, metabolism and excretion of exogenous chemicals.

The present disclosure discloses a small activating RNA, and the target gene of the small activating RNA can be human $p21^{WAF1/CIP1}$ gene. In particular, in some embodiments, the small activating RNA can specifically target the promoter region of the p21 gene. In some embodiments, the small activating RNA can specifically target the region from -1000 nucleotides upstream of the transcription start site (TSS) of the p21 gene promoter to the TSS.

In some embodiments, the sense oligonucleotide strand of the small activating RNA targeting p21 can be 17-30 nucleotides in length, and/or the antisense oligonucleotide strand can be 17-30 nucleotides in length. For example, the sense oligonucleotide strand of the small activating RNA can have 17, 18, 19, 20, 21, 22, 23, 24, 25, 26, 27, 28, 29, or 30 nucleotides in length; and/or, the antisense oligonucleotide strand length of the small activating RNA can have 17, 18, 19, 20, 21, 22, 23, 24, 25, 26, 27, 28, 29, or 30 nucleotides in length; the length of the sense oligonucleotide strand of the small activating RNA can be the same as or different from that of the antisense oligonucleotide strand.

In some embodiments, the sense oligonucleotide strand of the present disclosure, being p21-targeting, can have a segment of at least 15 nucleotides in length to form base complementarity with the antisense oligonucleotide strand. For example, the nucleotide segment in the sense oligonucleotide strand or the antisense oligonucleotide strand that is sequence matching with the target gene promoter can have 15, 16, 17, 18, 19, 20, 21, 22, 23, 24, 25, 26, 27, or 28 nucleotides in length. In some embodiments, the 3' end of the antisense oligonucleotide strand can have an overhang of 2 nucleotides, at least one of which can be complementary to the target gene promoter. In some embodiments, the 3' end of the antisense oligonucleotide strand can have an overhang of 1, 2, or 3 nucleotides, which, for example, can all be complementary to the target gene promoter. In some embodiments, there can be a mismatch of 1 nucleotide between the 3' region of the sense oligonucleotide strand and the coding strand of the target gene promoter sequence. In some embodiments, there can be one nucleotide mismatch between the 3' nucleotides of the sense oligonucleotide strand and the nucleotides at the corresponding positions in the coding strand of the target gene promoter. In some embodiments, there can be one nucleotide mismatch between the 3' end nucleotides of the sense oligonucleotide strand and the 5' end nucleotides of the antisense oligonucleotide strand. In some embodiments, there can be one nucleotide mismatch between the 5' end nucleotides of the sense oligonucleotide strand and the 3' end nucleotides of the antisense oligonucleotide strand.

In some embodiments, the sequence of the sense oligonucleotide strand of the small activating RNA targeting p21 can be selected from the group consisting of SEQ ID NOs: 5, 14, 15, and 16. In some embodiments, the sequence of the antisense oligonucleotide strand of the small activating RNA targeting p21 can be selected from the group consisting of SEQ ID NOs: 6, 8, 11, 12, 13, 18, and 19. In some embodiments, the sequences of the sense oligonucleotide strand and the antisense oligonucleotide strand of the small activating RNA targeting p21 can be selected from the paired sequences listed in Table 2.

The p21 gene-targeting small activating RNA provided in the present disclosure can be suitable for to all the different types of chemical modifications carried out on the small activating RNA, so that the p21 gene-targeting small activating RNA can exhibit improved pharmaceutical properties include, for example, stability of the saRNA duplex, lower immune stimulation and less off-target effects; more importantly, these modifications also improve the efficacy of saRNA-induced RNA activation. In some embodiments, the chemical modification of the p21 gene-targeting small activating RNA may need to maintain a certain immunostimulatory activity, for example, an immunostimulatory activity sufficient to cause suppression of cancer cell proliferation, such as suppression of the proliferation of bladder cancer cells.

In some embodiments, the antisense oligonucleotide strand targeting small activating RNA of p21 gene can comprise at least one chemically modified nucleotide. The chemical modification can be a chemical modification of the ribose 2'-OH in nucleotide(s), including, for example, one or more of 2'-fluoro, 2'-O-methyl, 2'-methoxyethyl, 2,4'-dinitrophenol, LNA, and 2'-amino modifications; a chemical modification of inter-nucleotide phosphodiester bond(s), including, for example, phosphorothioate modification or boranophosphate modification; a chemical modification of nucleotide base(s); the replacement of any nucleotide with a LNA; or modification with vinylphosphonate at 5'-end nucleotide(s) of the oligonucleotide. In some embodiments, the antisense oligonucleotide strand comprises 2, 3, 4, 5, 6, 7, 8, 9, 10, 11, 12, 13, 14, 15, 16, 17, 18, 19, 20, 21, 22, 23, or more chemically modified nucleotides. In some embodiments, the antisense oligonucleotide strand comprises at least 25%, at least 30%, at least 35%, at least 40%, at least 45%, at least 50%, at least 55%, at least 60%, at least 65%, at least 70%, at least 75%, at least 80%, at least85%, at least 90%, at least 95%, at least 97%, or 100% of chemically modified nucleotides.

In some embodiments, only part of the nucleotides constituting the antisense oligonucleotide strand of the small activating RNA targeting p21 gene is chemically modified nucleotides, for example, the strand comprises no more than 80%, no more than 75%, no more than 70%, no more than 65%, no more than 60%, no more than 55%, no more than 50%, no more than 45%, no more than 40%, not more than 35%, no more than 30%, no more than 25%, no more than 20%, no more than 15%, no more than 10%, no more than 5%, or no chemically modified nucleotides. The chemical modification can be a chemical modification of the ribose 2'-OH in nucleotide(s), a chemical modification of the phosphodiester bond between the nucleotides, or a chemical modification of nucleotide base(s), or replacement of any nucleotide with a locked nucleic acid (LNA), or modification with vinylphosphonate at 5'-end nucleotide(s) of the oligonucleotide. The inventors have found that when it is desired to improve the small activating RNA to possess a certain immunostimulatory activity, it is suitable to select a lower proportion of chemically modified nucleotides in the antisense oligonucleotide strand, such as lower than 80% or lower than 60%.

In some embodiments, the nucleotides constituting the sense oligonucleotide strand of the small activating RNA targeting p21 gene comprise at least one chemically modified nucleotide. The chemical modification type is the same as that of the antisense oligonucleotide strand. In some embodiments, the sense oligonucleotide strand comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23 or more chemically modified nucleotides. In some embodiments, the sense oligonucleotide strand can comprise at least 25%, at least 30%, at least 35%, at least 40%, at least 45%, at least 50%, at least 55%, at least 60%, at least 65%%, at least 70%, at least 75%, at least 80%, at least 85%, at least 90%, at least 95%, at least 97%, or 100% of chemically modified nucleotides. In some embodiments, all nucleotides constituting the sense oligonucleotide strand are chemically modified nucleotides.

In some embodiments, the nucleotides constituting the sense oligonucleotide strand of the p21 gene-targeting small activating RNA may only partially be chemically modified nucleotides, for example, the strand can comprise no more than 80%, no more than 75%, no more than 70%, no more than 65%, no more than 60%, no more than 55%, no more than 50%, no more than 45%, no more than 40%, not more than 35%, no more than 30%, no more than 25%, no more than 20%, no more than 15%, no more than 10%, no more than 5%, or no chemically modified nucleotides. The type of chemical modification is the same as for the antisense oligonucleotide strand.

Similarly, the inventors have found that when it is desired to improve the small activating RNA with a certain immunostimulatory activity, it is suitable to select a lower proportion of chemically modified nucleotides in the sense oligonucleotide strand, such as lower than 80%, or lower than 60%, or lower than 40%, or lower than 20%.

The inventors have found that the position of chemically modified nucleotides can influence the activity, stability, immunostimulatory, and/or off-target effects of the saRNA. In some embodiments, 1-5 nucleotides at the 5'-end of the antisense oligonucleotide strand can have a certain proportion of chemical modification of the ribose 2'-OH, for example, at least 2, at least 3, at least 4, or 5. In some embodiments, 1-6 nucleotides at the 3'-end of the antisense oligonucleotide strand have a higher proportion of chemical modification of the ribose 2'-OH, for example, at least 3, at least 4, at least 5, or 6. In some embodiments, the most central 1-5 nucleotides of the antisense oligonucleotide strand can have a higher proportion chemical modification of the ribose 2'-OH, for example, the most central one, two, three, four or five nucleotides can have chemical modification of the ribose 2'-OH.

In some embodiments, the small activating RNA targeting p21 comprises a chemical modification of nucleotide ribose(s), for example, a chemical modification of the ribose 2'-OH in nucleotides. In some embodiments, the ribose 2'-OH in at least one nucleotide of the small activating RNA targeting p21 can be substituted by one or more of 2'-fluoro, 2'-O-methyl, 2'-methoxyethyl, 2,4'-dinitrophenol, LNA, and 2'-amino modifications. In some embodiments, the chemical modification of at least one phosphodiester bond between the nucleotides of the small activating RNA targeting p21 can be a modification of the inter-nucleotide phosphodiester bond(s), including, for example, phosphorothioate modification, or boranophosphate modification, or combination thereof.

In some embodiments, one of the last two phosphodiester bonds at the 3' end of the sense oligonucleotide strand of the p21-targeting small activating RNA described herein is chemically modified, or both of the last two phosphodiester bonds at the 3' end are chemically modified. The modification of the phosphodiester bond of the sense oligonucleotide strand of the small activating RNA targeting p21 is commonly used in the art, including but not limited to modifying the non-bridging oxygen atom in the phosphodiester bond, such as phosphorthioate (PS) modification and boranophosphate modification. In some embodiments, the last two phosphodiester bonds at the 3' end can be replaced with sulfur and borane for the non-bridging oxygen atoms in the phosphodiester bonds, respectively. In some embodiments, the last two phosphodiester bonds at the 3' end of the sense oligonucleotide strand can be modified with sulfur in place of the non-bridging oxygen atom in the phosphodiester bond, that is, phosphorothioate (PS) modification. The inventors have found that the gene activation efficiency of the small activating saRNA can be improved when both of the last two phosphodiester bonds at the 3' end of the sense oligonucleotide strand comprise phosphorthioate modification, or boranophosphate modification, or combination thereof. In some embodiments, the activity of small activating RNA duplexes in suppressing cellular proliferation can be enhanced when both of the last two phosphodiester bonds at the 3' end of the sense oligonucleotide strand comprise phosphorthioate modification, or boranophosphate modification, or combination thereof. In some embodiments, the small activating RNA can target the p21 gene. In some embodiments, the cells can be cancer cells. In some embodiments, the cancer cells can include bladder cancer cells.

In some embodiments, one or both of the last two phosphodiester bonds at the 3' end of the antisense oligonucleotide strand of the p21-targeting small activating RNA of the present disclosure is chemically modified. In some embodiments, the last two phosphodiester bonds at the 3' end of the p21-targeting antisense oligonucleotide strand can comprise phosphorthioate modification, or boranophosphate modification, or combination thereof. In some embodiments, both of the last two phosphodiester bonds at the 3' end of the p21-targeting antisense oligonucleotide strand can be phosphorothioate modified. In some embodiments, both of the last two phosphodiester bonds at the 5' end and the 3' end of the antisense oligonucleotide strand of the p21-targeting small activating RNA can comprise phosphorothioate modification. In some embodiments, the antisense oligonucleotide strand targeting p21 has a higher proportion ribose 2'-OH modification for the most central 1-3 nucleotides, for example, for the most central 1, 2, or 3 nucleotides.

In some embodiments, the last two phosphodiester bonds at the 3' end of the sense oligonucleotide strand of the small activating RNA targeting p21 can be substituted by phosphorothioate bonds, and the two nucleotides at the 5' end and at 3' end of the sense oligonucleotide strand of the small activating RNA can be chemically modified with 2'-fluoro.

In some embodiments, one of the last two phosphodiester bonds at the 3' end of the antisense oligonucleotide strand of the small activating RNA targeting p21 can be substituted with a phosphorothioate bond, and the one of the last two phosphodiester bonds at the 5' end can be substituted by a phosphorothioate bond, and three of the five nucleotides at the 5' end can be chemically modified with 2'-fluoro or 2'-O-methyl, and three of six nucleotides at the 3' end can be chemically modified with 2'-fluoro or 2'-O-methyl.

In some embodiments, the last two phosphodiester bonds at the 3' end of the antisense oligonucleotide strand of the small activating RNA targeting p21 can both be substituted with phosphorothioate bonds, and both of the last two phosphodiester bonds at the 5' end can be substituted with phosphorothioate bonds, and three of five nucleotides at the 5' end can be chemically modified with 2'-fluoro, and three of six nucleotides at the 3' end can be chemically modified with 2 '-fluoro. In some embodiments, the 3$^{rd}$, 4$^{th}$, and 5$^{th}$ nucleotides at the 5' end are chemically modified with 2'-fluoro, and the 2$^{nd}$, 5$^{th}$, and 6$^{th}$ nucleotides at the 3' end can be chemically modified with 2 '-fluoro.

In some embodiments, the chemical modification pattern of the sense oligonucleotide strand of the p21-targeting small activating RNA described in the present disclosure can be selected from Figure 1A. In some embodiments, the chemical modification pattern of the antisense oligonucleotide strand of the p21-targeting small activating RNA described in the present disclosure can be selected from Figure 1B. In some embodiments, the chemical modification pattern of the sense oligonucleotide strand and the antisense oligonucleotide strand of the p21-targeting small activating RNA described in the present disclosure can be selected from Figure 2.

The method for preparing the small activating RNA of the present disclosure can be modified or improved on the basis of the conventional methods in the art. In some embodiments of preparing small activating RNAs, the method can substantially include the following steps: 1) using the coding strand of the target gene promoter sequence as a template, selecting a sequence comprising 17 to 28 bases as the target site; 2) synthesizing an RNA sequence corresponding to the target site sequence obtained in step 1) as a basic sequence to obtain a sense oligonucleotide strand; 3) synthesizing an antisense oligonucleotide strand with a length of 17 to 30 nucleotides, so that the antisense oligonucleotide strand comprises a segment of at least 15 nucleotides that is complementary to the sense oligonucleotide strand

obtained in step 2); 4) mixing the sense oligonucleotide strand obtained in step 2) with the same mole number of the antisense oligonucleotide strand obtained in step 3) in an RNA annealing buffer, before heating and then being naturally cooling to room temperature to obtain a double-stranded small activated RNA.

The present disclosure also provides a nucleic acid molecule comprising a fragment encoding the small activating RNA. Also provided is a cell comprising the small activating RNA or the nucleic acid molecule.

Optionally, the present disclosure also provides a nucleic acid molecule comprising a segment encoding the small activating RNA targeting p21 gene. Also provided is a cell comprising the small activating RNA targeting p21 gene or the nucleic acid molecule.

The present disclosure further provides a pharmaceutical composition comprising: the small activating RNA, or the nucleic acid molecule, and optionally, a pharmaceutically acceptable carrier. Likewise, provided herein is a preparation comprising the small activating RNA, or the nucleic acid molecule, or the cell, or the pharmaceutical composition described herein. And, also provided is a kit comprising the small activating RNA, or the nucleic acid molecule, or the cell, or the pharmaceutical composition, or the preparation described herein.

Optionally, the pharmaceutical composition can be a p21 gene-targeting pharmaceutical composition, comprising a p21 gene-targeting small activating RNA, a nucleic acid molecule, and optionally, a pharmaceutically acceptable carrier. Optionally, the preparation can be a p21 gene-targeting preparation, comprising the p21 gene-targeting small activating RNA, the nucleic acid molecule, the cell, or the pharmaceutical composition described herein. Optionally, the kit can comprise the p21 gene-targeting small activating RNA, nucleic acid molecule, cell, pharmaceutical composition, or preparation described herein.

The pharmaceutically acceptable carriers described herein may include lipid-based encapsulation systems used in the field of small nucleic acid active molecules such as lipid nanoparticles (LNP), carbohydrate receptor-targeting conjugates comprising, for example, N-acetylgalactosamine (GalNAc), glucose, mannose, and other suitable carbohydrate moieties, covalent conjugates with different lipid or polypeptide moieties as ligands. Choosing a suitable carrier can not only assist small nucleic acid molecules to access the organ or tissue of interest, but also assist small nucleic acid molecules to enter target cells and even subcellular structures, such as assisting double-stranded siRNA or antisense oligonucleotides strand (ASO) to enter the cytoplasm, or saRNA molecules to enter the nucleus, and then achieve the biological regulation with the corresponding small nucleic acid molecules.

Among various delivery technologies, lipid nanoparticles (LNPs), which can generally be composed of four components: ionizable lipids, cholesterol, phospholipids, and PEG, can be used to efficiently encapsulate oligonucleotides and protect them from nuclease digestion. Using ionizable lipids DLin-MC3-DMA (one of the most commonly used cationic lipids for making LNPs) or DLin-KC2-DMA, lipid nanoparticles can successfully deliver oligonucleotides to liver after intravenous injection. However, the LNP formulation, process and limited target organs (liver is specific, and the delivery efficiency is in the order of liver> spleen> kidney, only negligible accumulation in duodenum, lung, heart, and brain) can limit the application of LNP in pharmaceutical compositions.

The p21 gene-targeting small activating RNA described herein can form a preparation or a pharmaceutical composition with lipid nanoparticle (LNP) as needed. The p21 gene-targeting small activating RNA carried by LNP can be delivered to liver to treat liver cancer, or delivered to colorectum, prostate, or bladder by systemic or local administration to treat colorectal cancer, prostate or bladder cancer. For the composition of RNA molecule and LNP, its formulation, and its preparation method, reference can be made to the existing publications in this field, for example, US20190240354A1, US20190336452A1, US20160038612A1, etc. In some embodiments, the present disclosure provides a preparation comprising the p21 gene-targeting small activating RNA and LNP, which can be administered locally in bladder to treat bladder cancer. In some embodiments, the present disclosure provides a preparation comprising the p21 gene-targeting small activating RNA and LNP, which can be used to treat bladder cancer by intravesical infusion. In some embodiments, the present disclosure provides a preparation comprising the p21 gene-targeting small activating RNA and a DLin-KC2-DMA-based LNP, which can be used to treat bladder cancer by intravesical infusion. In some embodiments, the present disclosure provides a preparation comprising the p21 gene-targeting small activating RNA and a DLin-KC2-DMA-based LNP, which can be used to treat non-muscle-invasive bladder cancer (NMIBC) by intravesical infusion.

The present disclosure also provides a pharmaceutical composition of combined agents, comprising: the small activating RNA, and a small molecule drug. Optionally, the small molecule drug is selected from Mitomycin C or Valrubicin. At a certain concentration, the small activating RNA described in the present disclosure can produce a synergistic effect with the described small molecule drug such as Mitomycin C or Valrubicin, showing a stronger suppression on proliferation of bladder cancer cells.

The present disclosure also provides use of the small activating RNA, or the nucleic acid molecule, or the cell, or the pharmaceutical composition, or the pharmaceutical composition of combined agents, or the preparation in the manufacture of a medication or formulation for activating or up-regulating the expression of p21 gene in cells. In some embodiments, the use can be for the manufacture of the medication or formulation for preventing, treating or alleviating malignant tumors or benign proliferative lesions. In some embodiments, the malignancy may include bladder cancer,

prostate cancer, liver cancer, or colorectal cancer. In some embodiments, the bladder cancer may comprise non-muscle invasive bladder cancer.

The present disclosure also provides the use of the small activating RNA, or the nucleic acid molecule, or the cell, or the pharmaceutical composition, or the pharmaceutical composition of combined agents, or the preparation in the manufacture of a medicament with immunostimulatory activity.

The present disclosure also provides a method for preventing, treating or alleviating malignant tumors or benign proliferative lesions, comprising administering the small activating RNA, or the nucleic acid molecule, or the cell, or the pharmaceutical composition, or the pharmaceutical composition of combined agents, or the preparation, to an individual in need thereof. In some embodiments, the malignancy may include bladder cancer, prostate cancer, liver cancer, or colorectal cancer. In some embodiments, the bladder cancer may comprise non-muscle invasive bladder cancer (NMIBC). In some embodiments, the small activating RNA, or the nucleic acid molecule, or the cell, or the pharmaceutical composition, or the pharmaceutical composition of combined agents, or the preparation can all be p21 gene-targeting.

In summary, chemical modifications can improve the pharmaceutical properties of saRNA oligonucleotides. Optimized chemical modifications can increase the stability of saRNA duplexes, suppress their immune stimulation, and mitigate off-target effects. Most importantly, these modifications also increase the efficacy of saRNA-induced RNA activation. The combination of chemically modified small activating RNA and other small molecule chemotherapeutic drugs can exhibit stronger anti-tumor activity.

The invention is further illustrated through specific description. Unless otherwise expressly defined, all technical and scientific terms used herein have the same meaning as commonly understood by a person of ordinary skill in the art.

As used herein, the singular form "a", "an" and "the" include plural references unless the context clearly dictates otherwise.

## DEFINITIONS

The term "complementarity" or "complementary" as used herein refers to the capability of forming base pairs between two oligonucleotide strands. The base pairs are generally formed through inter-nucleotide hydrogen bonds in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing). "100% complementary (complementarity)" means 100% complementarity, i.e. all nucleotide units of the two strands are hydrogen bonded to each other.

"Complete complementarity", "completely complementary", "100% complementarity" or "100% complementary" means that each nucleotide from the first oligonucleotide strand can form a hydrogen bond with the second oligonucleotide strand in the double-stranded region of the double-stranded oligonucleotide molecule, with no base pair being "mismatched". "Incomplete complementarity" or "incompletely complementary" means that not all of the nucleotides of the two strands are bound to each other pairwise by a hydrogen bond. For example, for two oligonucleotide strands with each of 20 nucleotides in length in the double stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. Substantial complementarity or substantially complementary refers to more than about 79%, about 80%, about 85%, about 90%, about 95% or a higher complementarity.

The term "polynucleotide" refers to nucleotide polymers, and includes but is not limited to single-stranded or double-stranded molecules of DNA, RNA, DNA / RNA hybrids that include polynucleotide chains of deoxyribosyl portions and ribosyl portions that alternate regularly and / or irregularly (i.e., where the alternate nucleotide units have an -OH, then an - H, then an -OH, then a -H, and so on at the 2' position of a sugar portion), and modifications of these types of oligonucleotides, in which various entities or moieties are substituted or linked with nucleotide units at any position and naturally occurring or non-naturally occurring linkages. The oligonucleotide for activating the transcription of a target gene described herein is a small activating nucleic acid molecule (saRNA).

As used herein, the terms "oligonucleotide strand", and "oligonucleotide sequence" can be used interchangeably, referring to a generic term for short nucleotide sequences having less than 30 bases (including nucleotides in deoxyribonucleic acid (DNA) or ribonucleic acid (RNA)). In the present disclosure, the length of the oligonucleotide strand can be any length from 17 to 30 nucleotides.

As used herein, the term "gene" refers to the entire nucleotide sequence required to produce a polypeptide chain or functional RNA. A "gene" can be a gene that is endogenous to the host cell or fully or partially recombinant (e.g., due to the introduction of an exogenous oligonucleotide encoding a promoter and coding sequence or a heterologous promoter adjacent to an endogenous coding sequence into host cells). For example, the term "gene" includes a nucleic acid sequence that can be composed of exons and introns. A proteinencoding sequence is, for example, a sequence comprised of exons in an open reading frame between a start codon and a stop codon. As used herein, a "gene" may inlcude, for example, a promoter sequence such as a promoter, an enhancer and all other sequences known in the art

that control the transcription, expression or activity of another gene, whether or not another gene comprises coding or non-coding sequences. In some cases, for example, "gene" can be used to describe a functional nucleic acid comprising regulatory sequences such as a promoter or an enhancer. Expression of a recombinant gene can be controlled by one or more heterologous regulatory sequences.

As used herein, the term "target gene" can refer to nucleic acid sequences, transgenes, viral or bacterial sequences, chromosomes or extrachromosomal genes that are naturally present in organisms, and/or can be transiently or stably transfected or incorporated into cells and/or chromatins thereof. The target gene can be a protein-coding gene or a non-protein-coding gene (such as a microRNA gene and a long non-coding RNA gene). The target gene generally contains a promoter sequence, and the positive regulation for the target gene can be achieved by designing an saRNA having sequence identity (also called homology) to the promoter sequence, characterized as the up-regulation of expression of the target gene. "Target gene promoter" refers to a non-coding sequence of the target gene. The target gene promoter in the expression "complementary to target gene promoter" refers to the coding strand of the sequence, also referred to as a non-template strand, that is, a nucleic acid sequence having the same sequence as the coding strand of the gene.

As used herein, the term "coding strand" refers to a DNA strand which cannot be used for transcription, and the nucleotide sequence of this strand is the same as that of the RNA produced from transcription (in the RNA, T in DNA is replaced by U). Coding strand is also referred to as sense strand. The coding strand of the double-stranded DNA sequence of the target gene promoter described herein refers to a promoter sequence in the same DNA strand as the DNA coding strand of the target gene.

As used herein, the term "template strand" refers to the other strand complementary with the coding strand in the double-stranded DNA of the target gene, i.e., the strand that, as a template, can be transcribed into RNA, and this strand is complementary with the transcribed RNA (A to U and G to C). In the process of transcription, RNA polymerase binds to the template strand, moves in the 3'→5' direction along the template strand, and catalyzes the synthesis of the RNA in the 5'→3' direction. Template strand is also referred to as antisense strand, or negative strand. The template strand of the double-stranded DNA sequence of the target gene promoter described herein refers to a promoter sequence on the same DNA strand as the DNA template strand of the target gene.

As used herein, the term "identity" or "homology" means that one oligonucleotide strand (sense or antisense strand) of an saRNA has at least about 60% sequence similarity with the coding strand or template strand in a region of the promoter sequence of a target gene.

As used herein, the term "promoter" refers to a nucleic acid sequence that does not encode a protein, and that is spatially associated with a protein-coding or RNA-coding nucleic acid sequence and plays a regulatory role for the transcription of the protein-coding or RNA-coding nucleic acid sequence. Generally, an eukaryotic promoter contains 100 to 5000 base pairs, though this length range is not intended to limit the term "promoter" as used herein. Although the promoter sequence is generally located at the 5' terminus of a protein-coding or RNA-coding sequence, it may also exist in exon and intron sequences.

As used herein, the term "transcription start site" refers to a nucleotide marking the transcription start on the template strand of a gene. A gene can have more than one transcription start site.

As used herein, the term "overhang" refers to non-base-paired nucleotides at the terminus (5' or 3') of an oligonucleotide strand, which is formed by one strand extending out of the other strand in a double-stranded oligonucleotide. A single-stranded region extending out of the 3' terminus and/or 5' terminus of a duplex is referred to as an overhang.

As used herein, the terms "gene activation" or "activating gene" can be used interchangeably, and mean an increase in transcription, translation, expression or activity of a certain nucleic acid, as measured by measuring the transcriptional level, mRNA level, protein level, enzymatic activity, methylation state, chromatin state or configuration, translation level or the activity or state in a cell or biological system of a gene. These activities or states can be determined directly or indirectly. In addition, "gene activation", "activating gene" refers to an increase in activity associated with a nucleic acid sequence, regardless of the mechanism of such activation, for example, such as being regulated by a regulatory sequence, transcribed into RNA, translated into protein, resulting in increased protein expression.

As used herein, the terms "small activating RNA" and "saRNA" can be used interchangeably, and refer to a nucleic acid molecule that can facilitate gene expression and can be composed of a first ribonucleic acid strand (antisense strand, also called antisense oligonucleotide strand) containing a nucleotide sequence having sequence identity to the non-coding nucleic acid sequence (e.g., a promoter or an enhancer) of a target gene and a second ribonucleic acid strand (sense strand, also called sense oligonucleotide strand) containing a nucleotide sequence complementary to the first strand, wherein the first strand and the second strand form a duplex. A small activating RNA can also be composed of a single-stranded RNA molecule that can form a hairpin structure by two complementary regions (first and second regions) within the molecule, wherein the first region contains a nucleotide sequence having sequence identity to the target sequence of a promoter of a gene, and the second region contains a nucleotide sequence which is complementary with the first region. The length of the duplex region of the saRNA molecule is typically about 10 to about 50, about 12

to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45 or about 50 base pairs. In addition, the terms "saRNA" and "small activating RNA" also contain nucleic acids other than ribonucleotide portions, including, but not limited to, modified nucleotides or analogues.

As used herein, the terms "sense strand" and "sense oligonucleotide strand" are interchangeably used and refer to the first ribonucleic acid strand having sequence identity with the coding strand containing the promoter sequence of a target gene in the small activating RNA duplex.

As used in the application, the terms "antisense strand" and "antisense oligonucleotide strand" are interchangeably used and refer to the second ribonucleic acid strand complementary to the sense oligonucleotide strand in the small activating RNA duplex.

As used herein, the term "basic sequence" refers to an RNA sequence generated in a process of preparing a small activating RNA, in which the coding strand of the double-stranded DNA sequence of the target gene promoter is taken as a template, and a sequence comprising 17-30 (e.g., 19) bases is selected as the target site, based on which the RNA sequence synthesized corresponding to the target site sequence is a basic sequence. The basic sequence can be completely consistent with the sequence on the coding strand of the double-stranded DNA sequence of the target gene promoter, and can also have more than 60% homology therewith.

As used in this application, the term "synthesis" refers to the synthesis method of RNA, including any method capable of synthesizing RNA, such as chemical synthesis, in vitro transcription, etc.

As used in this application, the term "derived sequence" refers to an oligonucleotide sequence obtained after a specific oligonucleotide sequence is chemically modified.

Materials and methods

Double-stranded saRNA and transfection: Double-stranded saRNA was chemically synthesized by GenePharm (Shanghai, China) or GeneScript (Nanjing, China).

Cell Culture and Transfection

Human bladder cancer cells Ku-7-LG (ATCC), J82 (ATCC) and T24 (ATCC) were cultured in McCoy medium (Gibco) comprising 10% calf serum (Sigma-Aldrich) and 1% penicillin/Streptomycin (Gibco). Cells were cultured at 37°C under 5% $CO_2$. Ku-7-LG, J82 and T24 cells were plated into 12-well plates at $10 \times 10^4$ cells per well, transfected with the small RNA at a concentration of 10 nM (unless otherwise specified) for three days, using RNAiMax (Invitrogen, Carlsbad, CA) according to the manufacturer's instructions. Two replicate wells were set for each treatment. All saRNA sequences are listed in Table 2.

Two-step RT-qPCR

After transfection, the medium was discarded and to each well 500 μl cell lysate was added and incubated at room temperature for 5 minutes. RNA was extracted with the Qiagen RNeasy kit, and after reverse transcription, qPCR analysis was performed on the ABI 7500 Fast Real-time PCR system (Applied Biosystems). Each sample was amplified in 3 replicate wells. The PCR reaction conditions are shown in Table 5.

**TABLE 5 REVERSE TRANSCRIPTION (RT) REACTION PREPARATION**

| Reagent (RT reaction 1) | Volume |
|---|---|
| 5 × gDNA Eraser buffer | 2 μL |
| gDNA Eraser | 1 μL |
| Total RNA (1 μg) + D.W | 7 μL |
| Final volume | 10 μL |
| 42°C 2 min, stored at 4°C | |
| | |
| Reagent (RT reaction 2) | Volume |
| 5×PrimeScript buffer 2 | 4 μl |

(continued)

| Reagent (RT reaction 2) | Volume |
|---|---|
| Prime Script RT Enzyme mixture | 1 μl |
| RT Primer Mixture | 1 μl |
| RNase-free $dH_2O$ | 4 μl |
| RT Reaction 1 | 10 μl |
| Final volume | 20 μl |
| 37°C 15 min, 85°C 5 sec, stored at 4°C | |

The PCR reaction conditions are: 95°C for 30 seconds, 95°C for 5 seconds, 60°C for 30 seconds, and 40 cycles of amplification. The GAPDH gene was amplified as an internal reference at the same time. p21 was amplified with the p21F1/R1 primer pair, and the specific sequences of the amplification primers are shown in Table 1.

In order to calculate the expression value (Erel) of p21 (target gene) of a saRNA transfection sample relative to the control treatment, Formula 1 is used to input the Ct values of the target gene and the internal reference gene for calculation.

$$E_{rel} = 2^{(CtTm-CtTs)} / 2^{(CtRm-CtRs)} \quad \text{(Formula 1)}$$

wherein, CtTm is the Ct value of the target gene from the control treatment sample; CtTs is the Ct value of the target gene from the saRNA treated sample; CtRm is the Ct value of the internal reference gene from the control treatment sample; and CtRs is the Ct value of the internal reference gene from the saRNA treated sample.

**TABLE 1. PRIMER SEQUENCES FOR QRT-PCR ANALYSIS**

| Primer name | SEQ ID NO | Sequence (5'-3') |
|---|---|---|
| GAPDH F<br>GAPDH R | SEQ ID NO: 1<br>SEQ ID NO: 2 | ATCACCATCTTCCAGGAGCGA<br>TTCTCCATGGTGGTGAAGACG |
| CDKN1A F<br>CDKN1A R | SEQ ID NO: 3<br>SEQ ID NO: 4 | GGAAGACCATGTGGACCTGT<br>GGATTAGGGCTTCCTCTTGG |

Apoptosis detection

J82 cells were transfected with RAG1-40-31 at 0.1, 1, 10 and 25 nM. After 24 hours of transfection, the cells were refreshed with PBMC cell-containing fresh medium. The PBMC and J82 cells were co-cultured for 48 hours, and then the supernatant was discarded and the cells were collected. 500 μL PBS was added to each well for washing and then discarded, before the addition of 100 μL trypsin/well. 900 μL medium was added to stop digestion and the content was transferred to a 1.5 mL centrifuge tube. All the cells were collected through centrifugation (400 rcf, 5 min) and counted (1~2 × $10^5$ cells/100 μL Binding Buffer). 100 μL of 1 × Binding Buffer (B&D Systems, 51-66121E), 5 μL FITC Annexin V (B&D Systems, 51-65874X) and 5 μL PI (B&D Systems, 51-66211E) were added before incubation in dark at room temperature for 15 minutes. After the incubation, 100 μL of 1 × binding buffer was added before blending and transferring to a 96-well plate for analysis with flow cytometer (Beckman Coulter). The sample should be analyzed within 1 hour.

Cell cycle detection

After transfection, the supernatant was discarded to collect cells. To each well 500 μL PBS was added for one wash and then discarded. To each well 100 μL trypsin was further added. 900 μL medium was added to stop digestion before transferring to a 1.5 mL centrifuge tube. All the cells were collected through centrifugation (400 rcf, 5 min) and counted (2 × $10^5$ cells /200 μL PBS). Cold 70% absolute ethanol was added before incubation overnight at 4°C. After

washing twice with PBS, 200 μL Krishan buffer was added before incubation at 4 °C for 1 hour. After washing twice with PBS, the supernatant was discarded and 200 μL PBS was added for cell re-suspension, then the cells were transferred to a 96 -well plate and analyzed by flow cytometer. The collected data were analyzed with MotFit software.

Cellular Proliferation Assay (CCK8)

Cells were plated in a 96-well plate at 2~4×$10^3$ cells/well, cultured overnight, and transfected with the oligonucleotide duplexes. Three days after transfection, CCK8 (Dojindo) was used to detect cellular proliferation following manufacturer's instructions. The experimental steps are briefly described as follows: 10 μL of CCK8 solution was added to each well, incubated at 37 °C for 1 hour, and then the absorbance at 450 nm was measured using a microplate reader.

saRNA stability analysis

saRNA (1.5 μL, 10 μM) was incubated at a final concentration of 0.02 mg/mL in PBS in the presence of RNase A (Amresco, OH, USA) at 37°C for different durations. At the end of the incubation, the mixture was snap frozen in liquid nitrogen to stop nuclease activity. Samples were analyzed on a 4% agarose gel and examined for duplex degradation.

Analysis of off-target effects (plasmid construction and luciferase activity analysis)

A 26bp fragment comprising the Rag1-40 target sequence was cloned in the antisense orientation into a multiple cloning site (MCS) of the 3' untranslated region of firefly luciferase of the pmirGLO dual luciferase vector (Promega, Fitchburg, WI), to obtain pOff-Target plasmid. All oligonucleotide sequences used to generate the inserts are listed in Table 4. Following E. coli transformation and overnight incubation, plasmids for each construct were prepared by standard method. Each plasmid was transfected into COS-1 monkey kidney epithelial cells using Lipofectamine 3000 (Invitrogen, Carlsbad, CA). Subsequently, saRNA was transfected using RNAiMAX. After 24 hours of incubation, COS-1 cells were lysed with 1 × lysis buffer, and luciferase activity was measured using dual luciferase reporter gene assay system (Promega, WI, USA) following manufacturer's instructions.

Immunostimulatory activity assay (PBMC Cytokine Release Assay)

Human peripheral blood mononuclear cells (Allcells, Alameda, CA) were seeded in a 96-well microplate at a density of approximately 10×$10^4$ cells/well. Transfection of dsRNA was performed using RNAiMAX (Invitrogen, Carlsbad, CA) following manufacturer's instructions. Supernatants were collected 24 hours after transfection and immediately assayed for IFN-α and TNF-α production using ELISA kit (R&D Systems). Each treatment group was analyzed in triplicate.

Statistical Analysis

Results are expressed as mean ± standard deviation. GraphPad Prism software (GraphPad Software) was used for one-way analysis of variance followed by Tukey's t test for statistical analysis. The criteria for statistical significance were set as *$p<0.05$, **$p<0.01$ and ***$p<0.001$.

Without wishing to be bound by any particular theory, the following examples are only for illustrating and explaining the fusion protein, preparation method and use of the invention, and the present invention is not limited to the following examples.

EXAMPLES

The present disclosure is further illustrated by the following examples. It should be understood that the following examples are for illustrative purpose only and are not to be construed as limiting this invention in any manner.

**EXAMPLE 1. CHEMICAL MODIFICATION OF SARNA**

As shown in Table 2, Figure 1 and Figure 2, the chemical modifications of saRNA included four types of chemical modifications: 2'-O-methyl (2'Me), 2'-fluoro (2'F), phosphorothioate (PS) modification of phosphodiester bond, and replacement of DNA for ribonucleotide, applied to the sense oligonucleotide strand (RAG1-SS-S6) and antisense oligonucleotide strand (RAG1-SS-AS2) of Rag1-40 saRNA in different combinations, resulting in four sensederived sequences (RAG1-SS-S6A to RAG1-SS-S6D) and ten antisense-derived sequences (RAG1-SS-AS2A to RAG1-SS-AS2J) (Figure 1).

**TABLE 2 CHEMICAL MODIFICATIONS OF SARNA**

| Name | SEQ ID NO | SEQUENCE (5'-3') a-d | Length |
|---|---|---|---|
| Rag1-40-1 | SEQ ID NO 5<br>SEQ ID NO 6 | mCmCAACUCAUUCUCCAAGmUmC<br>fUAfCfUfUGGAGAAfUGAGfUfUGGfCA | 19 nt<br>21 nt |
| Rag1-40-2 | SEQ ID NO 5<br>SEQ ID NO 7 | mCmCAACUCAUUCUCCAAGmUmC<br>fUACUfUGGAGAAfUGAGUfUGGfCA | 19 nt<br>21 nt |
| Rag1-40-3 | SEQ ID NO 5<br>SEQ ID NO 8 | mCmCAACUCAUUCUCCAAGmUmC<br>fUmAfCfUfUmGmGmAmGmAmAfUmGmAmGfUfUmGm GfCmA | 19 nt<br>21 nt |
| Rag1-40-4 | SEQ ID NO 5<br>SEQ ID NO 9 | mCmCAACUCAUUCUCCAAGmUmC<br>fUACUfUGGAGAAfUGAGUfUGG*fC*A | 19 nt<br>21 nt |
| Rag1-40-5 | SEQ ID NO 5<br>SEQ ID NO 10 | mCmCAACUCAUUCUCCAAGmUmC<br>UACUfUGGAGAAfUGAGUfUGG*fC*A | 19 nt<br>21 nt |
| Rag1-40-6 | SEQ ID NO 5<br>SEQ ID NO 11 | mCmCAACUCAUUCUCCAAGmUmC<br>fU*A*fCfUfUGGAGAAfUGAGfUfUGG*fC*A | 19 nt<br>21 nt |
| Rag1-40-7 | SEQ ID NO 5<br>SEQ ID NO 12 | mCmCAACUCAUUCUCCAAGmUmC<br>U*A*fCfUfUGGAGAAfUGAGfUfUGG*fC*A | 19 nt<br>21 nt |
| Ragl-40-8 | SEQ ID NO 5<br>SEQ ID NO 13 | mCmCAACUCAUUCUCCAAGmUmC<br>fUactfUGGAGAAfUGAGfUfUGG*fC*A | 19 nt<br>21 nt |
| Ragl-40-9 | SEQ ID NO 14<br>SEQ ID NO 6 | fCfCAACUCAUUCUCCAAGfUfC<br>fUAfCfUfUGGAGAAfUGAGfUfUGGfCA | 19 nt<br>21 nt |
| Ragl-40-10 | SEQ ID NO 14<br>SEQ ID NO 7 | fCfCAACUCAUUCUCCAAGfUfC<br>fUACUfUGGAGAAfUGAGUfUGGfCA | 19 nt<br>21 nt |
| Ragl-40-11 | SEQ ID NO 14<br>SEQ ID NO 8 | fCfCAACUCAUUCUCCAAGfUfC<br>fUmAfCfUfUmGmGmAmGmAmAfUmGmAmGfUfUmGm GfCmA | 19 nt<br>21 nt |
| Ragl-40-12 | SEQ ID NO 14<br>SEQ ID NO 9 | fCfCAACUCAUUCUCCAAGfUfC<br>fUACUfUGGAGAAfUGAGUfUGG*fC*A | 19 nt<br>21 nt |
| Ragl-40-13 | SEQ ID NO 14<br>SEQ ID NO 10 | fCfCAACUCAUUCUCCAAGfUfC<br>UACUfUGGAGAAfUGAGUfUGG*fC*A | 19 nt<br>21 nt |
| Ragl-40-14 | SEQ ID NO 14<br>SEQ ID NO 11 | fCfCAACUCAUUCUCCAAGfUfC<br>fU*A*fCfUfUGGAGAAfUGAGfUfUGG*fC*A | 19 nt<br>21 nt |
| Ragl-40-15 | SEQ ID NO 14<br>SEQ ID NO 12 | fCfCAACUCAUUCUCCAAGfUfC<br>U*A*fCfUfUGGAGAAfUGAGfUfUGG*fC*A | 19 nt<br>21 nt |
| Ragl-40-16 | SEQ ID NO 14<br>SEQ ID NO 13 | fCfCAACUCAUUCUCCAAGfUfC<br>fUactfUGGAGAAfUGAGfUfUGG*fC*A | 19 nt<br>21 nt |
| Ragl-40-17 | SEQ ID NO 15<br>SEQ ID NO 6 | mCmCAACUCAUUCUCCAAG*U*C<br>fUAfCfUfUGGAGAAfUGAGfUfUGGfCA | 19 nt<br>21 nt |
| Ragl-40-18 | SEQ ID NO 15<br>SEQ ID NO 7 | mCmCAACUCAUUCUCCAAG*U*C<br>fUACUfUGGAGAAfUGAGUfUGGfCA | 19 nt<br>21 nt |
| Ragl-40-19 | SEQ ID NO 15<br>SEQ ID NO 8 | mCmCAACUCAUUCUCCAAG*U*C<br>fUmAfCfUfUmGmGmAmGmAmAfUmGmAmGfUfUmGm GfCmA | 19 nt<br>21 nt |
| Ragl-40-20 | SEQ ID NO 15<br>SEQ ID NO 9 | mCmCAACUCAUUCUCCAAG*U*C<br>fUACUfUGGAGAAfUGAGUfUGG*fC*A | 19 nt<br>21 nt |

(continued)

| Name | SEQ ID NO | SEQUENCE (5'-3') a-d | Length |
|---|---|---|---|
| Ragl-40-21 | SEQ ID NO 15<br>SEQ ID NO 10 | mCmCAACUCAUUCUCCAAG*U*C<br>UACUfUGGAGAAfUGAGUfUGG*fC*A | 19 nt<br>21 nt |
| Ragl-40-22 | SEQ ID NO 15<br>SEQ ID NO 11 | mCmCAACUCAUUCUCCAAG*U*C<br>fU*A*fCfUfUGGAGAAfUGAGfUfUGG*fC*A | 19 nt<br>21 nt |
| Ragl-40-23 | SEQ ID NO 15<br>SEQ ID NO 12 | mCmCAACUCAUUCUCCAAG*U*C<br>U*A*fCfUfUGGAGAAfUGAGfUfUGG*fC*A | 19 nt<br>21 nt |
| Ragl-40-24 | SEQ ID NO 15<br>SEQ ID NO 13 | mCmCAACUCAUUCUCCAAG*U*C<br>fUactfUGGAGAAfUGAGfUfUGG*fC*A | 19 nt<br>21 nt |
| Ragl-40-25 | SEQ ID NO 16<br>SEQ ID NO 6 | fCfCAACUCAUUCUCCAAG*U*C<br>fUAfCfUfUGGAGAAfUGAGfUfUGGfCA | 19 nt<br>21 nt |
| Ragl-40-26 | SEQ ID NO 16<br>SEQ ID NO 7 | fCfCAACUCAUUCUCCAAG*U*C<br>fUACUfUGGAGAAfUGAGUfUGGfCA | 19 nt<br>21 nt |
| Ragl-40-27 | SEQ ID NO 16<br>SEQ ID NO 8 | fCfCAACUCAUUCUCCAAG*U*C<br>fUmAfCfUfUmGmGmAmGmAmAfUmGmAmGfUfUmGm GfCmA | 19 nt<br>21 nt |
| Ragl-40-28 | SEQ ID NO 16<br>SEQ ID NO 9 | fCfCAACUCAUUCUCCAAG*U*C<br>fUACUfUGGAGAAfUGAGUfUGG*fC*A | 19 nt<br>21 nt |
| Ragl-40-29 | SEQ ID NO 16<br>SEQ ID NO 10 | fCfCAACUCAUUCUCCAAG*U*C<br>UACUfUGGAGAAfUGAGUfUGG*fC*A | 19 nt<br>21 nt |
| Ragl-40-30 | SEQ ID NO 16<br>SEQ ID NO 11 | fCfCAACUCAUUCUCCAAG*U*C<br>fU*A*fCfUfUGGAGAAfUGAGfUfUGG*fC*A | 19 nt<br>21 nt |
| Ragl-40-31 | SEQ ID NO 16<br>SEQ ID NO 12 | fCfCAACUCAUUCUCCAAG*U*C<br>U*A*fCfUfUGGAGAAfUGAGfUfUGG*fC*A | 19 nt<br>21 nt |
| Ragl-40-32 | SEQ ID NO 16<br>SEQ ID NO 13 | fCfCAACUCAUUCUCCAAG*U*C<br>fUactfUGGAGAAfUGAGfUfUGG*fC*A | 19 nt<br>21 nt |
| Ragl-40-33 | SEQ ID NO 17<br>SEQ ID NO 18 | CCAACUCAUUCUCCAAGUC<br>UactUGGAGAAUGAGUUGGCA | 19 nt<br>21 nt |
| Ragl-40-34 | SEQ ID NO 17<br>SEQ ID NO 19 | CCAACUCAUUCUCCAAGUC<br>UacttggAGAAUGAGUUGGCA | 19 nt<br>21 nt |
| Ragl-40-53 | SEQ ID NO 24<br>SEQ ID NO 25 | mCmCAACfUCAfUfUCfUCCAAG*mU*mC<br>fUAfCfUfUGGAGAAfUGAGfUfUGG*fC*fA | 19 nt<br>21 nt |

[a]Nucleotides in lower-case letters = DNA; [b]m = 2'OMe; [c]f = 2'F; [d]* = phosphorothioate (PS) The sense oligonucleotide strands are:

1) RAG1-SS-S6A comprising two 2'OMe modifications at its 5' and 3' ends;

2) RAG1-SS-S6B comprising two 2'F modifications at its 5' and 3' ends;

3) RAG1-SS-S6C, derived from RAG1-SS-S6A, having the last two 3'-end phosphodiester bonds being replaced by phosphorothioate bonds;

4) RAG1-SS-S6D, derived from RAG1-SS-S6B, having the last two 3'-end phosphodiester bonds being replaced by phosphorothioate bonds, respectively.

The antisense oligonucleotide strands are:

1) RAG1-SS-AS2A comprising eight 2'F modifications in total, at positions 1, 3, 4, 5, 12, 16, 17, and 20, respectively, counting from the 5' end;

2) RAG1-SS-AS2B comprising five 2'F modifications in total, at positions 1, 5, 12, 16, and 20, respectively, counting from the 5' end;

3) RAG1-SS-AS2C, derived from RAG1-SS-AS2 and fully modified, comprising 2'OMe modifications at positions 2, 6, 7, 8, 9, 10, 11, 13, 14, 15, 18, 19, and 21, respectively, counting from the 5' end;

4) RAG1-SS-AS2D, derived from RAG1-SS-AS2B, having the last two 3'-end phosphodiester bonds being replaced with PS;

5) RAG1-SS-AS2E, derived from RAG1-SS-AS2D, with the first nucleotide from its 5' end of which lacking 2'F modification;

6) RAG1-SS-AS2F, derived from RAG1-SS-AS2A, having the first phosphodiester bond at its 5' end and the last two phosphodiester bonds at its 3' end being replaced with PS;

7) RAG1-SS-AS2G, derived from RAG1-SS-AS2F, with the first nucleotide from its 5' end of which lacking 2'F modification;

8) RAG1-SS-AS2H, similar to RAG1-SS-AS2D, but having the three ribonucleotides at positions 2, 3 and 4 counting from the 5' end being replaced by deoxynucleotides, and 2'F modification at position 17 counting from the 5' end;

9) RAG1-SS-AS2I, comprising the three deoxynucleotides at positions 2, 3, and 4, respectively, counting from the 5' end;

10) RAG1-SS-AS2J, comprising six deoxynucleotides at positions 2, 3, 4, 5, 6, and 7, respectively, counting from the 5' end, respectively.

Each sense oligonucleotide strand-derived sequence was annealed to the first eight antisense oligonucleotide strand-derived sequences (RAG1-SS-AS2A-RAG1-SS-AS2H), resulting in a total of 32 duplexes, each duplex comprising a unique modification pattern (Figure 2). In addition, the unmodified sense oligonucleotide strand RAG1-SS-S6 was annealed to RAG1-SS-AS2I and RAG1-SS-AS2J to generate two additional duplexes (Rag1-40-33 and Rag1-40-34) (Figure 2).

## EXAMPLE 2 CHEMICAL MODIFICATIONS SIGNIFICANTLY INCREASE THE STABILITY OF THE DUPLEX IN THE PRESENCE OF RNASE AND URINE

To assess the stability of the saRNA RAG1-40-38 duplex in the presence of nucleases, the duplex was incubated in 0.02 μg/μL of RNase A solution for various durations of 0-1 hour. As shown in Figure 3, double-stranded saRNA started to degrade within 5 minutes and was completely degraded within 1 hour.

All chemically modified duplexes (Rag1-40-1 to Rag1-40-32) were incubated with 0.02 μg/μL of RNase A for different durations (0 to 96 hours). At the end of the incubation, the size and amount of duplexes were detected by agarose gel electrophoresis. As shown in Figure 4, among the 32 derived sequences, 20 double-stranded saRNAs (62.5%) showed little or no signs of degradation at 96 hours, including Rag1-40-1, Rag1-40-3, Rag1-40-6, Rag1-40-7, Rag1-40-8, Rag1-40-9, Rag1-40-11, Rag1-40-14, Rag1-40-15, Rag1-40-16, Rag1-40-17, Rag1-40-19, Rag1-40-22, Rag1-40-23, Rag1-40-24, Rag1-40-25, Rag1-40-27, Rag1-40-30, Rag1-40-31, and Rag1-40-32, while the remaining 12 (37.5%) double-stranded saRNA-derived sequences (Rag1-40-2, Rag1-40-4, Rag1-40-5, Rag1-40-10, Rag1-40-12, Rag1-40-13, Rag1-40-18, Rag1-40-20, Rag1-40-21, Rag1-40-26, Rag1-40-28, Rag1-40-29) were completely degraded within 24 hours. These data suggest that suitable chemical modifications can significantly improve the stability of saRNA duplexes.

Further analysis of the data in Figure 4 shows that the duplexes comprising antisense oligonucleotide strands RAG1-SS-AS2B, RAG1-SS-AS2D, and RAG1-SS-AS2E (such as Rag1-40-2, Rag1-40-4, Rag1-40-5, Rag1-40-10, Rag1-40-12, Rag1-40-13, Rag1-40-18, Rag1-40-20, Rag1-40-21, Rag1-40-26, Rag1-40-28, Rag1-40-29) were all degraded within 24 hours, while the remaining duplexes were stable within 96 hours. The difference in these three antisense oligonucleotide strands (RAG1-SS-AS2B, RAG1-SS-AS2D and RAG1-SS-AS2E) is that they lack 2'F modification of nucleotide at positions 3, 4 or 17 counting from the 5' end. Therefore, 2'F modifications near the 5' end and near the 3'

end positions on the antisense oligonucleotide strand are critical for stability.

To further test the stability of the chemically modified double-stranded saRNA in human urine, the duplexes were incubated in urine for different durations ranging from 0 to 48 hours. As shown in Figure 5, Rag1-0 and Rag1-40, which were not chemically modified, were mostly degraded within 24 hours and completely degraded within 48 hours. However, in eight selected chemically modified Rag1-40 derived sequences (Rag1-40-23, Rag1-40-25, Rag1-40-31, Rag1-40-17, Rag1-40-22, Rag1-40-7), no sign of degradation was shown within 48 hours; whereas Rag1-40-26 and Rag1-40-29 were mostly degraded after 48 hours. Consistent with the stability analysis for RNase A, these data suggest that suitable chemical modifications can also improve the stability of saRNA duplexes in urine.

## EXAMPLE 3 STRUCTURE-OPTIMIZED RAG1-40 SARNAS ENHANCE THE SUPPRESSION OF CELLULAR PROLIFERATION AND MITIGATE OFF-TARGET EFFECTS

RAG1-0 is the target sequence of CDKN1A (p21), based on which the structure was optimized to obtain 16 saRNAs: RAG1-29, RAG1-30, RAG1-31, RAG1-32, RAG1-33, RAG1-34, RAG1-35, RAG1-36, RAG1-37, RAG1-38, RAG1-39, RAG1-40, RAG1-41, RAG1-42, RAG1-43, RAG1-44.

Figure 6A shows the expression level of p21 mRNA in Ku-7-LG cells transfected with 10 nM of structurally optimized saRNA for 3 days. All saRNAs showed higher activity compared to the control group. Compared with the target sequence RAG1-0, all saRNAs were less active than RAG1-0 but still maintained relatively high activity (more than 2-fold). Figure 6B shows that RAG1-40 obtained lower cellular proliferation proportion (32.4%) than RAG1-0 (48.9%), indicating that RAG1-40 enhances the suppression of cellular proliferation. Figure 6C shows that the luciferase activity (0.84-fold) of RAG1-40 is higher than RAG1-0 (0.77-fold), indicating that RAG1-40 shows lower off-target effect than RAG1-0. The above results indicate that compared with the target sequence RAG1-0, the structurally optimized RAG1-40 can enhance the suppression of cellular proliferation and mitigate off-target effects.

## EXAMPLE 4 CHEMICAL MODIFICATION MAINTAINS OR ENHANCES THE RNA ACTIVATION OF SARNA DUPLEXES

The main purpose of introducing chemical modifications to regulatory oligonucleotides of a gene is to improve their pharmaceutical properties, including enhancing *in vivo* stability and mitigating off-target effects (e.g., sequence-dependent targeting of unrelated genes, sequence-dependent or independent immune stimulation), while maintaining their gene regulatory capacity (gene knockdown or activation). To assess the effect of chemical modification on the RNAa activity of saRNA on the p21 gene, 10 nM unmodified (Rag1-40) and modified (Rag1-40-1 to Rag1-40-34) duplexes were transfected into Ku-7-LG and T24 cells, respectively. After 72 hours, the mRNA expression level of p21 was assessed by RT-qPCR. All duplexes induced p21 mRNA expression in all 3 cell lines at least double that of the control. Figure 7A is in Ku-7-LG cells, compared with Rag1-40, all chemically modified derived sequences except Rag1-40-32, Rag1-40-33 and Rag1-40-34 showed improved or similar RNA activation activity. Figure 7B shows that in T24 cells, similar to Ku-7-LG cells, 29 (85.3%) of the chemically modified derived sequences have comparable or better RNA activation activity than Rag1-40; only 5 (Rag1-40-11, Rag1-40-31, Rag1-40-32, Rag1-40-33 and Rag1-40-34) showed reduced activity compared to Rag1-40.

In chemically modified duplexes Rag1-40-32, Rag1-40-33, and Rag1-40-34, the region of positions 2 to 4 from the 5' end of the antisense oligonucleotide strand was replaced with deoxynucleotide. All cell lines tested showed attenuated activity (Figures 7A-7B), which indicated that DNA replacement of ribonucleotides in the region near the 5' end of the antisense oligonucleotide strand was a degenerate design strategy for RNA activation. Taken together, these data suggest that suitable introduction of 2'F, 2'OMe, and PS modifications into saRNA duplexes does not negatively affect RNAa activity, but instead enhances activity in most combinations, which can be primarily attributable to chemical modifications that increase the resistance of saRNA duplexes to nucleases, and can also be related to chemical modifications that increase the entry of saRNA into the nucleus and increase the chance of antisense oligonucleotide strands being used as guide strands by Argonautes (Agos). The observation is summarized as follows:

1) The combination of 2'F, 2'OMe and PS modifications increases the ability of saRNA duplexes to induce p21 expression;

2) Two PS modifications at the 3' end of the sense oligonucleotide strand of the duplex can improve the gene activation efficacy of the duplex. This can be seen by comparing the gene activation efficacy of saRNA duplexes comprising such modification (Rag1-40-17 to Rag1-40-32) with those without the modification at corresponding positions (Rag1-40-1 to Rag1-40-16).

## EXAMPLE 5 CHEMICAL MODIFICATION ENHANCES THE SUPPRESSION ON CELLULAR PROLIFERATION BY P21 SARNA

It is known that p21 induction by saRNA is that a variety of tumor suppressor factors such as p21 protein suppress the proliferation of cancer cells. To evaluate the effect of chemically modified saRNAs on the function of cancer cells, cells (Ku-7-LG and T24) were transfected with 10 nM unmodified and modified saRNAs for 72 hours, and the cellular proliferation was determined by CCK8. As shown in Figures 8A-8B, Rag1-40 caused 40-60% suppression of proliferation in both cell lines, and most chemically modified Rag1-40-derived sequences showed enhanced suppression for both cell lines. Furthermore, there was a correlation between RNA activating activity (Figures 7A-7B) and suppression of cellular proliferation activity (Figures 8A-8B). Strong p21 activators are usually also strong cytostatics. For example, in Ku-7-LG cells, the weak cellular proliferation inhibitory activity of Rag1-40-32, Rag1-40-33, and Rag1-40-34 (Figure 8A) was correlated with their weak RNA activating activity (Figure 7A). From the perspective of the stability and gene activation data, the suitable incorporation of the selected chemical modifications in saRNAs can significantly enhance the proliferation suppressing properties of these saRNAs when transfected into cancer cells, and this enhancement is brought about by improved duplex stability.

Based on the proliferation suppressing activity of the saRNA duplex, it is suggested that:

1) The combination of 2'F, 2'OMe and PS modifications in the saRNA duplex increases the suppression of cancer cell proliferation by the saRNA duplex;

2) Two PS-modified phosphodiester bonds at the 3' end of the sense oligonucleotide strand enhance the activity of the saRNA duplex to suppress proliferation (by comparing the PS-modified sense oligonucleotide strand (Rag1-40-17~Rag1-40-32) and the sense oligonucleotide strand without the modification (Rag1-40-1~Rag1-40-16));

3) The replacement of ribonucleotides in the antisense oligonucleotide strand with DNA weakens the activity of the saNRA duplex to suppress cellular proliferation (for example, Rag1-40-8, Rag1-40-16, Rag1-40-24, and Rag1-40-32);

4) Except for Rag1-40-3 and Rag1-40-19, modification at all positions of the antisense oligonucleotide strand with 2'F + 2'OMe combination negatively affects proliferation suppression activity (e.g., Rag1-40-11, and Rag1-40-27). These four duplexes all comprise the same antisense oligonucleotide strand (RAG1-SS-AS2C), but Rag1-40-11 and Rag1-40-27 comprise 2'F modified sense oligonucleotide strands, while Rag1-40-3 and Rag1-40-19 are sense oligonucleotide strands comprising 2'OMe modification. Such difference suggests that the negative effect on proliferation suppressing activity produced by complete modification of the antisense oligonucleotide strand can be counteracted by 2'OMe modification of the sense oligonucleotide strand.

## EXAMPLE 6 CHEMICAL MODIFICATION OF SARNA INHIBITS IMMUNE STIMULATION

Oligonucleotides are used in the treatment of diseases by interfering with gene expression, and the immunostimulatory effect of oligonucleotides is an undesirable side effect in some cases. To assess the immunostimulatory effect of chemically modified saRNAs, human peripheral blood mononuclear cells (PBMC) from healthy donors were incubated with the double-stranded saRNAs, and INF-$\alpha$ and TNF-$\alpha$ levels in the supernatant of treated cells after 24 hours were measured.

As shown in Figure 9A, compared with the blank control (~60pg/ml), the cells treated with the positive control, double-stranded oligonucleotide RAG1-IS-1 (known as a strong immunostimulant, the sequence of which is listed in Table 3) showed significantly increased protein level of INF-$\alpha$ (708 pg/ml). Rag1-40 without chemical modification also has certain immunostimulatory activity, and the protein level of INF-$\alpha$ in the treated cells reached 155pg/ml. However, 15 of the chemically modified Rag1-40-derived sequences (46.9% of the total) showed reduced immunostimulatory activity, including Rag1-40-4, Rag1-40-5, Rag1-40-6, Rag1-40-7, Rag1-40-8, Rag1-40-9, Rag1-40-10, Rag1-40-11, Rag1-40-12, Rag1-40-13, Rag1-40-15, Rag1-40-16, Rag1-40-22, Rag1-40-27, Rag1-40-29 and Rag1-40-32. Unexpectedly, compared with Rag1-40, Rag1-40-23, Rag1-40-28, Rag1-40-30 and Rag1-40-31 showed stronger stimulation activity for INF-$\alpha$, and Rag1-40-31 even more, the activity of which was higher than that of the positive control sequence (Rag1-IS-1) known to be active. As shown in Figure 9B, all chemically modified Rag1-40-derived sequences exhibited inhibited immunostimulatory effects for TNF-$\alpha$ induction, with the only exception being Rag1-40-3, which showed unchanged immunostimulatory activity when compared with Rag 1-40.

These data suggest that most of the various combinations of chemical modifications of saRNA duplexes suppress the immunostimulatory effects of the duplexes, but specific modification permutations promote the immunostimulatory activity of dsRNAs. This immune-enhancing effect is valuable for the treatment of certain diseases, for example,

intravesical infusion administration in the treatment of non-muscle invasive bladder cancer (NMIBC), because such saRNAs not only directly act on the target genes, but also activate the immune system to achieve tumor suppression.

**TABLE 3 POSITIVE CONTROL SEQUENCE**

| NAME | SEQ ID NO | SEQUENCE (5'-3') |
|---|---|---|
| RAG-IS-1 S | SEQ ID NO 20 | GUCAUCACACUGAAUACCAAU |
| RAG-IS-1 AS | SEQ ID NO 21 | AUUGGUAUUCAGUGUGAUGACAC |

## EXAMPLE 7 THE CHEMICAL MODIFICATION OF SARNA MITIGATES THE OFF-TARGET EFFECT MEDIATED BY THE SENSE OLIGONUCLEOTIDE STRAND

In RNA activation, the strand identical to the sequence of the promoter's sense DNA strand is called the sense oligonucleotide strand, and its targeting activity should be inhibited to mitigate potential off-target effects. To assess the off-target effects of unmodified and modified saRNAs, a sequence complementary to the sense oligonucleotide strand of Rag1-40 was cloned into the 3'UTR region of the luciferase gene in a pmirGLO dual luciferase vector. The vector plasmid was co-transfected with the saRNA into COS-1 cells. Cells were lysed 48 hours after transfection and the resulting lysates were assessed for luciferase activity. See Table 4 for primer sequences.

**TABLE 4 PRIMERS FOR LUCIFERASE GENE DETECTION**

| NAME | SEQ ID NO | SEQUENCE (5'-3') |
|---|---|---|
| pOFF-target F | SEQ ID NO 22 | CTAGCGAGTGCCAACTCATTCTCCAAGTAAAT |
| pOFF-target R | SEQ ID NO 23 | CTAGATTTACTTGGAGAATGAGTTGGCACTCG |

As shown in Figure 10, most of the chemically modified sequences derived from Rag1-40 showed completely inhibited, mitigated or similar off-target effects compared to unmodified Rag1-40. Among the 34 chemically modified sequences derived from Rag1-40, the off-target effects of 9 (26.5%) derived sequences were completely inhibited, including Rag1-40-1, Rag1-40-3, Rag1-40-4, Rag1-40-5, Rag1-40-10, Rag1-40-11, Rag1-40-22, Rag1-40-24, Rag1-40-27; 22 (64.7%) derived sequences showed mitigated or similar off-target effects (Rag1-40-2, Rag1-40-6, Rag1-40-7, Rag1-40-8, Rag1-40-9, Rag1-40-12, Rag1-40-13, Ragl-40-14, Rag1-40-15, Rag1-40-17, Rag1-40-18, Rag1-40-19, Rag1-40-20, Rag1-40-21, Ragl-40-23, Rag1-40-25, Rag1-40-28, Rag1-40-29, Rag1-40-30, Rag1-40-31, Rag1-40-33, Ragl-40-34). Only 3 (8.8%) derived sequences had increased off-target effects (Rag1-40-16, Rag1-40-26, and Rag1-40-32).

This result indicates that chemical modifications and their combinations used in the disclosure can mitigate the off-target effect of saRNA oligonucleotides, and one possible mechanism is to prevent Ago from loading the sense strand as the guide strand. For the 3 saRNA duplexes that showed increased off-target effects, two of them (Rag 1-40-16 and Rag1-40-32) had DNA modification in the antisense oligonucleotide strand and 2'F modification in the sense oligonucleotide strand, indicating that DNA modification, especially when combined with 2'F modification in the sense oligonucleotide strand, should be avoided.

## EXAMPLE 8 CHEMICALLY MODIFIED SARNA DUPLEXES MAINTAIN ACTIVATION ACTIVITY IN BLADDER CANCER CELLS AFTER DIFFERENT DURATIONS OF TREATMENT

In order to further evaluate the expression level of p21 mRNA after the different durations of treatment by the chemically modified saRNA duplexes, two saRNAs (RAG1-40-31 and RAG1-40-53) were randomly selected from the above chemically modified saRNA duplexes for detection. RAG1-40-31 and RAG1-40-53 were transfected into Ku-7-LG and T24 cells at a final concentration of 10 nM, for 1, 2, 3, 4, 7 and 9 days, respectively. Each treatment was set up in duplicate. Two-step RT-qPCR was performed as described in the general protocal above.

Figure 11A shows the expression levels of p21 mRNA in Ku-7-LG cells treated with of RAG1-40-31 and RAG1-40-53 for different durations. Compared with the control group, the expression level of p21 mRNA increased by 1.3-fold, 11.4-fold, 8.7-fold, 6.1-fold, 2.6-fold and 3.8-fold after treatment with RAG1-40-31 for 1, 2, 3, 4, 7 and 9 days, respectively, and 1.4-fold, 13.0-fold, 9.1-fold, 4.5-fold, 3.2-fold and 4.1-fold after treatment with RAG1-40-53 for 1, 2, 3, 4, 7 and 9 days, respectively. Figure 11B shows the expression levels of p21 mRNA in T24 cells treated with RAG1-40-5 and RAG1-40-53 for different durations. Compared with the control group, the expression level of p21 mRNA increased by 2.1-fold, 3.7-fold, 4.7-fold, 3.3-fold, 1.2-fold and 1.3-fold after treatment with RAG1-40-31 for 1, 2, 3, 4, 7 and 9 days,

respectively, and the expression level of p21 mRNA increased by 2.2-fold, 3.5-fold, 7.8-fold, 4.5-fold, 1.2-fold and 1.1-fold after treatment with RAG1-40-53 for 1, 2, 3, 4, 7 and 9 days, respectively.

This suggests that the randomly selected saRNAs RAG1-40-31 and RAG1-40-53 gradually increased the expression level of p21 mRNA with increasing treatment duration, reached the peak after 2 and 3 days of treatment, respectively, and then the expression began to decline but the activation activity was remained.

## EXAMPLE 9 CHEMICALLY MODIFIED SARNA DUPLEX SUPPRESSES CELLULAR PROLIFERATION BY BLOCKING CELL CYCLE PROGRESSION IN KU-7-LG CELLS

Example 5 above shows that the chemically modified saRNA duplexes can suppress cellular proliferation. In order to study the mechanism of cellular proliferation suppression, change in cell cycle was analyzed using flow cytometry in this example. Randomly selected saRNAs RAG1-40-31 and RAG1-40-53 were transfected into Ku-7-LG cells at final concentrations of 0.1, 1, 10 and 50 nM, respectively, for 48 hours. Each treatment was set in duplicate. Cell cycle was detected and analyzed as described in the general protocol above.

Figure 12 shows the percentage of Ku-7-LG cells after treated with different concentrations of RAG1-40-31 and RAG1-40-53 for different durations. For RAG1-40-31 treatments at 0.1, 1, 10 and 50 nM, the percentages of cells in prophase of DNA synthesis (G0/G1) were 62%, 69%, 81% and 81%, respectively; for RAG1-40-31 treatments at 0.1, 1, 10 and 50 nM, the percentages of cells in DNA replication phase (S phase) were 32%, 22%, 11% and 6%, respectively; for RAG1-40-31 treatments at 0.1, 1, 10 and 50 nM, the percentages of cells in DNA anaphase/mitosis (G2/M) were 6%, 9%, 8% and 13%, respectively. For RAG1-40-53 treatments at 0.1, 1, 10 and 50 nM, the percentages of cells in prophase of DNA synthesis (G0/G1) were 57%, 66%, 79% and 76% respectively; for RAG1-40-53 treatments at 0.1, 1, 10 and 50 nM, the percentages of cells in DNA replication phase (S phase) were 36%, 24%, 12% and 16% respectively; for RAG1-40-53 treatments at 0.1, 1, 10 and 50 nM, the percentages of cells in G2/M were 7%, 10%, 9% and 8%, respectively.

This suggests that the randomly selected chemically modified saRNAs RAG1-40-31 and RAG1-40-53 suppress cellular proliferation by blocking replication in the S phase of the cell cycle.

## EXAMPLE 10 CHEMICALLY MODIFIED SARNA DUPLEX PROMOTES APOPTOSIS IN THE CO-CULTURE OF J82 CELLS AND PBMC CELLS

Peripheral blood mononuclear cells (PBMC) are cells with a single nucleus in peripheral blood, including lymphocytes, monocytes and dendritic cells. PBMC cells can activate immune stimulation by secreting inflammatory factors, thereby inhibiting the growth and reproduction of tumor cells.

Given that chemically modified RAG1-40-31 exhibited stronger immunostimulatory activity (Example 6), we speculated that this property could be exploited to promote tumor cell suppression. In order to verify this hypothesis, in this example, RAG1-40-31 duplex was selected to transfect J82 cells, and after 24 hours of transfection, the cells were refreshed with fresh medium and PBMC cells at a certain ratio (J82 cells:PBMC cells = 1:4) were added, or cells free of PBMC were replaced as control. After 48 hours of co-culture, FITC Annexin V kit was used to detect the apoptosis of tumor cells on a flow cytometer. As shown in Figure 13, flow cytometric analysis was performed after co-culture of J82 cells and PBMC cells with different concentrations of RAG1-40-31. In PBMC-free cell culture, for RAG1-40-31 treatments at 0.1, 1, 10 and 25 nM, the percentages of cells in early apoptosis were 2.3%, 2.6%, 9.4% and 15.5%, respectively, and the percentages of cells in late apoptosis were 5.3%, 5.2%, 6.7% and 9.1%, respectively. In co-culture with PBMC cells, for RAG1-40-31 treatments at 0.1, 1, 10 and 25 nM, the percentages of cells in early apoptosis were 2.0%, 4.5%, 31.5% and 49.3%, respectively, and the percentages of cells in late apoptosis were 5.6%, 7.3%, 6.3% and 5.8%, respectively. The results showed that in the presence of PBMC cells, RAG1-40-31 had a stronger activity of promoting early apoptosis of tumor cells, especially at high transfection concentrations, which fully suggests that the combination of specific chemical modifications and their arrangement in the sequence can improve the immunostimulatory activity of the double-stranded RNA, resulting better killing effects on tumor cells.

## EXAMPLE 11 SYNERGISTIC SUPPRESSING EFFECT OF CHEMICALLY MODIFIED SARNA DUPLEX COMBINED WITH CHEMICAL AGENTS ON J82 CELL GROWTH AND PROLIFERATION

In this example, chemically modified saRNA RAG1-40-31 was used in combination with clinical chemical agents to evaluate the suppressing effect of the combined agents on cancer cells.

Mitomycin C (MMC) is a chemical agent clinically used to treat tumors. It mainly inhibits DNA synthesis by generating DNA cross-links (adherence to cancer cell DNA), making cells unable to divide and eventually causing cell death.

Valrubicin is a chemotherapy drug for the clinical treatment of bladder cancer. It mainly interacts with DNA

topoisomerase II to affect the normal break between DNA and enzyme compounds, thereby inhibiting the incorporation of nucleosides into nucleic acids, causing chromosomal damage and arresting cell cycle entry into G2 phase.

Figures 14A-14B show the relative proliferation proportions of J82 cells after the combined treatment with chemically modified saRNA RAG1-40-31 and Mitomycin C. For saRNA RAG1-40-31 treatments at 0.1-50 nM, the relative proliferation proportion of cells after treatment with Mitomycin C at concentrations of 100, 1000 and 10000 nM were all lower than 35%. For saRNA RAG1-40-31 treatments at 5-50 nM, the relative proliferation proportion of cells after treatment with Mitomycin C at concentrations of 1, 10, 100, 1000 and 10000 nM were all lower than 43%. For saRNA RAG1-40-31 treatments at 25 nM and 50 nM, the relative proliferation proportion of cells after treatment with Mitomycin C at concentrations of 1, 10, 100, 1000 and 10000 nM were all lower than 26%. The relative proliferation proportion of J82 cells after treatment with specific combinations is shown in Table 6.

**TABLE 6 RELATIVE PROLIFERATION PROPORTION OF J82 CELLS AFTER COMBINED TREATMENT WITH RAG1-40-31 AND MITOMYCIN C**

| Group | Control | MMC (1 nM) | MMC (10 nM) | MMC (100 nM) | MMC (1000 nM) | MMC (10000 nM) |
|---|---|---|---|---|---|---|
| RAG1-40-31 (0 nM) | 100.0 | 99.9 | 99.9 | 99.9 | 100.0 | 100.0 |
| RAG1-40-31 (0.1 nM) | 97.9 | 74.3 | 71.5 | 32.9 | 10.8 | 1.5 |
| RAG1-40-31 (0.5 nM) | 89.9 | 70.1 | 69.3 | 34.9 | 10.8 | 0.9 |
| RAG1-40-31 (1 nM) | 79.8 | 55.8 | 57.2 | 30.1 | 9.3 | 1.4 |
| RAG1-40-31 (5 nM) | 59.3 | 42.8 | 40.8 | 26.2 | 7.2 | 1.2 |
| RAG1-40-31 (10 nM) | 48.9 | 37.0 | 37.7 | 22.2 | 6.9 | 1.0 |
| RAG1-40-31 (25 nM) | 38.4 | 20.8 | 25.7 | 17.2 | 5.0 | 0.4 |
| RAG1-40-31 (50 nM) | 34.8 | 25.9 | 25.8 | 13.9 | 3.4 | 0.6 |

Figure 14C shows the combination index (CI) of chemically modified RAG1-40-31 and Mitomycin C administrated in combination. A CI value between 0 and 0.3 indicates strong synergy, and a CI value between 0.3 and 0.7 indicates synergy. The specific combination index of RAG1-40-31 and Mitomycin C is shown in Table 7.

**TABLE 7 THE COMBINATION INDEX OF RAG1-40-31 AND MITOMYCIN C COMBINATION IN J82 CELLS**

| | Combination Index (CI) | | | | |
|---|---|---|---|---|---|
| Group | MMC (1 nM) | MMC (10 nM) | MMC (100 nM) | MMC (1000 nM) | MMC (10000 nM) |
| RAG1-40-31 (0.1 nM) | 0.08 | 0.42 | 0.47 | 0.73 | 0.50 |
| RAG1-40-31 (0.5 nM) | 0.17 | 0.47 | 0.54 | 0.75 | 0.25 |
| RAG1-40-31 (1 nM) | 0.13 | 0.58 | 0.52 | 0.76 | 0.46 |
| RAG1-40-31 (5 nM) | 0.29 | 0.32 | 0.40 | 0.43 | 0.41 |
| RAG1-40-31 (10 nM) | 0.40 | 0.47 | 0.37 | 0.41 | 0.29 |
| RAG1-40-31 (25 nM) | 0.32 | 0.50 | 0.37 | 0.28 | 0.09 |
| RAG1-40-31 (50 nM) | 0.96 | 0.98 | 0.44 | 0.19 | 0.15 |

Figures 15A-15B show the relative proliferation proportions of J82 cells after combined treatment with chemically modified saRNA RAG1-40-31 and Valrubicin. For saRNA RAG1-40-31 treatments at 0.1-50 nM, the relative proliferation

proportion of cells after treatment with Valrubicin at concentrations of 1000 nM and 10000 nM was lower than 30%. For saRNA RAG1-40-31 treatments at 25 nM and 50 nM, the relative proliferation proportion of cells after treatment with Valrubicin at concentrations of 1, 10, 100, 1000 and 10000 nM were all lower than 30%. See Table 8 for the relative proliferation proportions of J82 cells after treatment with specific combinations.

**TABLE 8 RELATIVE PROLIFERATION PROPORTIONS OF J82 CELLS AFTER COMBINED TREATMENT WITH RAG1-40-31 AND VALRUBICIN**

| | Relative proliferation proportion of J82 cells (%) | | | | | |
|---|---|---|---|---|---|---|
| Group | Control | valrubicin (1 nM) | valrubicin (10 nM) | valrubicin (100 nM) | valrubicin (1000 nM) | valrubicin (10000 nM) |
| RAG1-40-31 (0 nM) | 100.7 | 100.0 | 100.0 | 100.0 | 100.0 | 100.0 |
| RAG1-40-31 (0.1 nM) | 85.1 | 71.0 | 58.2 | 73.2 | 26.9 | 9.9 |
| RAG1-40-31 (0.5 nM) | 77.8 | 62.4 | 54.1 | 72.8 | 24.4 | 7.7 |
| RAG1-40-31 (1 nM) | 73.9 | 59.8 | 51.6 | 54.4 | 22.7 | 7.5 |
| RAG1-40-31 (5 nM) | 58.4 | 37.9 | 46.6 | 50.9 | 22.6 | 5.5 |
| RAG1-40-31 (10 nM) | 50.6 | 33.1 | 37.1 | 40.4 | 20.3 | 4.3 |
| RAG1-40-31 (25 nM) | 45.6 | 26.1 | 23.9 | 26.3 | 13.3 | 2.7 |
| RAG1-40-31 (50 nM) | 33.5 | 19.7 | 21.4 | 23.9 | 14.8 | 3.2 |

Figure 15C shows the combination index (CI) of chemically modified RAG1-40-31 combined with valrubicin. A CI value between 0 and 0.3 indicates strong synergy, and a CI value between 0.3 and 0.7 indicates synergy. The specific combination index of RAG1-40-31 and Mitomycin C is shown in Table 9.

**TABLE 9 THE COMBINATION INDEX OF RAG1-40-31 AND VALRUBICIN IN J82 CELLS**

| | Combination Index (CI) | | | | |
|---|---|---|---|---|---|
| Group | valrubicin (1 nM) | valrubicin (10 nM) | valrubicin (100 nM) | valrubicin (1000 nM) | valrubicin (10000 nM) |
| RAG1-40-31 (0.1 nM) | 0.10 | 0.42 | 0.85 | 0.18 | 0.19 |
| RAG1-40-31 (0.5 nM) | 0.18 | 0.08 | 1.31 | 0.14 | 0.11 |
| RAG1-40-31 (1 nM) | 0.26 | 0.12 | 0.30 | 0.12 | 0.11 |
| RAG1-40-31 (5 nM) | 0.12 | 0.32 | 0.61 | 0.13 | 0.06 |
| RAG1-40-31 (10 nM) | 0.14 | 0.22 | 0.37 | 0.11 | 0.04 |
| RAG1-40-31 (25 nM) | 0.14 | 0.10 | 0.16 | 0.05 | 0.02 |
| RAG1-40-31 (50 nM) | 0.11 | 0.14 | 0.22 | 0.09 | 0.02 |

The above results indicate that the combined use of chemically modified RAG1-40-31, Mitomycin C or valrubicin has a synergistic effect, and the combined use can provide a better inhibitory effect on J82 tumor cells.

## EXAMPLE 12 SYNERGISTIC SUPPRESSING EFFECT OF CHEMICALLY MODIFIED SARNA DUPLEXES COMBINED WITH CHEMICAL AGENTS ON THE GROWTH AND PROLIFERATION OF T24 CELLS

The information of compound drugs is as described in Example 10.

Figures 16A-16B show the relative proliferation proportions of T24 cells after combined treatment with chemically modified saRNA RAG1-40-31 and Mitomycin C. For saRNA RAG1-40-31 treatments at 0.1-50 nM, the relative proliferation proportions of cells after treatment with Mitomycin C at concentrations of 1000 and 10000 nM were all lower than 18%. For saRNA RAG1-40-31 treatments at 5-50 nM, the relative proliferation proportions of cells after treatment with Mitomycin C at concentrations of 1, 10, 100, 1000 and 10000 nM were all lower than 40%. For saRNA RAG1-40-31 treatments at 25 nM and 50 nM, the relative proliferation proportions of cells after treatment with Mitomycin C at concentrations of 1, 10, 100, 1000 and 10000 nM were all lower than 21%. The relative proliferation proportion of T24 cells after treatment with specific combinations is shown in Table 10.

**TABLE 10 THE RELATIVE PROLIFERATION PROPORTION OF T24 CELLS AFTER COMBINED TREATMENT WITH RAG1-40-31 AND MITOMYCIN C**

| Group | Relative proliferation proportion of T24 cells (%) | | | | | |
|---|---|---|---|---|---|---|
| | Control | MMC (1 nM) | MMC (10 nM) | MMC (100 nM) | MMC (1000 nM) | MMC (10000 nM) |
| RAG1-40-31 (0 nM) | 100.0 | 100.0 | 100.0 | 100.0 | 100.0 | 100.0 |
| RAG1-40-31 (0.1 nM) | 97.9 | 93.6 | 94.5 | 63.3 | 17.6 | 5.6 |
| RAG1-40-31 (0.5 nM) | 91.7 | 90.1 | 90.0 | 59.8 | 16.8 | 5.5 |
| RAG1-40-31 (1 nM) | 85.9 | 81.9 | 78.2 | 55.6 | 14.7 | 5.4 |
| RAG1-40-31 (5 nM) | 42.1 | 37.4 | 36.3 | 26.0 | 7.7 | 3.1 |
| RAG1-40-31 (10 nM) | 22.0 | 19.8 | 27.0 | 16.7 | 5.3 | 2.5 |
| RAG1-40-31 (25 nM) | 13.6 | 15.4 | 20.9 | 12.4 | 4.1 | 1.9 |
| RAG1-40-31 (50 nM) | 14.7 | 20.1 | 19.8 | 10.8 | 4.2 | 2.1 |

Figure 16C shows the combination index (CI) of chemically modified RAG1-40-31 and Mitomycin C used in combination. A CI value between 0 and 0.3 indicates strong synergy, and a CI value between 0.3 and 0.7 indicates synergy. The specific combination index of RAG1-40-31 and Mitomycin C is shown in Table 11.

**TABLE 11 COMBINATION INDEX OF RAG1-40-31 COMBINED WITH MITOMYCIN C IN T24 CELLS**

| | Combination Index (CI) | | | | |
|---|---|---|---|---|---|
| Group | MMC (1 nM) | MMC (10 nM) | MMC (100 nM) | MMC (1000 nM) | MMC (10000 nM) |
| RAG1-40-31 (0.1 nM) | 0.42 | 1.59 | 0.64 | 0.49 | 1.05 |
| RAG1-40-31 (0.5 nM) | 0.97 | 1.40 | 0.67 | 0.46 | 1.06 |
| RAG1-40-31 (1 nM) | 0.96 | 0.88 | 0.69 | 0.41 | 0.84 |
| RAG1-40-31 (5 nM) | 0.61 | 0.60 | 0.45 | 0.24 | 0.46 |

(continued)

| | Combination Index (CI) | | | | |
|---|---|---|---|---|---|
| Group | MMC (1 nM) | MMC (10 nM) | MMC (100 nM) | MMC (1000 nM) | MMC (10000 nM) |
| RAG1-40-31 (10 nM) | 0.52 | 0.77 | 0.47 | 0.19 | 0.30 |
| RAG1-40-31 (25 nM) | 0.92 | 1.38 | 0.73 | 0.28 | 0.36 |
| RAG1-40-31 (50 nM) | 2.59 | 2.60 | 1.31 | 0.50 | 0.47 |

Figures 17A-17B show the relative proliferation proportions of T24 cells after combined treatment with chemically modified saRNA RAG1-40-31 and valrubicin. For saRNA RAG1-40-31 treatments at 0.1-50 nM, the relative proliferation proportions of cells after treatment with valrubicin at concentrations of 1000 nM and 10000 nM were lower than 60%. For saRNA RAG1-40-31 treatment at 5-50 nM, the relative proliferation proportions of cells after treatment with valrubicin at concentrations of 1, 10, 100, 1000 and 10000 nM were all lower than 40%. For saRNA RAG1-40-31 treatment at 25 nM and 50 nM, the relative proliferation proportion of cells after treatment with valrubicin at concentrations of 1, 10, 100, 1000 and 10000 nM were all lower than 21%. See Table 12 for the relative proliferation proportion of T24 cells after treatment with specific combination.

**TABLE 12 RELATIVE PROLIFERATION PROPORTION OF T24 CELLS AFTER COMBINED TREATMENT WITH RAG1-40-31 AND VALRUBICIN**

| | Relative proliferation proportion of T24 cells (%) | | | | | |
|---|---|---|---|---|---|---|
| Group | Control | valrubicin(1 nm) | valrubicin (10 nm) | valrubicin ( 100 nm) | valrubicin(1 000 nm) | valrubicin(10000 nm) |
| RAG1-40-31 (0 nM) | 100.0 | 100.0 | 100.0 | 100.0 | 100.0 | 100.0 |
| RAG1-40-31 (0.1 nM) | 93.5 | 96.3 | 97.8 | 93.4 | 59.6 | 18.9 |
| RAG1-40-31 (0.5 nM) | 84.8 | 91.3 | 94.3 | 91.4 | 51.8 | 19.5 |
| RAG1-40-31 (1 nM) | 79.7 | 76.0 | 71.3 | 67.6 | 49.6 | 16.6 |
| RAG1-40-31 (5 nM) | 38.5 | 37.2 | 33.3 | 42.1 | 25.7 | 8.0 |
| RAG1-40-31 (10 nM) | 23.9 | 27.2 | 23.3 | 26.9 | 21.7 | 5.1 |
| RAG1-40-31 (25 nM) | 15.1 | 16.5 | 20.4 | 19.5 | 13.2 | 3.4 |
| RAG1-40-31 (50 nM) | 13.8 | 18.4 | 18.8 | 18.7 | 10.9 | 3.1 |

Figure 17C shows the combination index (CI) of chemically modified RAG1-40-31 combined with valrubicin. A CI value between 0 and 0.3 indicates strong synergy, and a CI value between 0.3 and 0.7 indicates synergy. The specific combination index of RAG1-40-31 and Mitomycin C is shown in Table 13.

**TABLE 13 COMBINATION INDEX OF RAG1-40-31 AND VALRUBICIN IN T24 CELLS**

| | Combination Index (CI) | | | | |
|---|---|---|---|---|---|
| Group | valrubicin (1 nM) | valrubicin (10 nM) | valrubicin (100 nM) | valrubicin (1000 nM) | Valrubicin (10000 nM) |
| RAG1-40-31 (0.1 nM) | 1.9 | 17.16 | 11.04 | 1.48 | 0.38 |

(continued)

| | Combination Index (CI) | | | | |
|---|---|---|---|---|---|
| Group | valrubicin (1 nM) | valrubicin (10 nM) | valrubicin (100 nM) | valrubicin (1000 nM) | Valrubicin (10000 nM) |
| RAG1-40-31 (0.5 nM) | 2.71 | 6.02 | 8.68 | 0.92 | 0.4 |
| RAG1-40-31 (1 nM) | 1.23 | 0.92 | 1.02 | 0.93 | 0.33 |
| RAG1-40-31 (5 nM) | 0.73 | 0.59 | 0.99 | 0.47 | 0.12 |
| RAG1-40-31 (10 nM) | 0.82 | 0.62 | 0.83 | 0.63 | 0.09 |
| RAG1-40-31 (25 nM) | 0.97 | 1.25 | 1.25 | 0.67 | 0.1 |
| RAG1-40-31 (50 nM) | 2.12 | 2.3 | 2.3 | 1.04 | 0.19 |

The above results indicated that the combined use of chemically modified RAG1-40-31, Mitomycin C or valrubicin had a synergistic effect, and the combined use can achieve a better suppressing effect on T24 tumor cells.

Incorporation by reference

The entire disclosure of each patent document and scientific document cited in the present disclosure is hereby incorporated by reference for all purposes.

Equivalence

The present disclosure can be implemented in other specific forms without departing from its essential characteristics. Accordingly, the foregoing embodiments are considered to be illustrative and not limiting of the invention described herein. The scope of the invention is indicated by the appended claims rather than the foregoing description, and all changes that come within the meaning and range of equivalency of the claims are intended to be embraced therein.

References

[1] The role of helper lipids in lipid nanoparticles (LNPs) designed for oligonucleotide delivery. Cheng et.al., Volume 99, Part A, 1 April 2016, p129-137;

[2] Biodistribution of Small Interfering RNA at the Organ and Cellular Levels after Lipid Nanoparticle-mediated Delivery, Shi et.al., J Histochem Cytochem. 2011 Aug; 59(8): 727 - 740.

[3] WO2017192679A1

[4] US20190240354A1

[5] US20190336452A1

[6] US20160038612A1

## Claims

1. A chemically modified small activating RNA, wherein the small activating RNA consists of a sense oligonucleotide strand and an antisense oligonucleotide strand, wherein the sense oligonucleotide strand or the antisense oligonucleotide strand is at least 75%, at least 80%, at least 85%, at least 90%, at least 95% or 100% homologous or complementary to the promoter sequence of a target gene; wherein the two nucleotides at the 5' end and at the 3' end of the sense oligonucleotide strand are all chemically modified nucleotides, and the antisense oligonucleotide strand comprises at least one chemically modified nucleotide.

2. The small activating RNA according to claim 1, wherein the chemical modification is one or more selected from the following modifications: a) chemical modification of nucleotide ribose(s); b) chemical modification of inter-nucleotide phosphodiester bond(s); (3) chemical modification of nucleotide base(s); (4) inclusion of locked nucleic acid(s); and (5) modification with vinylphosphonate at 5'-end nucleotide(s).

3. The small activating RNA according to any of claims 1-2, wherein the chemical modification of nucleotide ribose(s) comprises modification of the hydroxyl group (2'-OH) in the pentose of the nucleotide.

4. The small activating RNA according to any of claims 1-3, wherein the chemical modification of nucleotide ribose(s) comprises the substitution of the ribose 2'-OH of a nucleotide with one or more of 2'-fluoro, 2'-O-methyl, 2'-methoxyethyl, 2,4'-dinitrophenol, a locked nucleic acid, 2'-amino, and 2'-H.

5. The small activating RNA according to any of claims 1-4, wherein the chemical modification of nucleotide base(s) includes 5'-bromouracil modification, 5'-iodouracil modification, N-methyl uracil modification and/or 2,6-diaminopurine modification.

6. The small activating RNA according to any of claims 1-5, wherein at least one of the last two phosphodiester bonds at the 3' end of the sense oligonucleotide strand has a chemical modification defined in any of claims 1-5.

7. The small activating RNA according to any of claims 1-6, wherein the last two phosphodiester bonds at the 3' end of the sense oligonucleotide strand are modified by sulfur and borane respectively in place of the non-bridging oxygen atoms in the phosphodiester bonds; and/or, the last two phosphodiester bonds at the 3' end of the sense oligonucleotide strand are both modified with phosphorothioate (PS).

8. The small activating RNA according to any of claims 1-7, wherein the sense oligonucleotide strand comprises at least one chemically modified nucleotide, and the chemical modification includes a chemical modification of the ribose 2'-OH in nucleotide(s), a chemical modification of inter-nucleotide phosphodiester bond(s), a chemical modification of nucleotide base (s), replacement of any nucleotide with a locked nucleic acid (LNA), and/or modification with vinylphosphonate at 5'-end nucleotide(s) of the sense oligonucleotide strand.

9. The small activating RNA according to any of claims 1-8, wherein at least one of the last two phosphodiester bonds at the 3' end or at the 5' end of the antisense oligonucleotide strand has a chemical modification defined in any of claims 1-5.

10. The small activating RNA according to any of claims 1-9, wherein the last two phosphodiester bonds at the 3' end and/or at 5' end of the antisense oligonucleotide strand comprise phosphorothioate modification and/or boranophosphate modification.

11. The small activating RNA according to any of claims 1-10, wherein the antisense oligonucleotide strand comprises at least one chemically modified nucleotide, and the chemical modification includes a chemical modification of the ribose 2'-OH in nucleotide(s), a chemical modification of inter-nucleotide phosphodiester bond(s), a chemical modification of nucleotide base (s), replacement of any nucleotide with a locked nucleic acid (LNA), and/or modification with vinylphosphonate at 5'-end nucleotide(s) of the sense oligonucleotide strand.

12. The small activating RNA according to any of claims 1-11, wherein the nucleotides at the 3' end and/or 5' end of the antisense oligonucleotide strand have a chemical modification of ribose 2'-OH.

13. The small activating RNA according to any of claims 1-12, wherein the antisense oligonucleotide strand has a chemical modification of the ribose 2'-OH in the most central 1-5 nucleotides.

**14.** The small activating RNA according to any of claims 1-13, wherein the chemical modification of the ribose 2'-OH comprises 2'-fluoro modification.

**15.** The small activating RNA according to any of claims 1-14, wherein the antisense oligonucleotide strand and/or the sense oligonucleotide strand comprise a combination of three modifications: 2'-fluoro, 2'-O-methyl and phosphorothioate (PS) modifications.

**16.** The small activating RNA according to any of claims 1-15, wherein the antisense oligonucleotide strand does not comprise a modification in which a ribonucleotide can be replaced by a deoxyribonucleotide (DNA).

**17.** The small activating RNA according to any of claims 1-16, wherein the segment between the two nucleotides at the 5' end and the two nucleotides at the 3' end of the sense oligonucleotide strand comprises no more than 40%, no more than 30%, no more than 20%, no more than 10%, no more than 5% of chemically modified nucleotides, or is completely free of chemically modified nucleotides.

**18.** The small activating RNA according to any of claims 1-17, wherein the last two phosphodiester bonds at the 3' end of the sense oligonucleotide strand both comprise phosphorothioate modification and/or boranophosphate modification.

**19.** The small activating RNA according to any of claims 1-18, wherein at least 50%, at least 60%, at least 70%, at least 80%, or at least 90% or 100% of the nucleotides of the antisense oligonucleotide strand are chemically modified with 2'-fluoro and/or 2'-O-methyl, and the sense oligonucleotide strand comprises at least 1, at least 2, at least 3, or at least 5 2'-O-methyl modifications.

**20.** The small activating RNA according to any of claims 1-19, wherein the sense oligonucleotide strand or the antisense oligonucleotide strand is conjugated with one or more groups selected from the group consisting of: lipid, fatty acids, fluorescent groups, ligands, sugars, polymers, polypeptides, antibodies, and cholesterol

**21.** The small activating RNA according to any of claims 1-20, wherein either or both of the sense oligonucleotide strand and the antisense oligonucleotide strand have an overhang of 6, 5, 4, 3, 2, 1 or 0 nucleotides, located at the 5' end or 3' end or 5' and 3' ends of the sense oligonucleotide strand or the antisense oligonucleotide strand.

**22.** The small activating RNA according to claim 21, wherein the overhang is dTdT or dTdTdT, or nucleotide(s) identical or complementary to the nucleotide(s) at the corresponding position(s) of the target gene, or neither identical nor complementary to the nucleotide(s) at the corresponding position(s) of the target gene.

**23.** The small activating RNA according to any of claims 1-22, wherein the sense oligonucleotide strand and the antisense oligonucleotide strand each comprise 17-30 nucleotides in length, and the sense oligonucleotide strand and the antisense oligonucleotide strand form a duplex region of base complementarity comprising at least 10, at least 11, at least 12, at least 13, at least 14, or at least 15 nucleotides in length.

**24.** The small activating RNA according to any of claims 1-23, wherein there is 1-3 nucleotide mismatches between the 3' or 5' region of the sense oligonucleotide strand and the corresponding nucleotides in the 5' or 3' region of the antisense oligonucleotide strand; or, there is 1-3 nucleotide mismatches between the 3' or 5' region of the sense oligonucleotide strand and the corresponding nucleotides on the target gene; or, there is 1-3 nucleotide mismatches between the 3' or 5' region of the antisense oligonucleotide strand and the corresponding nucleotides on the target gene.

**25.** The small activating RNA according to any of claims 1-24, wherein chemical modification of the sense oligonucleotide strand is selected from the group consisting of the following four patterns: RAG1-SS-S6A, RAG1-SS-S6B, RAG1-SS-S6C, and RAG1-SS-S6D.

**26.** The small activating RNA according to any of claims 1-25, wherein the chemical modification of the antisense oligonucleotide strand is selected from the group consisting of the following ten patterns: RAG1-SS-AS2A, RAG1-SS-AS2B, RAG1-SS-AS2C, RAG1-SS-AS2D, RAG1-SS-AS2E, RAG1-SS-AS2F, RAG1-SS-AS2G, RAG1-SS-AS2H, RAG1-SS-AS2I, and RAG1-SS-AS2J.

**27.** The small activating RNA according to any of claims 1-26, wherein the chemical modification of the sense oligonucleotide strand is selected from the group consisting of the following four patterns: RAG1-SS-S6A, RAG1-SS-S6B, RAG1-SS-S6C, and RAG1-SS-S6D, and the chemical modification of the antisense oligonucleotide strand is selected from the group consisting of the following ten patterns: RAG1-SS-AS2A, RAG1-SS-AS2B, RAG1-SS-AS2C, RAG1-SS-AS2D, RAG1-SS-AS2E, RAG1-SS-AS2F, RAG1-SS-AS2G, RAG1-SS-AS2H, RAG1-SS-AS2I, and RAG1-SS-AS2J.

**28.** The small activating RNA according to any of claims 1-27, wherein the chemical modification of the sense oligonucleotide strand is the pattern set forth in RAG1-SS-S6D, and the chemical modification of the antisense oligonucleotide strand is the pattern set forth in RAG1-SS-AS2G.

**29.** The small activating RNA according to any of claims 1-28, wherein the target gene comprises human p21 $^{WAF1/CIP1}$ gene (P21 gene).

**30.** The small activating RNA according to claim 29, wherein the small activating RNA specifically targets the promoter region of the p21 gene.

**31.** The small activating RNA according to any of claims 29-30, wherein the sequence of the sense oligonucleotide strand of the small activating RNA is selected from the group consisting of SEQ ID NOs: 5, 14, 15, and 16.

**32.** The small activating RNA according to any of claims 29-31, wherein the sequence of the antisense oligonucleotide strand of the small activating RNA is selected from the group consisting of SEQ ID NOs: 6, 8, 11, 12, 13, 18, and 19.

**33.** The small activating RNA according to any of claims 29-32, wherein the sequences of the sense oligonucleotide strand and the antisense oligonucleotide strand are selected from:

1) SEQ ID NO: 5 and SEQ ID NO: 6;
2) SEQ ID NO: 5 and SEQ ID NO: 7;
3) SEQ ID NO: 5 and SEQ ID NO: 8;
4) SEQ ID NO: 5 and SEQ ID NO: 9;
5) SEQ ID NO: 5 and SEQ ID NO: 10;
6) SEQ ID NO: 5 and SEQ ID NO: 11;
7) SEQ ID NO: 5 and SEQ ID NO: 12;
8) SEQ ID NO: 5 and SEQ ID NO: 13;
9) SEQ ID NO: 14 and SEQ ID NO: 6;
10) SEQ ID NO: 14 and SEQ ID NO: 7;
11) SEQ ID NO: 14 and SEQ ID NO: 8;
12) SEQ ID NO: 14 and SEQ ID NO: 9;
13) SEQ ID NO: 14 and SEQ ID NO: 10;
14) SEQ ID NO: 14 and SEQ ID NO: 11;
15) SEQ ID NO: 14 and SEQ ID NO: 12;
16) SEQ ID NO: 14 and SEQ ID NO: 13;
17) SEQ ID NO: 15 and SEQ ID NO: 6;
18) SEQ ID NO: 15 and SEQ ID NO: 7;
19) SEQ ID NO: 15 and SEQ ID NO: 8;
20) SEQ ID NO: 15 and SEQ ID NO: 9;
21) SEQ ID NO: 15 and SEQ ID NO: 10;
22) SEQ ID NO: 15 and SEQ ID NO: 11;
23) SEQ ID NO: 15 and SEQ ID NO: 12;
24) SEQ ID NO: 15 and SEQ ID NO: 13;
25) SEQ ID NO: 16 and SEQ ID NO: 6;
26) SEQ ID NO: 16 and SEQ ID NO: 7;
27) SEQ ID NO: 16 and SEQ ID NO: 8;
28) SEQ ID NO: 16 and SEQ ID NO: 9;
29) SEQ ID NO: 16 and SEQ ID NO: 10;
30) SEQ ID NO: 16 and SEQ ID NO: 11;
31) SEQ ID NO: 16 and SEQ ID NO: 12;
32) SEQ ID NO: 16 and SEQ ID NO: 13;
33) SEQ ID NO: 17 and SEQ ID NO: 18; and

34) SEQ ID NO: 17 and SEQ ID NO: 19.

**34.** A nucleic acid molecule comprising a fragment encoding the small activating RNA according to any of claims 1-33.

**35.** The nucleic acid molecule of claim 34, wherein the nucleic acid molecule is an expression vector.

**36.** A cell comprising the small activating RNA according to any of claims 1-33 or the nucleic acid molecule according to any of claims 34-35.

**37.** A pharmaceutical composition comprising the small activating RNA according to any of claims 1-33 or the nucleic acid molecule according to any of claims 34-35, and optionally, pharmaceutically acceptable carrier.

**38.** The pharmaceutical composition according to claim 37, wherein the pharmaceutically acceptable carrier comprises lipid nanoparticles (LNPs).

**39.** The pharmaceutical composition according to claim 38, wherein the lipid nanoparticles comprises DLIN-KC2-DMA and/or DLin-MC3-DMA as a component.

**40.** A pharmaceutical composition of combined agents, comprising the small activating RNA according to any of claims 1-33, and a small molecule drug.

**41.** The combined pharmaceutical composition according to claim 40, wherein the small molecule drug is selected from Mitomycin C or Valrubicin.

**42.** A preparation comprising the small activating RNA according to any of claims 1-33, or the nucleic acid molecule according to any of claims 34-35, or the cell according to claim 36, or the pharmaceutical composition according to any of claims 37-39, and/or the pharmaceutical composition of combined agents according to any of claims 40-41.

**43.** A kit comprising the small activating RNA according to any of claims 1-33, or the nucleic acid molecule according to any of claims 34-35, or the cell described according to claim 36, or the pharmaceutical composition according to any of claims 37-39, the pharmaceutical composition of combined agents according to any of claims 40-41, and/or the preparation according to claim 42.

**44.** Use of the small activating RNA according to any of claims 1-33, or the nucleic acid molecule according to any of claims 34-35, or the cell according to claim 36, or the pharmaceutical composition according to any of claims 37-39, the pharmaceutical composition of combined agents according to any of claims 40-41, and/or the preparation according to claim 42 in the manufacture of a medicament with immunostimulatory activity.

**45.** Use of the small activating RNA according to any of claims 1-33, or the nucleic acid molecule according to any of claims 34-35, or the cell according to claim 36, or the pharmaceutical composition according to any of claims 37-39, the pharmaceutical composition of combined agents according to any of claims 40-41, and/or the preparation according to claim 42 in the manufacture of a medicament or formulation for activating or up-regulating the expression of p21 gene in a cell.

**46.** The use according to claim 45, wherein the use is for the manufacture of a medicament or formulation for preventing, treating or alleviating a malignant tumor or a benign proliferative lesion.

**47.** The use according to any of claims 45-46, wherein the use is for the manufacture of a medicament or formulation for preventing, treating or alleviating bladder cancer, prostate cancer, liver cancer, or colorectal cancer.

**48.** A method for preventing, treating or alleviating a malignant tumor or a benign proliferative lesion, wherein the method comprises administering the small activating RNA according to any of claims 1-33, or the nucleic acid molecule according to any of claims 34-35, or the cell according to claim 36, or the pharmaceutical composition according to any of claims 37-39, the pharmaceutical composition of combined agents according to any of claims 40-41, and/or the preparation according to claim 42 to an individual in need thereof.

**49.** The method according to claim 48, wherein the malignant tumor is selected from the group consisting of bladder cancer, prostate cancer, liver cancer and colorectal cancer.

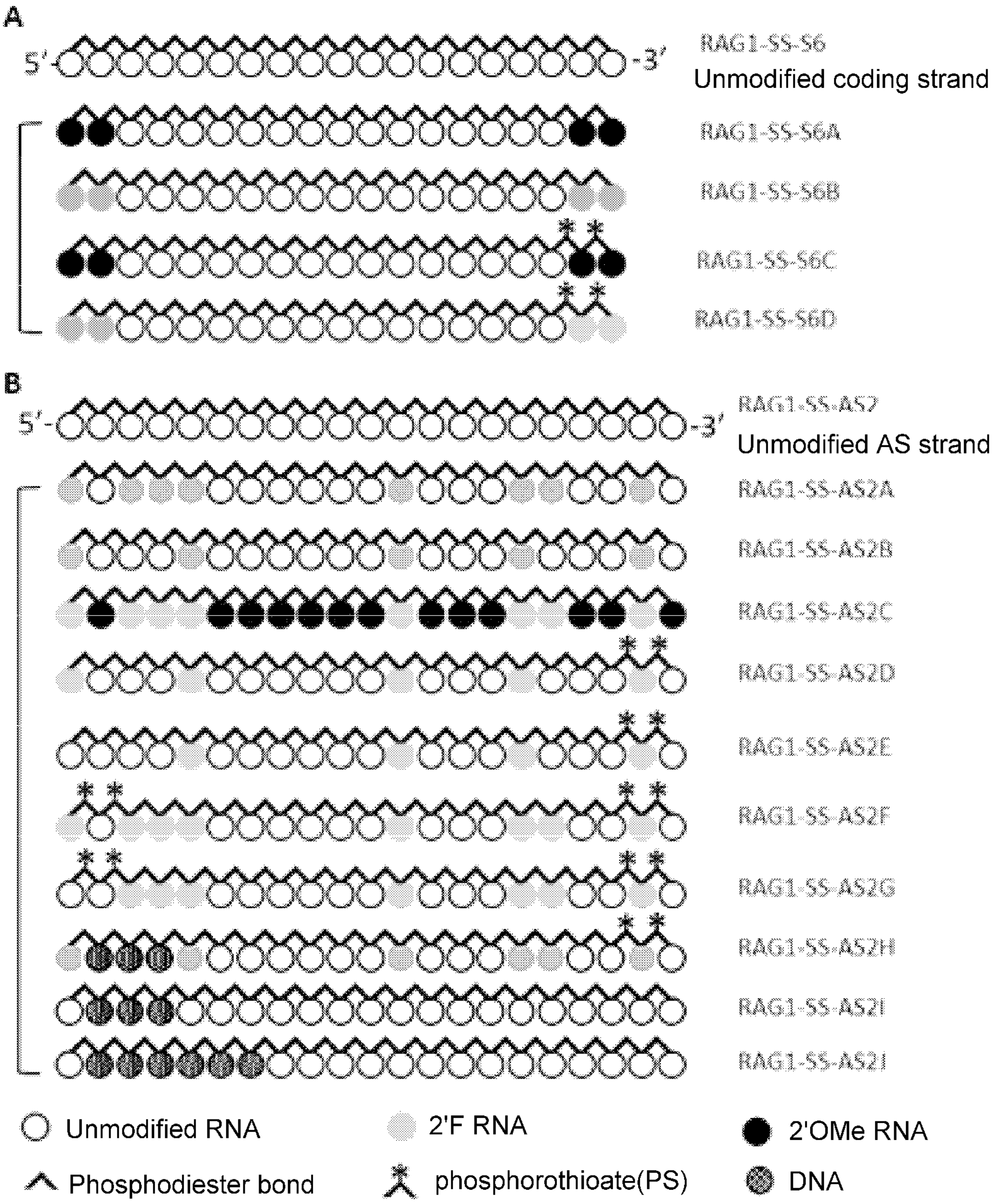
A
5'-
-3'
RAG1-SS-S6
Unmodified coding strand
RAG1-SS-S6A
RAG1-SS-S6B
RAG1-SS-S6C
RAG1-SS-S6D
B
5'-
-3'
RAG1-SS-AS2
Unmodified AS strand
RAG1-SS-AS2A
RAG1-SS-AS2B
RAG1-SS-AS2C
RAG1-SS-AS2D
RAG1-SS-AS2E
RAG1-SS-AS2F
RAG1-SS-AS2G
RAG1-SS-AS2H
RAG1-SS-AS2I
RAG1-SS-AS2J
Unmodified RNA
2'F RNA
2'OMe RNA
Phosphodiester bond
phosphorothioate(PS)
DNA

Figure 1

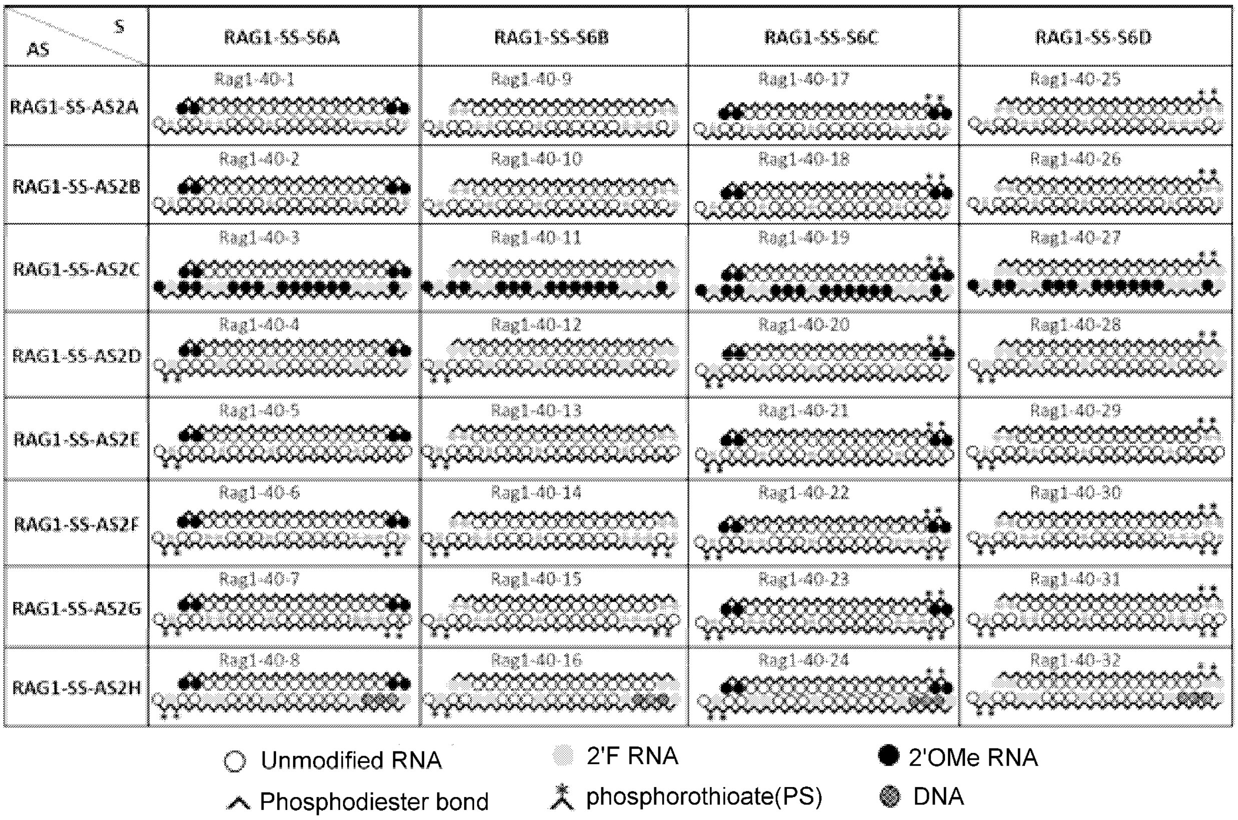


Figure 2

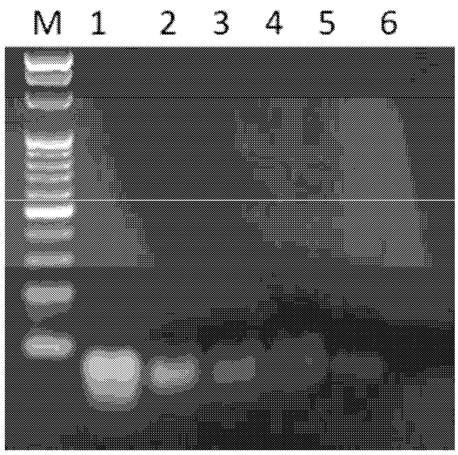


Figure 3

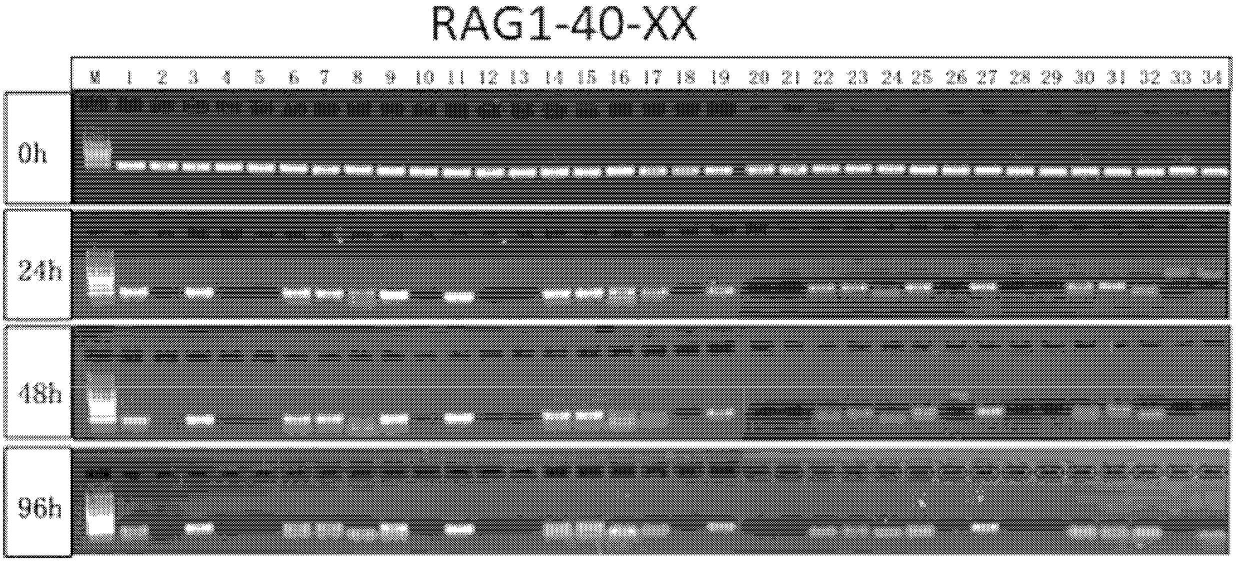


Figure 4

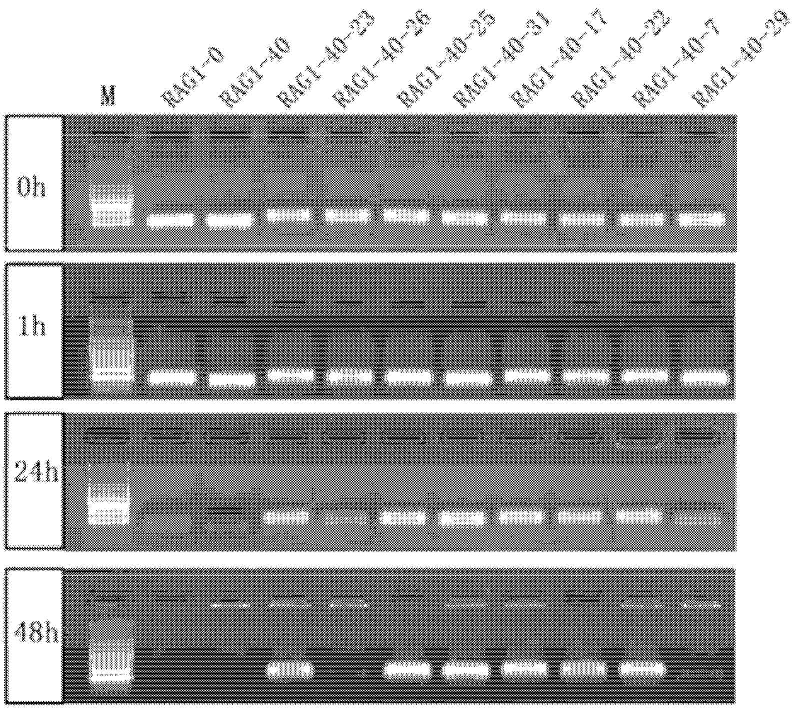


Figure 5

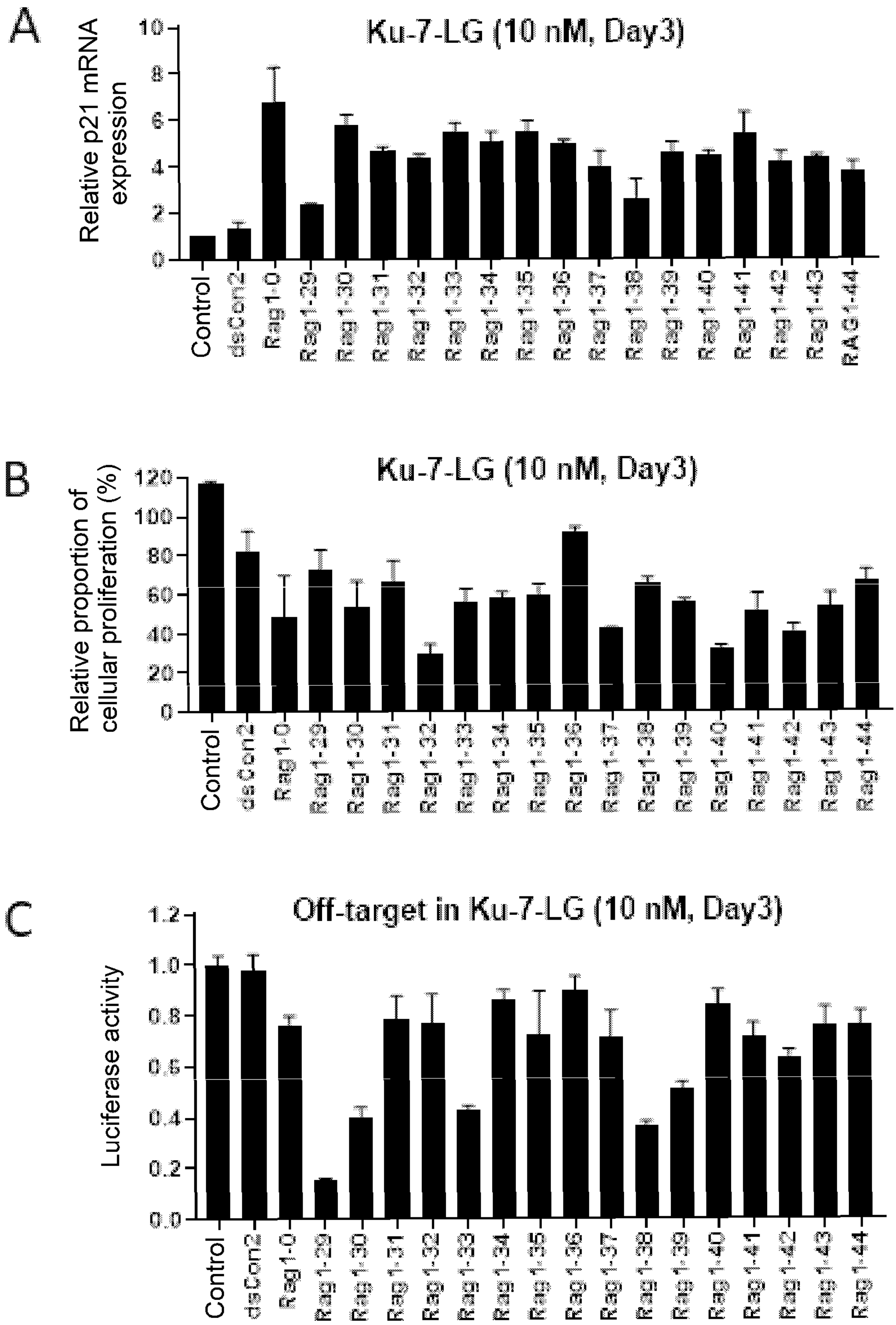
A
Ku-7-LG (10 nM, Day3)
Relative p21 mRNA expression
0
2
4
6
8
10
Control
dsCon2
Rag1-0
Rag1-29
Rag1-30
Rag1-31
Rag1-32
Rag1-33
Rag1-34
Rag1-35
Rag1-36
Rag1-37
Rag1-38
Rag1-39
Rag1-40
Rag1-41
Rag1-42
Rag1-43
RAG1-44
B
Ku-7-LG (10 nM, Day3)
Relative proportion of cellular proliferation (%)
0
20
40
60
80
100
120
Control
dsCon2
Rag1-0
Rag1-29
Rag1-30
Rag1-31
Rag1-32
Rag1-33
Rag1-34
Rag1-35
Rag1-36
Rag1-37
Rag1-38
Rag1-39
Rag1-40
Rag1-41
Rag1-42
Rag1-43
Rag1-44
C
Off-target in Ku-7-LG (10 nM, Day3)
Luciferase activity
0.0
0.2
0.4
0.6
0.8
1.0
1.2
Control
dsCon2
Rag1-0
Rag1-29
Rag1-30
Rag1-31
Rag1-32
Rag1-33
Rag1-34
Rag1-35
Rag1-36
Rag1-37
Rag1-38
Rag1-39
Rag1-40
Rag1-41
Rag1-42
Rag1-43
Rag1-44

Figure 6

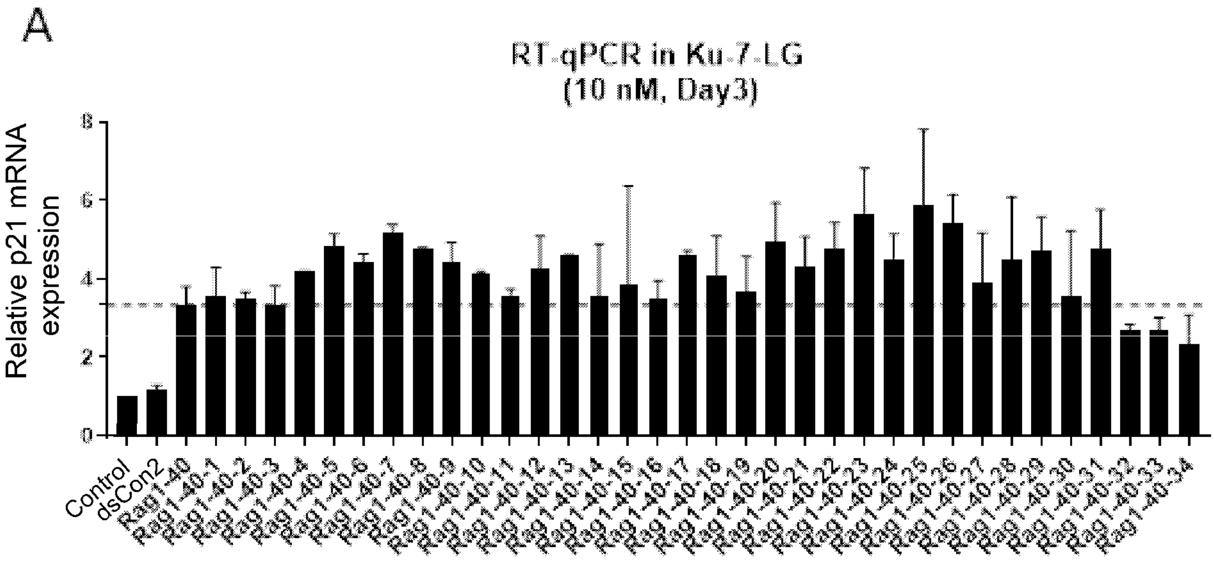
A
RT-qPCR in Ku-7-LG
(10 nM, Day3)
Relative p21 mRNA expression
0
2
4
6
8
Control
dsCon2
Rag1-40
Rag1-40-1
Rag1-40-2
Rag1-40-3
Rag1-40-4
Rag1-40-5
Rag1-40-6
Rag1-40-7
Rag1-40-8
Rag1-40-9
Rag1-40-10
Rag1-40-11
Rag1-40-12
Rag1-40-13
Rag1-40-14
Rag1-40-15
Rag1-40-16
Rag1-40-17
Rag1-40-18
Rag1-40-19
Rag1-40-20
Rag1-40-21
Rag1-40-22
Rag1-40-23
Rag1-40-24
Rag1-40-25
Rag1-40-26
Rag1-40-27
Rag1-40-28
Rag1-40-29
Rag1-40-30
Rag1-40-31
Rag1-40-32
Rag1-40-33
Rag1-40-34

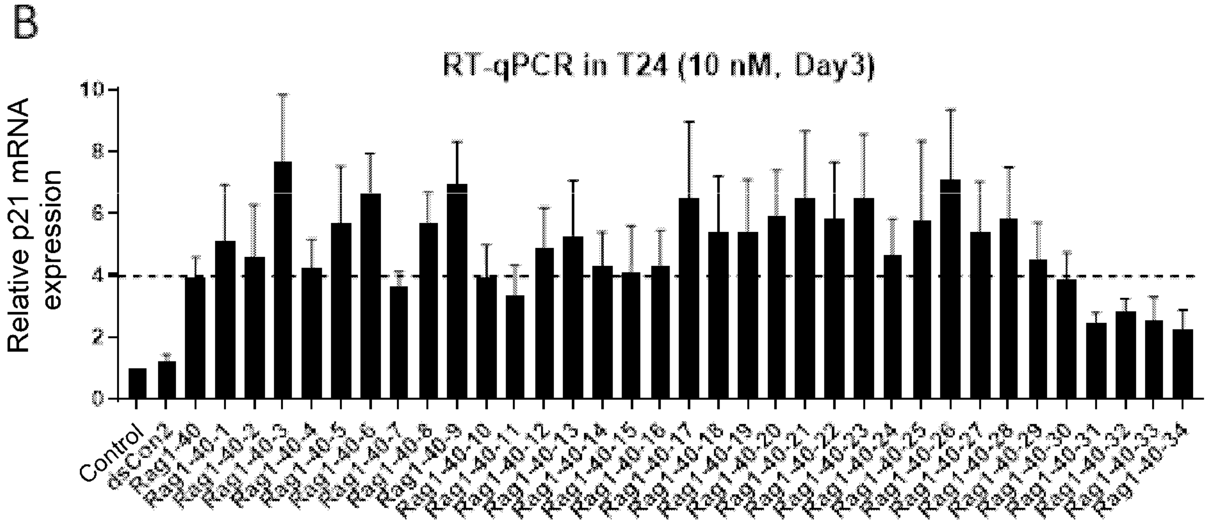
B
RT-qPCR in T24 (10 nM, Day3)
Relative p21 mRNA expression
0
2
4
6
8
10
Control
dsCon2
Rag1-40
Rag1-40-1
Rag1-40-2
Rag1-40-3
Rag1-40-4
Rag1-40-5
Rag1-40-6
Rag1-40-7
Rag1-40-8
Rag1-40-9
Rag1-40-10
Rag1-40-11
Rag1-40-12
Rag1-40-13
Rag1-40-14
Rag1-40-15
Rag1-40-16
Rag1-40-17
Rag1-40-18
Rag1-40-19
Rag1-40-20
Rag1-40-21
Rag1-40-22
Rag1-40-23
Rag1-40-24
Rag1-40-25
Rag1-40-26
Rag1-40-27
Rag1-40-28
Rag1-40-29
Rag1-40-30
Rag1-40-31
Rag1-40-32
Rag1-40-33
Rag1-40-34

Figure 7

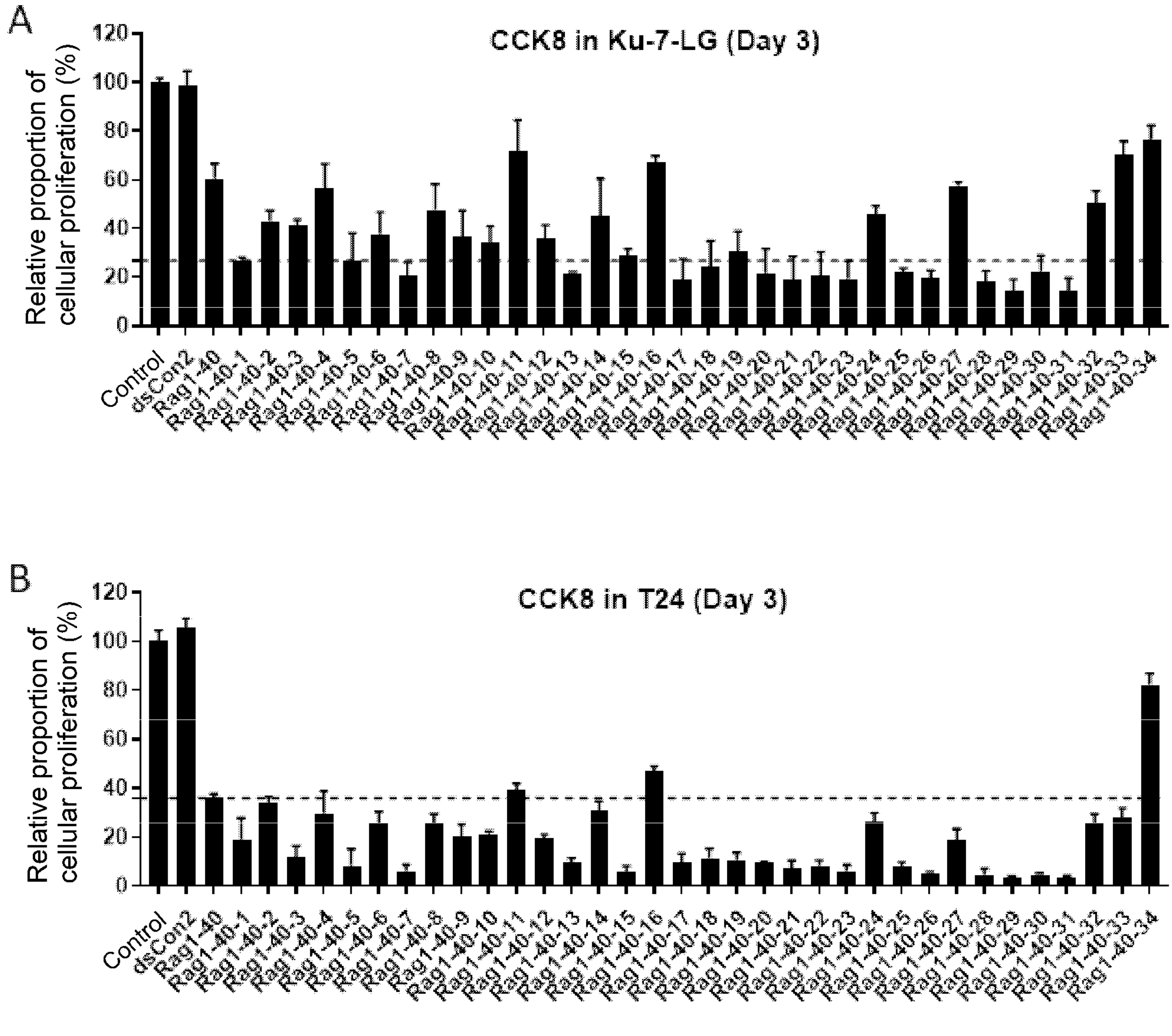
A
CCK8 in Ku-7-LG (Day 3)
Relative proportion of cellular proliferation (%)
0
20
40
60
80
100
120
Control
dsCon2
Rag1-40
Rag1-40-1
Rag1-40-2
Rag1-40-3
Rag1-40-4
Rag1-40-5
Rag1-40-6
Rag1-40-7
Rag1-40-8
Rag1-40-9
Rag1-40-10
Rag1-40-11
Rag1-40-12
Rag1-40-13
Rag1-40-14
Rag1-40-15
Rag1-40-16
Rag1-40-17
Rag1-40-18
Rag1-40-19
Rag1-40-20
Rag1-40-21
Rag1-40-22
Rag1-40-23
Rag1-40-24
Rag1-40-25
Rag1-40-26
Rag1-40-27
Rag1-40-28
Rag1-40-29
Rag1-40-30
Rag1-40-31
Rag1-40-32
Rag1-40-33
Rag1-40-34
B
CCK8 in T24 (Day 3)
Relative proportion of cellular proliferation (%)
0
20
40
60
80
100
120
Control
dsCon2
Rag1-40
Rag1-40-1
Rag1-40-2
Rag1-40-3
Rag1-40-4
Rag1-40-5
Rag1-40-6
Rag1-40-7
Rag1-40-8
Rag1-40-9
Rag1-40-10
Rag1-40-11
Rag1-40-12
Rag1-40-13
Rag1-40-14
Rag1-40-15
Rag1-40-16
Rag1-40-17
Rag1-40-18
Rag1-40-19
Rag1-40-20
Rag1-40-21
Rag1-40-22
Rag1-40-23
Rag1-40-24
Rag1-40-25
Rag1-40-26
Rag1-40-27
Rag1-40-28
Rag1-40-29
Rag1-40-30
Rag1-40-31
Rag1-40-32
Rag1-40-33
Rag1-40-34

Figure 8

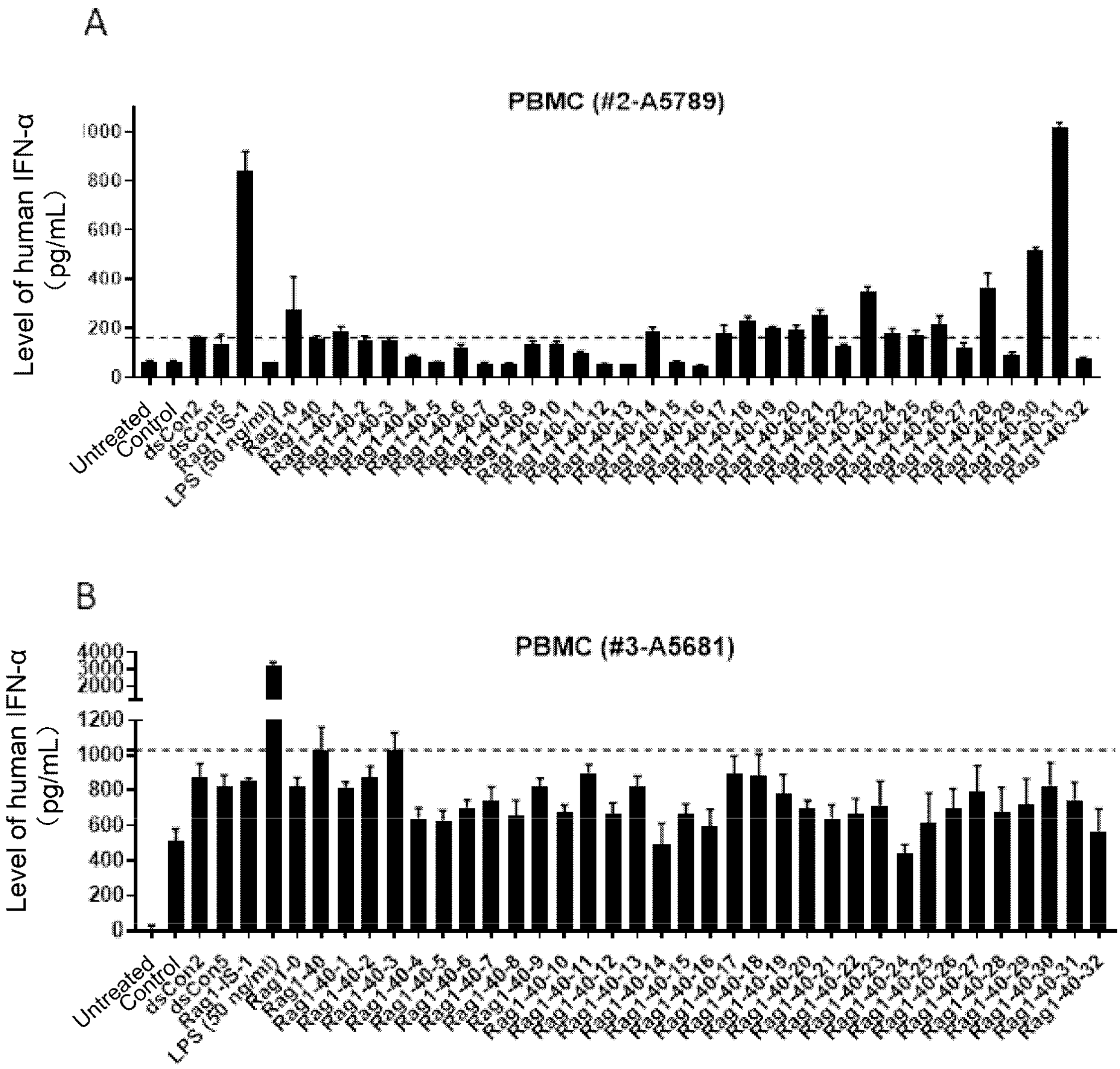


Figure 9

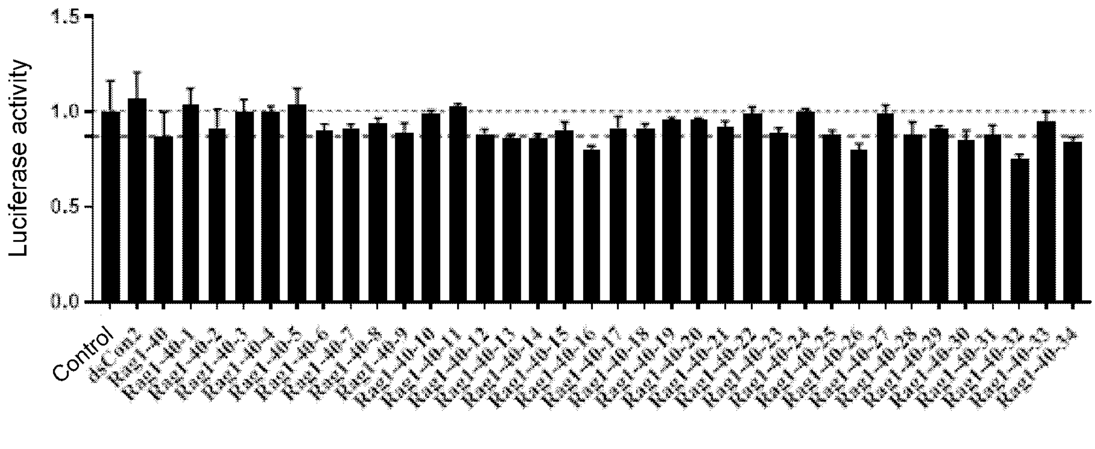


Figure 10

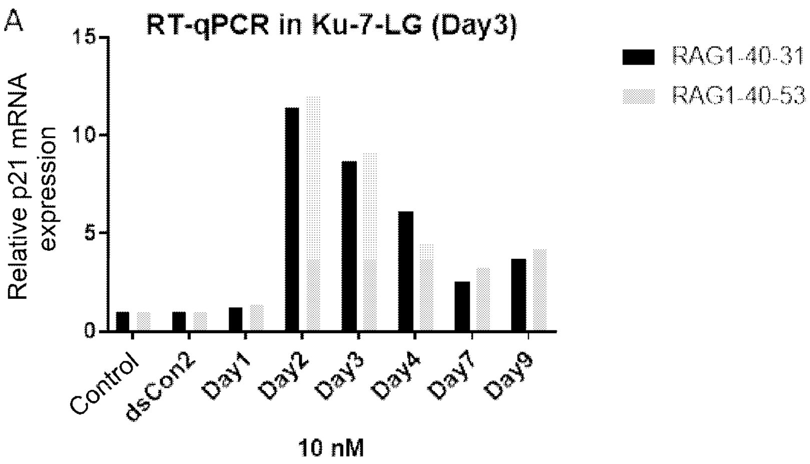


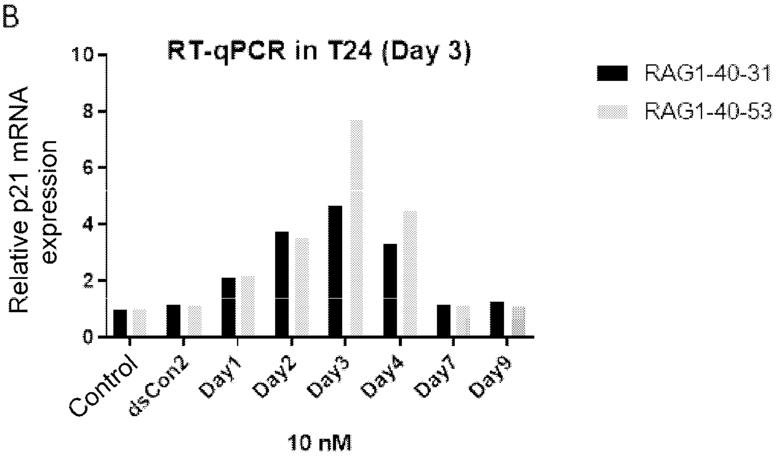


Figure 11

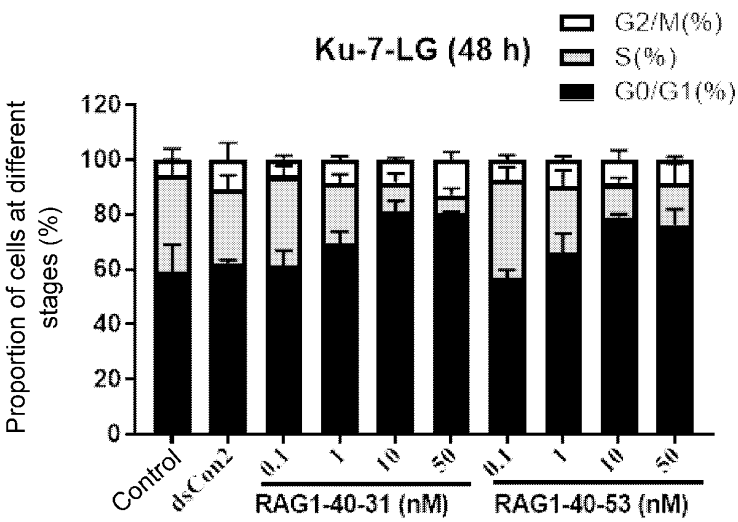


Figure 12

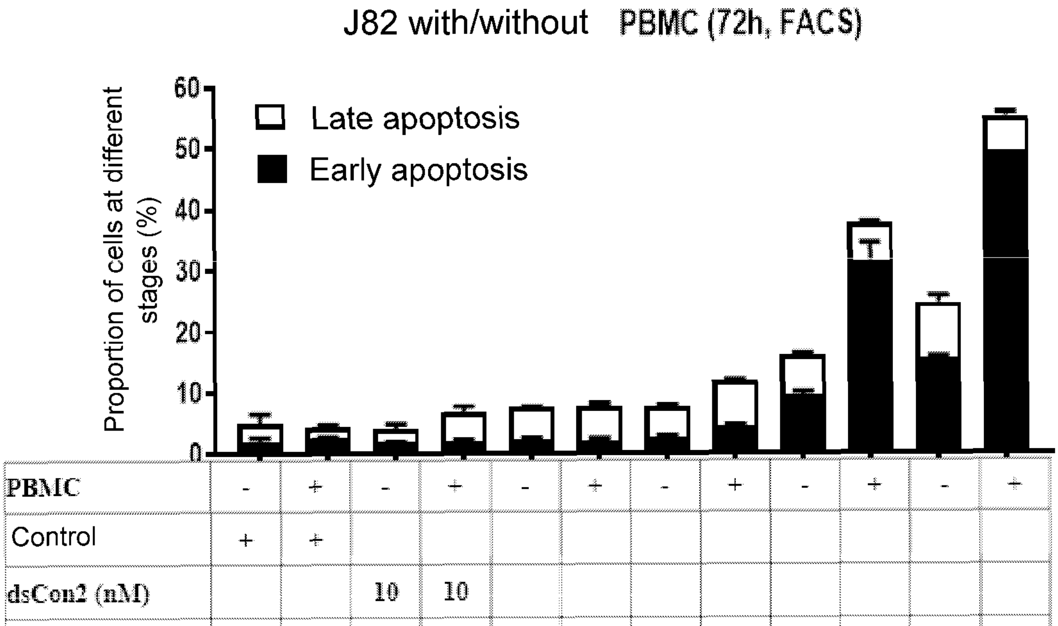


| | | | | | | | | | | | | |
|---|---|---|---|---|---|---|---|---|---|---|---|---|
| PBMC | - | + | - | + | - | + | - | + | - | + | - | + |
| Control | + | + | | | | | | | | | | |
| dsCon2 (nM) | | | 10 | 10 | | | | | | | | |
| RAG-40-31 (nM) | | | | | 0.1 | 0.1 | 1 | 1 | 10 | 10 | 25 | 25 |

Figure 13

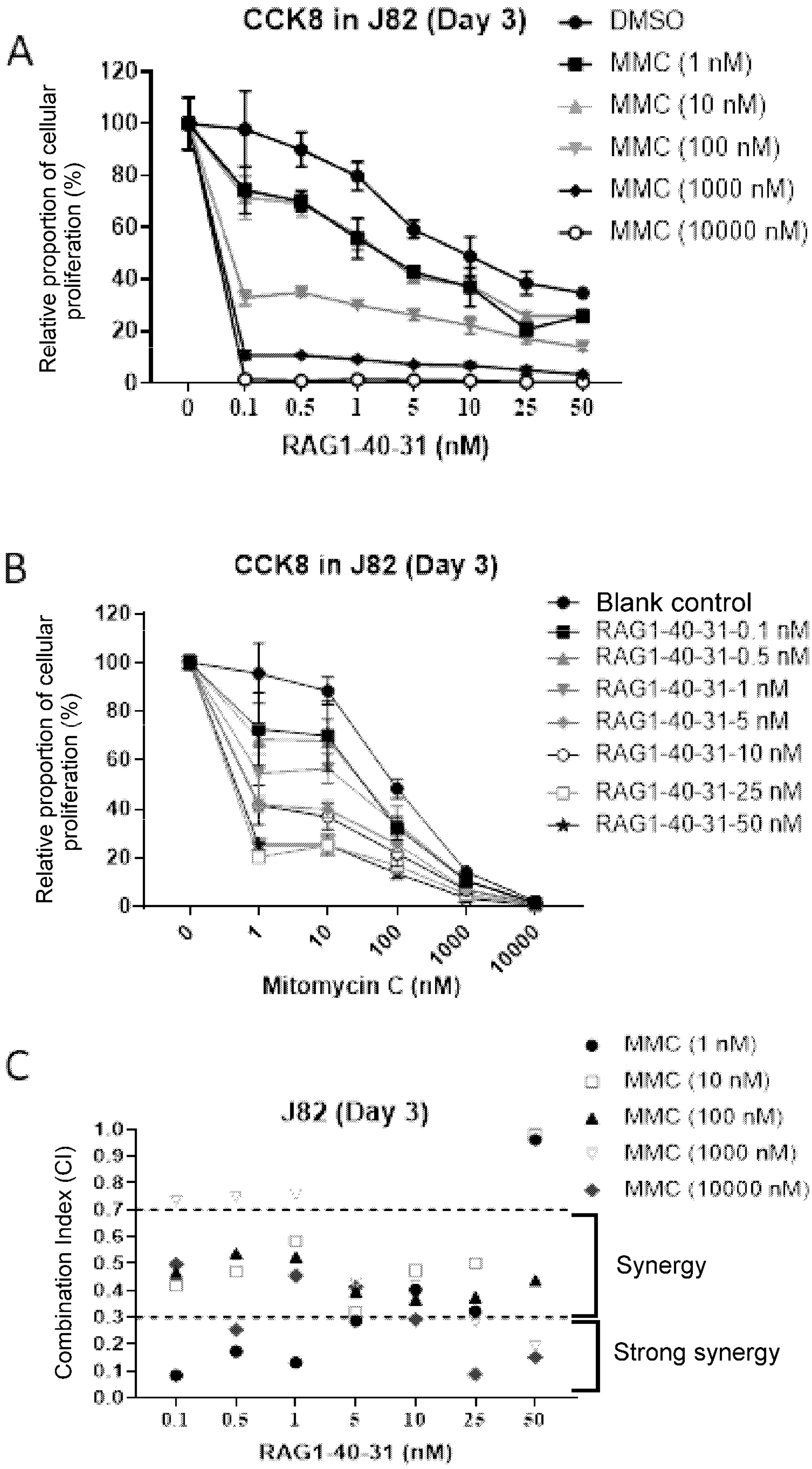
A
CCK8 in J82 (Day 3)
DMSO
MMC (1 nM)
MMC (10 nM)
MMC (100 nM)
MMC (1000 nM)
MMC (10000 nM)
Relative proportion of cellular proliferation (%)
120
100
80
60
40
20
0
0
0.1
0.5
1
5
10
25
50
RAG1-40-31 (nM)
B
CCK8 in J82 (Day 3)
Blank control
RAG1-40-31-0.1 nM
RAG1-40-31-0.5 nM
RAG1-40-31-1 nM
RAG1-40-31-5 nM
RAG1-40-31-10 nM
RAG1-40-31-25 nM
RAG1-40-31-50 nM
Relative proportion of cellular proliferation (%)
0
1
10
100
1000
10000
Mitomycin C (nM)
C
J82 (Day 3)
MMC (1 nM)
MMC (10 nM)
MMC (100 nM)
MMC (1000 nM)
MMC (10000 nM)
Combination Index (CI)
1.0
0.9
0.8
0.7
0.6
0.5
0.4
0.3
0.2
0.1
0.0
Synergy
Strong synergy
0.1
0.5
1
5
10
25
50
RAG1-40-31 (nM)

Figure 14

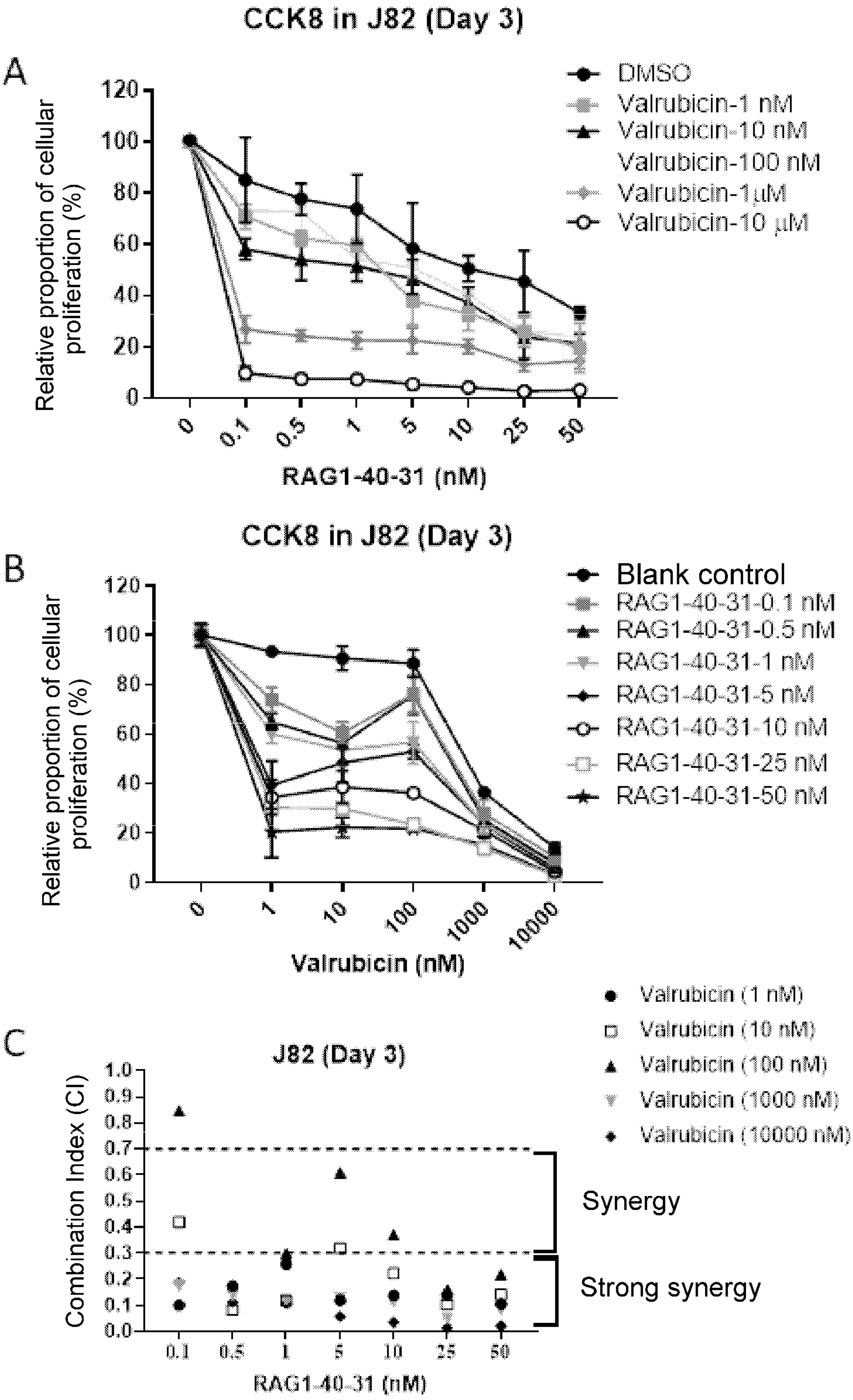
CCK8 in J82 (Day 3)
A
Relative proportion of cellular proliferation (%)
0
20
40
60
80
100
120
0
0.1
0.5
1
5
10
25
50
RAG1-40-31 (nM)
DMSO
Valrubicin-1 nM
Valrubicin-10 nM
Valrubicin-100 nM
Valrubicin-1 μM
Valrubicin-10 μM
CCK8 in J82 (Day 3)
B
Relative proportion of cellular proliferation (%)
0
20
40
60
80
100
120
0
1
10
100
1000
10000
Valrubicin (nM)
Blank control
RAG1-40-31-0.1 nM
RAG1-40-31-0.5 nM
RAG1-40-31-1 nM
RAG1-40-31-5 nM
RAG1-40-31-10 nM
RAG1-40-31-25 nM
RAG1-40-31-50 nM
C
J82 (Day 3)
Combination Index (CI)
0.0
0.1
0.2
0.3
0.4
0.5
0.6
0.7
0.8
0.9
1.0
0.1
0.5
1
5
10
25
50
RAG1-40-31 (nM)
Valrubicin (1 nM)
Valrubicin (10 nM)
Valrubicin (100 nM)
Valrubicin (1000 nM)
Valrubicin (10000 nM)
Synergy
Strong synergy

Figure 15

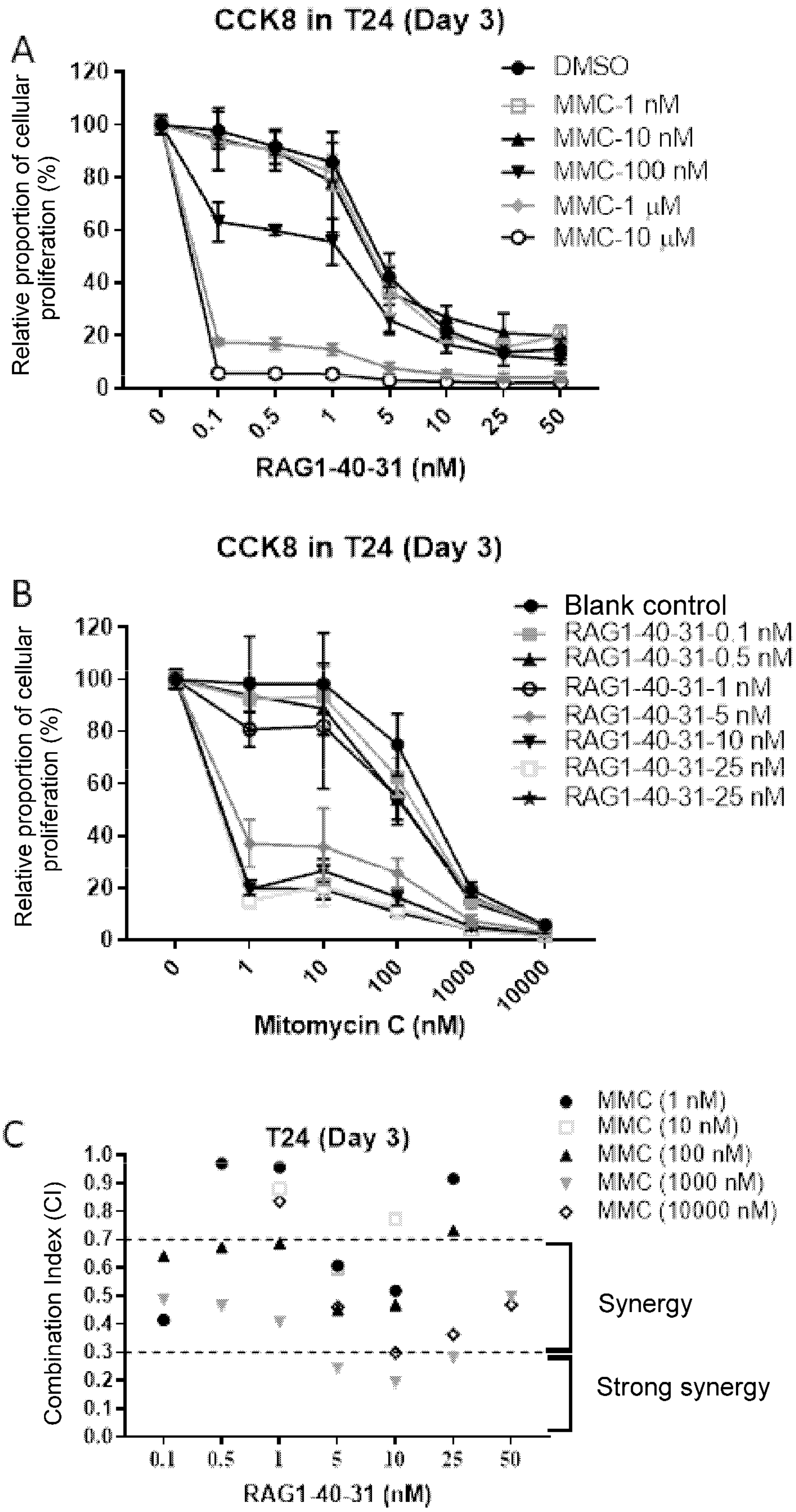
CCK8 in T24 (Day 3)
A
Relative proportion of cellular proliferation (%)
RAG1-40-31 (nM)
DMSO
MMC-1 nM
MMC-10 nM
MMC-100 nM
MMC-1 μM
MMC-10 μM
CCK8 in T24 (Day 3)
B
Relative proportion of cellular proliferation (%)
Mitomycin C (nM)
Blank control
RAG1-40-31-0.1 nM
RAG1-40-31-0.5 nM
RAG1-40-31-1 nM
RAG1-40-31-5 nM
RAG1-40-31-10 nM
RAG1-40-31-25 nM
RAG1-40-31-25 nM
C
T24 (Day 3)
Combination Index (CI)
RAG1-40-31 (nM)
MMC (1 nM)
MMC (10 nM)
MMC (100 nM)
MMC (1000 nM)
MMC (10000 nM)
Synergy
Strong synergy

Figure 16

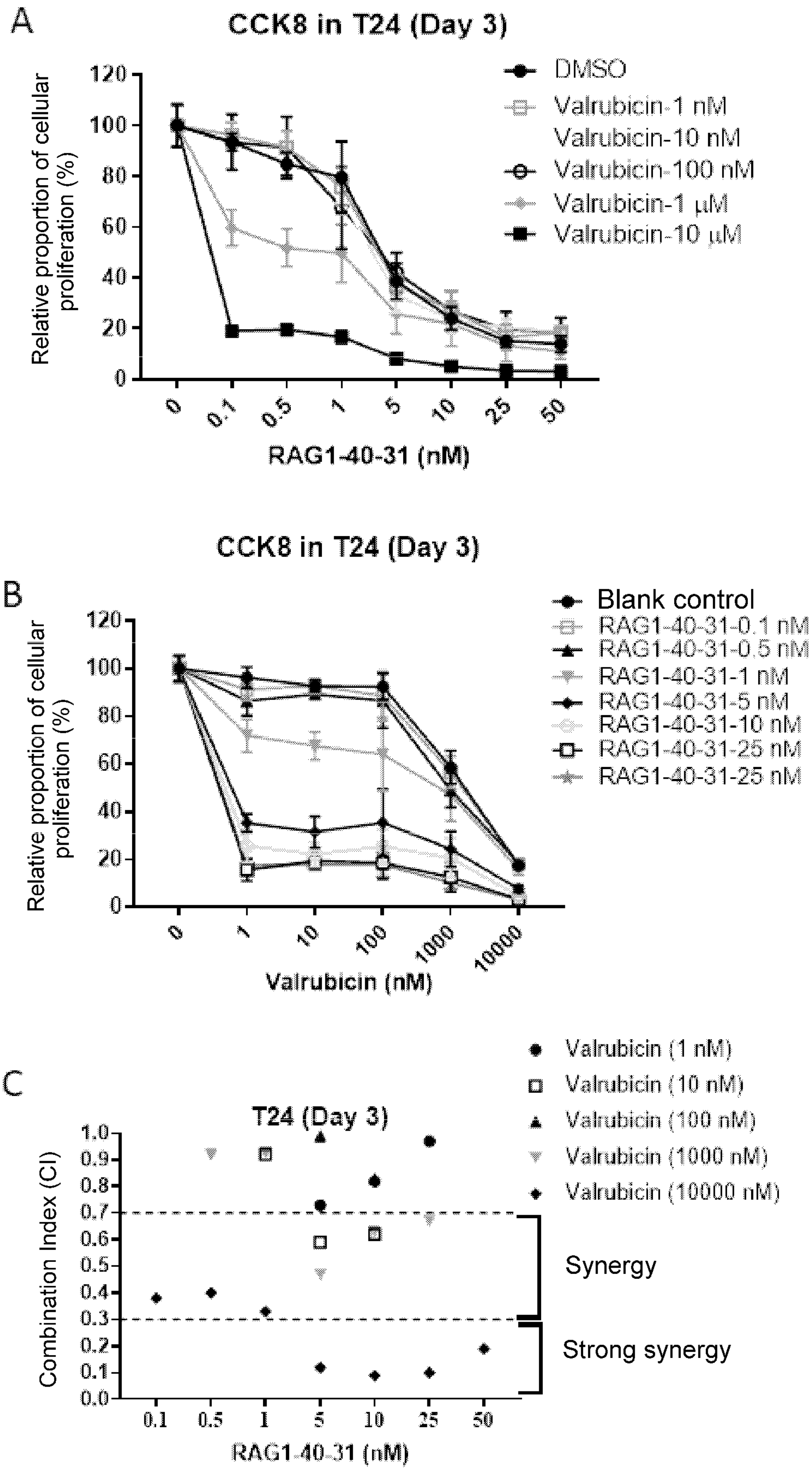
A
CCK8 in T24 (Day 3)
Relative proportion of cellular proliferation (%)
RAG1-40-31 (nM)
DMSO
Valrubicin-1 nM
Valrubicin-10 nM
Valrubicin-100 nM
Valrubicin-1 μM
Valrubicin-10 μM
B
CCK8 in T24 (Day 3)
Relative proportion of cellular proliferation (%)
Valrubicin (nM)
Blank control
RAG1-40-31-0.1 nM
RAG1-40-31-0.5 nM
RAG1-40-31-1 nM
RAG1-40-31-5 nM
RAG1-40-31-10 nM
RAG1-40-31-25 nM
RAG1-40-31-25 nM
C
T24 (Day 3)
Combination Index (CI)
RAG1-40-31 (nM)
Valrubicin (1 nM)
Valrubicin (10 nM)
Valrubicin (100 nM)
Valrubicin (1000 nM)
Valrubicin (10000 nM)
Synergy
Strong synergy

Figure 17

## INTERNATIONAL SEARCH REPORT

International application No.
**PCT/CN2022/074635**

**A. CLASSIFICATION OF SUBJECT MATTER**

C12N 15/113(2010.01)i；C12N 15/85(2006.01)i；A61P 35/00(2006.01)i；A61K 31/713(2006.01)i

According to International Patent Classification (IPC) or to both national classification and IPC

**B. FIELDS SEARCHED**

Minimum documentation searched (classification system followed by classification symbols)

C12N; A61P; A61K

Documentation searched other than minimum documentation to the extent that such documents are included in the fields searched

Electronic data base consulted during the international search (name of data base and, where practicable, search terms used)

CNTXT, CNABS, VEN, WOTXT, EPTXT, USTXT, CNKI, 万方, WANFANG, pubmed, ISI web of knowledge, STN: 小激活RNA, 活化RNA, RNA激活, saRNA, small activation RNA, agRNA, antigene RNA, RNA activation, RNAa, 化学修饰, 2'-氟, 2'-F, 2'-甲氧基, 2'-OME, 2'-O-methyl, 硫代磷酸, chemical modification, SEQ ID NOs: 5-19, RAG1-40, 中美瑞康核酸技术(南通)研究院有限公司, 李龙承, Li longcheng

**C. DOCUMENTS CONSIDERED TO BE RELEVANT**

| Category* | Citation of document, with indication, where appropriate, of the relevant passages | Relevant to claim No. |
|---|---|---|
| X | US 2020208152 A1 (MINA THERAPEUTICS LTD.) 02 July 2020 (2020-07-02)<br>description, paragraphs 25, 58-61, 90, 120-136, 169-170, 173-187, and 368 | 1-28, 34-44, 48-49 |
| Y | US 2020208152 A1 (MINA THERAPEUTICS LTD.) 02 July 2020 (2020-07-02)<br>description, paragraphs 25, 58-61, 90, 120-136, 169-170, 173-187, and 368 | 29-49 |
| Y | WO 2019196887 A1 (RACTIGEN THERAPEUTICS) 17 October 2019 (2019-10-17)<br>description, p. 2, paragraph 2 to p. 4, paragraph 1, table 1, and embodiment 10 | 29-49 |
| X | US 2015126578 A1 (PLACE, R. F. et al.) 07 May 2015 (2015-05-07)<br>description, paragraphs 8, 10-11, 13-16, 82-91, 114, and 170-171, and table 1 | 1-30, 34-49 |
| Y | US 2015126578 A1 (PLACE, R. F. et al.) 07 May 2015 (2015-05-07)<br>description, paragraphs 8, 10-11, 13-16, 82-91, 114, and 170-171, and table 1 | 31-49 |
| X | WO 2019196883 A1 (RACTIGEN THERAPEUTICS) 17 October 2019 (2019-10-17)<br>description, p. 2, paragraph 1 to p. 4, paragraph 5, and embodiment 1 | 1-30, 34-49 |
| Y | WO 2019196883 A1 (RACTIGEN THERAPEUTICS) 17 October 2019 (2019-10-17)<br>description, p. 2, paragraph 1 to p. 4, paragraph 5, and embodiment 1 | 31-49 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 April 2022** | **26 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.
PCT/CN2022/074635

C. DOCUMENTS CONSIDERED TO BE RELEVANT

| Category* | Citation of document, with indication, where appropriate, of the relevant passages | Relevant to claim No. |
|---|---|---|
| X | WATTS, J. K. et al. "Effect of chemical modifications on modulation of gene expression by duplex antigene RNAs that are complementary to non-coding transcripts at gene promoters" *Nucleic Acids Research,* Vol. 38, No. 15, 19 April 2010 (2010-04-19), pp. 5242-5259 | 1-28, 34-49 |
| Y | WATTS, J. K. et al. "Effect of chemical modifications on modulation of gene expression by duplex antigene RNAs that are complementary to non-coding transcripts at gene promoters" *Nucleic Acids Research,* Vol. 38, No. 15, 19 April 2010 (2010-04-19), pp. 5242-5259 | 29-49 |
| X | PLACE, R. F. et al. "Formulation of Small Activating RNA Into Lipidoid Nanoparticles Inhibits Xenograft Prostate Tumor Growth by Inducing p21 Expression" *Molecular Therapy-Nucleic Acids,* Vol. 1, No. e15, 27 March 2012 (2012-03-27), pp. 1-12 | 1-30, 34-49 |
| Y | PLACE, R. F. et al. "Formulation of Small Activating RNA Into Lipidoid Nanoparticles Inhibits Xenograft Prostate Tumor Growth by Inducing p21 Expression" *Molecular Therapy-Nucleic Acids,* Vol. 1, No. e15, 27 March 2012 (2012-03-27), pp. 1-12 | 31-49 |
| X | PLACE, R. F. et al. "Defining Features and Exploring Chemical Modifications to Manipulate RNAa Activity" *Current Pharmaceutical Biotechnology,* Vol. 11, No. 5, 31 August 2010 (2010-08-31), pp. 518-526 | 1-30, 34-49 |
| Y | PLACE, R. F. et al. "Defining Features and Exploring Chemical Modifications to Manipulate RNAa Activity" *Current Pharmaceutical Biotechnology,* Vol. 11, No. 5, 31 August 2010 (2010-08-31), pp. 518-526 | 31-49 |
| X | KANG, M. R. et al. "Intravesical Delivery of Small Activating RNA Formulated into Lipid Nanoparticles Inhibits Orthotopic Bladder Tumor Growth" *Cancer Research,* Vol. 72, No. 19, 01 October 2012 (2012-10-01), pp. 5069-5079 | 1-30, 34-49 |
| Y | KANG, M. R. et al. "Intravesical Delivery of Small Activating RNA Formulated into Lipid Nanoparticles Inhibits Orthotopic Bladder Tumor Growth" *Cancer Research,* Vol. 72, No. 19, 01 October 2012 (2012-10-01), pp. 5069-5079 | 31-49 |
| A | KWOK, A. et al. "Developing small activating RNA as a therapeutic: current challenges and promises" *Ther. Deliv.,* Vol. 10, No. 3, 26 March 2019 (2019-03-26), pp. 151-164 | 1-49 |

Form PCT/ISA/210 (second sheet) (January 2015)

INTERNATIONAL SEARCH REPORT

International application No.
**PCT/CN2022/074635**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**

International application No.
**PCT/CN2022/074635**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **48-49**
because they relate to subject matter not required to be searched by this Authority, namely:

[1] Claims 48-49 relate to a method for preventing, treating, or alleviating malignant tumors or benign proliferative lesions. That is, claims 48-49 relate to the subject matter defined in PCT Rule 39.1 that does not warrant a search conducted by the international searching authority: (4) a method for treating a human body or animal body by surgery or therapy and a diagnosis method implemented on the human body or animal body. The international search authority conducts a search on the basis of a use of small activation RNA, a nucleic acid molecule, cells, a pharmaceutical composition, a combined pharmaceutical composition, and/or a formulation in the preparation of a drug for preventing, treating, or alleviating malignant tumors or benign proliferative lesions.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/074635**

| Patent document cited in search report | Publication date (day/month/year) | Patent family member(s) | Publication date (day/month/year) |
|---|---|---|---|
| US 2020208152 A1 | 02 July 2020 | EP 3679140 A1 | 15 July 2020 |
| | | WO 2019048645 A1 | 14 March 2019 |
| WO 2019196887 A1 | 17 October 2019 | CA 3094575 A1 | 17 October 2019 |
| | | EP 3778892 A1 | 17 February 2021 |
| | | US 2021332366 A1 | 28 October 2021 |
| | | IL 277921 D0 | 30 November 2020 |
| | | CN 110959042 A | 03 April 2020 |
| | | AU 2019252204 A1 | 26 November 2020 |
| | | JP 2021520221 A | 19 August 2021 |
| | | KR 20200140377 A | 15 December 2020 |
| US 2015126578 A1 | 07 May 2015 | None | |
| WO 2019196883 A1 | 17 October 2019 | US 2021024915 A1 | 28 January 2021 |
| | | KR 20200143428 A | 23 December 2020 |
| | | JP 2021520220 A | 19 August 2021 |
| | | EP 3778893 A1 | 17 February 2021 |
| | | CN 110678549 A | 10 January 2020 |
| | | CN 113584027 A | 02 November 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20190240354 A1
- US 20190336452 A1
- US 20160038612 A1
- WO 2017192679 A1


### Non-patent literature cited in the description


- **LI.** *Adv Exp Med Biol.,* 2007, vol. 983, 1-20
- **MANOHARAN.** *Curr Opin Chem Biol.,* 2004, vol. 8 (6), 570-579
- **KHVOROVA ; WATTS.** *Nat Biotechnol.,* 2017, vol. 35 (3), 238-248
- **KHVOROVA ; WATTS.** *Nat Biotechnol,* 2017, vol. 35 (3), 238-248
- **WATTS ; DELEAVEY et al.** *Drug Discov Today,* 2008, vol. 13 (19-20), 842-855
- **CHENG.** *The role of helper lipids in lipid nanoparticles (LNPs) designed for oligonucleotide delivery,* 01 April 2016, vol. 99, 129-137
- **SHI.** Biodistribution of Small Interfering RNA at the Organ and Cellular Levels after Lipid Nanoparticle-mediated Delivery. *J Histochem Cytochem.,* August 2011, vol. 59 (8), 727-740